# EUROPEAN PATENT APPLICATION

(11) **EP 2 085 478 A1**
(43) Date of publication of application: **05.08.2009**
(21) Application number: 09156996.2
(22) Date of filing: 20.12.1996
(51) Int. Cl.: C12N 15/63, C12N 5/00, C12N 15/00, A01N 43/04, A61K 31/70

(54) **A long QT syndrome gene which encodes KVLQT1 and its association with minK**

(30) Priority: 22.12.1995 US 19014 P; 29.10.1996 US 739383
(62) Divisional of application: 96946233.2
(71) Applicant: University of Utah Research Foundation, Salt Lake City, Utah 84108 (US); Genzyme Genetics, Framingham, MA 01701 (US)
(72) Inventor: Keating, Mark, F., Salt Lake City, UT 84103 (US); Curran, Mark, E., Hopkinton, MA 01748 (US); Landes, Gregory, M., Northboro, MA 01532 (US); Connors, Timothy, D., Hopkinton, MA 01748 (US); Burn, Timothy, C., Hockessin, DE 19707 (US); Wang, Qing, Shaker Heights, OH 44120 (US)
(74) Representative: Dörries, Hans Ulrich

(57) **Abstract**

One aspect of the invention relates to the identification of the molecular basis of long QT syndrome. More specifically, the invention has identified that mutated KVLQT1 causes long QT syndrome. The analysis of this gene will provide an early diagnosis of subjects with long QT syndrome. The diagnostic methods comprise analyzing the nucleic acid sequence of the KVLQT1 gene of an individual to be tested and comparing them with the nucleic acid sequence of the native, non-variant gene. Alternatively, the amino acid sequence of KVLQT1 may be analyzed for mutations which cause long QT syndrome. Presymptomatic diagnosis of long QT syndrome will enable practitioners to treat this disorder using existing medical therapy. A second aspect of the invention relates to the realization that KVLQT1 coassembles with minK to form a cardiac potassium channel.; This allows one to assay for drugs which interact with this channel to identify new drugs which are useful for treating or preventing long QT.

SEQUENCE LISTING

(1) GENERAL INFORMATION:

(i) APPLICANT: University of Utah Research Foundation

(ii) TITLE OF INVENTION: KVLQT1 - A LONG QT SYNDROME GENE WHICH
ENCODES KVLQT1 WHICH COASSEMBLES WITH minK TO FORM
CARDIAC I_{Ks} POTASSIUM CHANNELS

(iii) NUMBER OF SEQUENCES: 26

(iv) CORRESPONDENCE ADDRESS:
(A) ADDRESSEE: Venable, Baetjer, Howard & Civiletti, LLP
(B) STREET: 1201 New York Avenue, N.W., Suite 1000
(C) CITY: Washington
(D) STATE: DC
(E) COUNTRY: U.S.A.
(F) ZIP: 20005

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: Word for Windows 6.0

(vi) CURRENT APPLICATION DATA:
(A) APPLICATION NUMBER: WO
(B) FILING DATE: 20-DEC-1996
(C) CLASSIFICATION:

(viii) ATTORNEY/AGENT INFORMATION:
(A) NAME: Ihnen, Jeffrey L.
(B) REGISTRATION NUMBER: 28,957
(C) REFERENCE/DOCKET NUMBER: 19780-116871-WO

(ix) TELECOMMUNICATION INFORMATION:
(A) TELEPHONE: 202-962-4800
(B) TELEFAX: 202-962-8300

(2) INFORMATION FOR SEQ ID NO:1:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic oligomer"

(xi) SEQUENCE DESCRIPTION: SEQ ID NO:1:

CAGATCCTGA GGATGCT 17

(2) INFORMATION FOR SEQ ID NO:2:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "Synthetic oligomer"

(xi) SEQUENCE DESCRIPTION: SEQ ID NO:2:

GTACCTGGCT GAGAAGG 17

(2) INFORMATION FOR SEQ ID NO:3:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:3:

GAGATCGTGC TGGTGGTGTT CT 22

(2) INFORMATION FOR SEQ ID NO:4:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:4:

CTTCCTGGTC TGGAAACCTG G 21

(2) INFORMATION FOR SEQ ID NO:5:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:5:

CTCTTCCCTG GGGCCCTGGC 20

(2) INFORMATION FOR SEQ ID NO:6:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:6:

TGCGGGGGAG CTTGTGGCAC AG 22

(2) INFORMATION FOR SEQ ID NO:7:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:7:

GGGCATCCGC TTCCTGCAGA 20

(2) INFORMATION FOR SEQ ID NO:8:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:8:

CTGGGCCCCT ACCCTAACCC 20

(2) INFORMATION FOR SEQ ID NO:9:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:9:

TCCTGGAGCC CGAACTGTGT GT 22

(2) INFORMATION FOR SEQ ID NO:10:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:10:

TGTCCTGCCC ACTCCTCAGC CT 22

(2) INFORMATION FOR SEQ ID NO:11:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:11:

CCCCAGGACC CCAGCTGTCC AA 22

(2) INFORMATION FOR SEQ ID NO:12:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:12:

AGGCTGACCA CTGTCCCTCT 20

(2) INFORMATION FOR SEQ ID NO:13:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:13:

GCTGGCAGTG GCCTGTGTGG A 21

(2) INFORMATION FOR SEQ ID NO:14:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "PCR primer"

(xi) SEQUENCE DESCRIPTION: SEQ ID NO:14:

AACAGTGACC AAAATGACAG TGAC 24

(2) INFORMATION FOR SEQ ID NO:15:

(i) SEQUENCE CHARACTERISTICS:
(A) LENGTH: 2633 base pairs
(B) TYPE: nucleic acid
(C) STRANDEDNESS: double
(D) TOPOLOGY: linear

(ii) MOLECULE TYPE: cDNA

(iii) HYPOTHETICAL: NO

(iv) ANTI-SENSE: NO

(vi) ORIGINAL SOURCE:
(A) ORGANISM: Homo sapiens

(ix) FEATURE:
(A) NAME/KEY: CDS
(B) LOCATION: 2..1642

(xi) SEQUENCE DESCRIPTION: SEQ ID NO:15:

C TTC CTC ATC GTC CTG GTC TGC CTC ATC TTC AGC GTG CTG TCC ACC 46
Phe Leu Ile Val Leu Val Cys Leu Ile Phe Ser Val Leu Ser Thr
1               5                   10                  15

ATC GAG CAG TAT GCC GCC CTG GCC ACG GGG ACT CTC TTC TGG ATG GAG 94
Ile Glu Gln Tyr Ala Ala Leu Ala Thr Gly Thr Leu Phe Trp Met Glu
                20                  25                  30

ATC GTG CTG GTG GTG TTC TTC GGG ACG GAG TAC GTG GTC CGC CTC TGG 142
Ile Val Leu Val Val Phe Phe Gly Thr Glu Tyr Val Val Arg Leu Trp
            35                  40                  45

TCC GCC GGC TGC CGC AGC AAG TAC GTG GGC CTC TGG GGG CGG CTG CGC 190
Ser Ala Gly Cys Arg Ser Lys Tyr Val Gly Leu Trp Gly Arg Leu Arg
        50                  55                  60

TTT GCC CGG AAG CCC ATT TCC ATC ATC GAC CTC ATC GTG GTC GTG GCC 238
Phe Ala Arg Lys Pro Ile Ser Ile Ile Asp Leu Ile Val Val Val Ala
    65                  70                  75

TCC ATG GTG GTC CTC TGC GTG GGC TCC AAG GGG CAG GTG TTT GCC ACG 286
Ser Met Val Val Leu Cys Val Gly Ser Lys Gly Gln Val Phe Ala Thr
80                  85                  90                  95

TCG GCC ATC AGG GGC ATC CGC TTC CTG CAG ATC CTG AGG ATG CTA CAC 334
Ser Ala Ile Arg Gly Ile Arg Phe Leu Gln Ile Leu Arg Met Leu His
                100                 105                 110

GTC GAC CGC CAG GGA GGC ACC TGG AGG CTC CTG GGC TCC GTG GTC TTC 382
Val Asp Arg Gln Gly Gly Thr Trp Arg Leu Leu Gly Ser Val Val Phe
            115                 120                 125

ATC CAC CGC CAG GAG CTG ATA ACC ACC CTG TAC ATC GGC TTC CTG GGC 430
Ile His Arg Gln Glu Leu Ile Thr Thr Leu Tyr Ile Gly Phe Leu Gly
        130                 135                 140

CTC ATC TTC TCC TCG TAC TTT GTG TAC CTG GCT GAG AAG GAC GCG GTG 478
Leu Ile Phe Ser Ser Tyr Phe Val Tyr Leu Ala Glu Lys Asp Ala Val
    145                 150                 155

AAC GAG TCA GGC CGC GTG GAG TTC GGC AGC TAC GCA GAT GCG CTG TGG 526
Asn Glu Ser Gly Arg Val Glu Phe Gly Ser Tyr Ala Asp Ala Leu Trp
160                 165                 170                 175

TGG GGG GTG GTC ACA GTC ACC ACC ATC GGC TAT GGG GAC AAG GTG CCC 574
Trp Gly Val Val Thr Val Thr Thr Ile Gly Tyr Gly Asp Lys Val Pro
                180                 185                 190

CAG ACG TGG GTC GGG AAG ACC ATC GCC TCC TGC TTC TCT GTC TTT GCC 622
Gln Thr Trp Val Gly Lys Thr Ile Ala Ser Cys Phe Ser Val Phe Ala
            195                 200                 205

ATC TCC TTC TTT GCG CTC CCA GCG GGG ATT CTT GGC TCG GGG TTT GCC 670
Ile Ser Phe Phe Ala Leu Pro Ala Gly Ile Leu Gly Ser Gly Phe Ala
        210                 215                 220

CTG AAG GTG CAG CAG AAG CAG AGG CAG AAG CAC TTC AAC CGG CAG ATC 718
Leu Lys Val Gln Gln Lys Gln Arg Gln Lys His Phe Asn Arg Gln Ile
    225                 230                 235

CCG GCG GCA GCC TCA CTC ATT CAG ACC GCA TGG AGG TGC TAT GCT GCC 766
Pro Ala Ala Ala Ser Leu Ile Gln Thr Ala Trp Arg Cys Tyr Ala Ala
240                 245                 250                 255

GAG AAC CCC GAC TCC TCC ACC TGG AAG ATC TAC ATC CGG AAG GCC CCC     814
Glu Asn Pro Asp Ser Ser Thr Trp Lys Ile Tyr Ile Arg Lys Ala Pro
                260                 265                 270

CGG AGC CAC ACT CTG CTG TCA CCC AGC CCC AAA CCC AAG AAG TCT GTG     862
Arg Ser His Thr Leu Leu Ser Pro Ser Pro Lys Pro Lys Lys Ser Val
            275                 280                 285

GTG GTA AAG AAA AAA AAG TTC AAG CTG GAC AAA GAC AAT GGG GTG ACT     910
Val Val Lys Lys Lys Lys Phe Lys Leu Asp Lys Asp Asn Gly Val Thr
        290                 295                 300

CCT GGA GAG AAG ATG CTC ACA GTC CCC CAT ATC ACG TGC GAC CCC CCA     958
Pro Gly Glu Lys Met Leu Thr Val Pro His Ile Thr Cys Asp Pro Pro
    305                 310                 315

GAA GAG CGG CGG CTG GAC CAC TTC TCT GTC GAC GGC TAT GAC AGT TCT     1006
Glu Glu Arg Arg Leu Asp His Phe Ser Val Asp Gly Tyr Asp Ser Ser
320                 325                 330                 335

GTA AGG AAG AGC CCA ACA CTG CTG GAA GTG AGC ATG CCC CAT TTC ATG     1054
Val Arg Lys Ser Pro Thr Leu Leu Glu Val Ser Met Pro His Phe Met
                340                 345                 350

AGA ACC AAC AGC TTC GCC GAG GAC CTG GAC CTG GAA GGG GAG ACT CTG     1102
Arg Thr Asn Ser Phe Ala Glu Asp Leu Asp Leu Glu Gly Glu Thr Leu
            355                 360                 365

CTG ACA CCC ATC ACC CAC ATC TCA CAG CTG CGG GAA CAC CAT CGG GCC     1150
Leu Thr Pro Ile Thr His Ile Ser Gln Leu Arg Glu His His Arg Ala
        370                 375                 380

ACC ATT AAG GTC ATT CGA CGC ATG CAG TAC TTT GTG GCC AAG AAG AAA     1198
Thr Ile Lys Val Ile Arg Arg Met Gln Tyr Phe Val Ala Lys Lys Lys
    385                 390                 395

TTC CAG CAA GCG CGG AAG CCT TAC GAT GTG CGG GAC GTC ATT GAG CAG     1246
Phe Gln Gln Ala Arg Lys Pro Tyr Asp Val Arg Asp Val Ile Glu Gln
400                 405                 410                 415

TAC TCG CAG GGC CAC CTC AAC CTC ATG GTG CGC ATC AAG GAG CTG CAG     1294
Tyr Ser Gln Gly His Leu Asn Leu Met Val Arg Ile Lys Glu Leu Gln
                420                 425                 430

AGG AGG CTG GAC CAG TCC ATT GGG AAG CCC TCA CTG TTC ATC TCC GTC     1342
Arg Arg Leu Asp Gln Ser Ile Gly Lys Pro Ser Leu Phe Ile Ser Val
            435                 440                 445

TCA GAA AAG AGC AAG GAT CGC GGC AGC AAC ACG ATC GGC GCC CGC CTG     1390
Ser Glu Lys Ser Lys Asp Arg Gly Ser Asn Thr Ile Gly Ala Arg Leu
        450                 455                 460

AAC CGA GTA GAA GAC AAG GTG ACG CAG CTG GAC CAG AGG CTG GCA CTC     1438
Asn Arg Val Glu Asp Lys Val Thr Gln Leu Asp Gln Arg Leu Ala Leu
    465                 470                 475

ATC ACC GAC ATG CTT CAC CAG CTG CTC TCC TTG CAC GGT GGC AGC ACC     1486
Ile Thr Asp Met Leu His Gln Leu Leu Ser Leu His Gly Gly Ser Thr
480                 485                 490                 495

CCC GGC AGC GGC GGC CCC CCC AGA GAG GGC GGG GCC CAC ATC ACC CAG     1534
Pro Gly Ser Gly Gly Pro Pro Arg Glu Gly Gly Ala His Ile Thr Gln
                500                 505                 510

CCC TGC GGC AGT GGC GGC TCC GTC GAC CCT GAG CTC TTC CTG CCC AGC     1582
Pro Cys Gly Ser Gly Gly Ser Val Asp Pro Glu Leu Phe Leu Pro Ser
            515                 520                 525

AAC ACC CTG CCC ACC TAC GAG CAG CTG ACC GTG CCC AGG AGG GGC CCC     1630
Asn Thr Leu Pro Thr Tyr Glu Gln Leu Thr Val Pro Arg Arg Gly Pro
        530                 535                 540

GAT GAG GGG TCC TGAGGAGGGG ATGGGGCTGG GGGATGGGCC TGAGTGAGAG         1682
Asp Glu Gly Ser
    545

GGGAGGCCAA GAGTGGCCCC ACCTGGCCCT CTCTGAAGGA GGCCACCTCC TAAAAGGCCC   1742

AGAGAGAAGA GCCCCACTCT CAGAGGCCCC AATACCCCAT GGACCATGCT GTCTGGCACA   1802

GCCTGCACTT GGGGGCTCAG CAAGGCCACC TCTTCCTGGC CGGTGTGGGG GCCCCGTCTC   1862

AGGTCTGAGT TGTTACCCCA AGCGCCCTGG CCCCCACATG GTGATGTTGA CATCACTGGC   1922

ATGGTGGTTG GGACCCAGTG GCAGGGCACA GGGCCTGGCC CATGTATGGC CAGGAAGTAG   1982

CACAGGCTGA GTGCAGGCCC ACCCTGCTTG GCCCAGGGGG CTTCCTGAGG GGAGACAGAG   2042

CAACCCCTGG ACCCCAGCCT CAAATCCAGG ACCCTGCCAG GCACAGGCAG GGCAGGACCA   2102

GCCCACGCTG ACTACAGGGC CACCGGCAAT AAAAGCCCAG GAGCCCATTT GGAGGGCCTG   2162

GGCCTGGCTC CCTCACTCTC AGGAAATGCT GACCCATGGG CAGGAGACTG TGGAGACTGC   2222

TCCTGAGCCC CCAGCTTCCA GCAGGAGGGA CAGTCTCACC ATTTCCCCAG GGCACGTGGT   2282

TGAGTGGGGG GAACGCCCAC TTCCCTGGGT TAGACTGCCA GCTCTTCCTA GCTGGAGAGG   2342

AGCCCTGCCT CTCCGCCCCT GAGCCCACTG TGCGTGGGGC TCCCGCCTCC AACCCCTCGC   2402

CCAGTCCCAG CAGCCAGCCA AACACACAGA AGGGGACTGC CACCTCCCCT TGCCAGCTGC   2462

TGAGCCGCAG AGAAGTGACG GTTCCTACAC AGGACAGGGG TTCCTTCTGG GCATTACATC   2522

GCATAGAAAT CAATAATTTG TGGTGATTTG GATCTGTGTT TTAATGAGTT TCACAGTGTG   2582

ATTTTGATTA TTAATTGTGC AAGCTTTTCC TAATAAACGT GGAGAATCAC A            2633

(2) INFORMATION FOR SEQ ID NO:16:

      (i) SEQUENCE CHARACTERISTICS:
            (A) LENGTH: 547 amino acids
            (B) TYPE: amino acid
            (D) TOPOLOGY: linear

     (ii) MOLECULE TYPE: protein

     (xi) SEQUENCE DESCRIPTION: SEQ ID NO:16:

Phe Leu Ile Val Leu Val Cys Leu Ile Phe Ser Val Leu Ser Thr Ile
1               5                   10                  15

Glu Gln Tyr Ala Ala Leu Ala Thr Gly Thr Leu Phe Trp Met Glu Ile
            20                  25                  30

Val Leu Val Val Phe Phe Gly Thr Glu Tyr Val Val Arg Leu Trp Ser
        35                  40                  45

Ala Gly Cys Arg Ser Lys Tyr Val Gly Leu Trp Gly Arg Leu Arg Phe
    50                  55                  60

Ala Arg Lys Pro Ile Ser Ile Ile Asp Leu Ile Val Val Val Ala Ser
65                  70                  75                  80

Met Val Val Leu Cys Val Gly Ser Lys Gly Gln Val Phe Ala Thr Ser
                85                  90                  95

Ala Ile Arg Gly Ile Arg Phe Leu Gln Ile Leu Arg Met Leu His Val
            100                 105                 110

Asp Arg Gln Gly Gly Thr Trp Arg Leu Leu Gly Ser Val Val Phe Ile
        115                 120                 125

His Arg Gln Glu Leu Ile Thr Thr Leu Tyr Ile Gly Phe Leu Gly Leu
    130                 135                 140

Ile Phe Ser Ser Tyr Phe Val Tyr Leu Ala Glu Lys Asp Ala Val Asn
145                 150                 155                 160

Glu Ser Gly Arg Val Glu Phe Gly Ser Tyr Ala Asp Ala Leu Trp Trp
                165                 170                 175

Gly Val Val Thr Val Thr Thr Ile Gly Tyr Gly Asp Lys Val Pro Gln
            180                 185                 190

Thr Trp Val Gly Lys Thr Ile Ala Ser Cys Phe Ser Val Phe Ala Ile
        195                 200                 205

Ser Phe Phe Ala Leu Pro Ala Gly Ile Leu Gly Ser Gly Phe Ala Leu
    210                 215                 220

Lys Val Gln Gln Lys Gln Arg Gln Lys His Phe Asn Arg Gln Ile Pro
225                 230                 235                 240

Ala Ala Ala Ser Leu Ile Gln Thr Ala Trp Arg Cys Tyr Ala Ala Glu
                245                 250                 255

Asn Pro Asp Ser Ser Thr Trp Lys Ile Tyr Ile Arg Lys Ala Pro Arg
            260                 265                 270

Ser His Thr Leu Leu Ser Pro Ser Pro Lys Pro Lys Lys Ser Val Val
        275                 280                 285

Val Lys Lys Lys Lys Phe Lys Leu Asp Lys Asp Asn Gly Val Thr Pro
    290                 295                 300

Gly Glu Lys Met Leu Thr Val Pro His Ile Thr Cys Asp Pro Pro Glu
305                 310                 315                 320

Glu Arg Arg Leu Asp His Phe Ser Val Asp Gly Tyr Asp Ser Ser Val
                325                 330                 335

Arg Lys Ser Pro Thr Leu Leu Glu Val Ser Met Pro His Phe Met Arg
            340                 345                 350

Thr Asn Ser Phe Ala Glu Asp Leu Asp Leu Glu Gly Glu Thr Leu Leu
        355                 360                 365

Thr Pro Ile Thr His Ile Ser Gln Leu Arg Glu His His Arg Ala Thr
    370                 375                 380

Ile Lys Val Ile Arg Arg Met Gln Tyr Phe Val Ala Lys Lys Lys Phe
385                 390                 395                 400

Gln Gln Ala Arg Lys Pro Tyr Asp Val Arg Asp Val Ile Glu Gln Tyr
                405                 410                 415

Ser Gln Gly His Leu Asn Leu Met Val Arg Ile Lys Glu Leu Gln Arg
            420                 425                 430

Arg Leu Asp Gln Ser Ile Gly Lys Pro Ser Leu Phe Ile Ser Val Ser
        435                 440                 445

Glu Lys Ser Lys Asp Arg Gly Ser Asn Thr Ile Gly Ala Arg Leu Asn
    450                 455                 460

Arg Val Glu Asp Lys Val Thr Gln Leu Asp Gln Arg Leu Ala Leu Ile
465                 470                 475                 480

Thr Asp Met Leu His Gln Leu Leu Ser Leu His Gly Gly Ser Thr Pro
                485                 490                 495

Gly Ser Gly Gly Pro Pro Arg Glu Gly Gly Ala His Ile Thr Gln Pro
            500                 505                 510

Cys Gly Ser Gly Gly Ser Val Asp Pro Glu Leu Phe Leu Pro Ser Asn
        515                 520                 525

Thr Leu Pro Thr Tyr Glu Gln Leu Thr Val Pro Arg Arg Gly Pro Asp
    530                 535                 540

Glu Gly Ser
545

(2) INFORMATION FOR SEQ ID NO:17:

     (i) SEQUENCE CHARACTERISTICS:
          (A) LENGTH: 26 amino acids
          (B) TYPE: amino acid
          (C) STRANDEDNESS: single
          (D) TOPOLOGY: linear

    (ii) MOLECULE TYPE: peptide

   (iii) HYPOTHETICAL: NO

(xi) SEQUENCE DESCRIPTION: SEQ ID NO:17:

Phe Leu Ile Val Leu Val Cys Leu Ile Phe Ser Val Leu Ser Thr Ile
1               5                   10                  15

Glu Gln Tyr Ala Ala Leu Ala Thr Gly Thr
            20                  25

(2) INFORMATION FOR SEQ ID NO:18:

(i) SEQUENCE CHARACTERISTICS:
(A) LENGTH: 61 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO:18:

Leu Phe Trp Met Glu Ile Val Leu Val Val Phe Phe Gly Thr Glu Tyr
1               5                   10                  15

Val Val Arg Leu Trp Ser Ala Gly Cys Arg Ser Lys Tyr Val Gly Leu
            20                  25                  30

Trp Gly Arg Leu Arg Phe Ala Arg Lys Pro Ile Ser Ile Ile Asp Leu
        35                  40                  45

Ile Val Val Val Ala Ser Met Val Val Leu Cys Val Gly
    50                  55                  60

(2) INFORMATION FOR SEQ ID NO:19:

(i) SEQUENCE CHARACTERISTICS:
(A) LENGTH: 137 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO:19:

Ser Lys Gly Gln Val Phe Ala Thr Ser Ala Ile Arg Gly Ile Arg Phe
1               5                   10                  15

Leu Gln Ile Leu Arg Met Leu His Val Asp Arg Gln Gly Gly Thr Trp
            20                  25                  30

Arg Leu Leu Gly Ser Val Val Phe Ile His Arg Gln Glu Leu Ile Thr
        35                  40                  45

Thr Leu Tyr Ile Gly Phe Leu Gly Leu Ile Phe Ser Ser Tyr Phe Val
    50                  55                  60

Tyr Leu Ala Glu Lys Asp Ala Val Asn Glu Ser Gly Arg Val Glu Phe
65                  70                  75                  80

Gly Ser Tyr Ala Asp Ala Leu Trp Trp Gly Val Val Thr Val Thr Thr
                85                  90                  95

Ile Gly Tyr Gly Asp Lys Val Pro Gln Thr Trp Val Gly Lys Thr Ile
            100                 105                 110

Ala Ser Cys Phe Ser Val Phe Ala Ile Ser Phe Phe Ala Leu Pro Ala
        115                 120                 125

Gly Ile Leu Gly Ser Gly Phe Ala Leu
    130                 135

(2) INFORMATION FOR SEQ ID NO:20:

(i) SEQUENCE CHARACTERISTICS:
(A) LENGTH: 66 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO:20:

Ile Leu Leu Ser Ile Val Ile Phe Cys Leu Glu Thr Leu Pro Glu Phe
1               5                   10                  15

Lys His Tyr Lys Val Phe Asn Thr Thr Thr Asn Gly Thr Lys Ile Glu
            20                  25                  30

Glu Asp Glu Val Pro Asp Ile Thr Asp Pro Phe Phe Leu Ile Glu Thr
        35                  40                  45

Leu Cys Ile Ile Trp Phe Thr Phe Glu Leu Thr Val Arg Phe Leu Ala
    50                  55                  60

Cys Pro
65

(2) INFORMATION FOR SEQ ID NO:21:

(i) SEQUENCE CHARACTERISTICS:
(A) LENGTH: 123 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO:21:

Asn Lys Leu Asn Phe Cys Arg Asp Val Met Asn Val Ile Asp Ile Ile
1               5                   10                  15

Ala Ile Ile Pro Tyr Phe Ile Thr Leu Ala Thr Val Val Ala Glu Glu
            20                  25                  30

Glu Asp Thr Leu Asn Leu Pro Lys Ala Pro Val Ser Pro Gln Asp Lys
        35                  40                  45

Ser Ser Asn Gln Ala Met Ser Leu Ala Ile Leu Arg Val Ile Arg Leu
    50                  55                  60

Val Arg Val Phe Arg Ile Phe Lys Leu Ser Arg His Ser Lys Gly Leu
65                  70                  75                  80

Gln Ile Leu Gly Arg Thr Leu Lys Ala Ser Met Arg Glu Leu Gly Leu
                85                  90                  95

Leu Ile Phe Phe Leu Phe Ile Gly Val Val Leu Phe Ser Ser Ala Val
            100                 105                 110

Tyr Phe Ala Glu Ala Gly Ser Glu Asn Ser Phe
        115                 120

(2) INFORMATION FOR SEQ ID NO:22:

(i) SEQUENCE CHARACTERISTICS:
(A) LENGTH: 58 amino acids
(B) TYPE: amino acid
(C) STRANDEDNESS: single
(D) TOPOLOGY: linear

(ii) MOLECULE TYPE: peptide

(iii) HYPOTHETICAL: NO

(xi) SEQUENCE DESCRIPTION: SEQ ID NO:22:

Phe Lys Ser Ile Pro Asp Ala Phe Trp Trp Ala Val Val Thr Met Thr
1               5                   10                  15

Thr Val Gly Tyr Gly Asp Met Thr Pro Val Gly Phe Trp Gly Lys Ile
            20                  25                  30

Val Gly Ser Leu Cys Val Val Ala Gly Val Leu Thr Ile Ala Leu Pro
        35                  40                  45

Val Pro Val Ile Val Ser Asn Phe Asn Tyr
    50                  55

(2) INFORMATION FOR SEQ ID NO:23:

(i) SEQUENCE CHARACTERISTICS:
(A) LENGTH: 376 amino acids
(B) TYPE: amino acid
(C) STRANDEDNESS: single
(D) TOPOLOGY: linear

(ii) MOLECULE TYPE: protein

(iii) HYPOTHETICAL: NO

(vi) ORIGINAL SOURCE:
(A) ORGANISM: Xenopus

(xi) SEQUENCE DESCRIPTION: SEQ ID NO:23:

Met Asn Glu Asn Ala Ile Asn Ser Leu Tyr Glu Ala Ile Pro Leu Pro
1               5                   10                  15

Gln Asp Gly Ser Ser Asn Gly Gln Arg Gln Glu Asp Arg Gln Ala Asn
            20                  25                  30

Ser Phe Glu Leu Lys Arg Glu Thr Leu Val Ala Thr Asp Pro Pro Arg
        35                  40                  45

Pro Thr Ile Asn Leu Asp Pro Arg Val Ser Ile Tyr Ser Gly Arg Arg
    50                  55                  60

Pro Leu Phe Ser Arg Thr Asn Ile Gln Gly Arg Val Tyr Asn Phe Leu
65                  70                  75                  80

Glu Arg Pro Thr Gly Trp Lys Cys Phe Val Tyr His Phe Thr Val Phe
                85                  90                  95

Leu Ile Val Leu Ile Cys Leu Ile Phe Ser Val Leu Ser Thr Ile Gln
            100                 105                 110

Gln Tyr Asn Asn Leu Ala Thr Glu Thr Leu Phe Trp Met Glu Ile Val
        115                 120                 125

Leu Val Val Phe Phe Gly Ala Glu Tyr Val Val Arg Leu Trp Ser Ala
    130                 135                 140

Gly Cys Arg Ser Lys Tyr Val Gly Val Trp Gly Arg Leu Arg Phe Ala
145                 150                 155                 160

Arg Lys Pro Ile Ser Val Ile Asp Leu Ile Val Val Val Ala Ser Val
                165                 170                 175

Ile Val Leu Cys Val Gly Ser Asn Gly Gln Val Phe Ala Thr Ser Ala
            180                 185                 190

Ile Arg Gly Ile Arg Phe Leu Gln Ile Leu Arg Met Leu His Val Asp
        195                 200                 205

Arg Gln Gly Gly Thr Trp Arg Leu Leu Gly Ser Val Val Phe Ile His
    210                 215                 220

Arg Gln Glu Leu Ile Thr Thr Leu Tyr Ile Gly Phe Leu Gly Leu Ile
225                 230                 235                 240

Phe Ser Ser Tyr Phe Val Tyr Leu Ala Glu Lys Asp Ala Ile Asp Ser
                245                 250                 255

Ser Gly Glu Tyr Gln Phe Gly Ser Tyr Ala Asp Ala Leu Trp Trp Gly
            260                 265                 270

Val Val Thr Val Thr Thr Ile Gly Tyr Gly Asp Lys Val Pro Gln Thr
        275                 280                 285

Trp Ile Gly Lys Thr Ile Ala Ser Cys Phe Ser Val Phe Ala Ile Ser
    290                 295                 300

Phe Phe Ala Leu Pro Ala Gly Ile Leu Gly Ser Gly Phe Ala Leu Lys
305                 310                 315                 320

Val Gln Gln Lys Gln Arg Gln Lys His Phe Asn Arg Gln Ile Pro Ala
                325                 330                 335

Ala Ala Ser Leu Ile Gln Thr Ala Trp Arg Cys Tyr Ala Ala Glu Asn
            340                 345                 350

Pro Asp Ser Ala Thr Trp Lys Ile Tyr Ile Arg Lys Gln Ser Arg Asn
        355                 360                 365

His His Ile Met Ser Pro Ser Pro
    370                 375

(2) INFORMATION FOR SEQ ID NO:24:

    (i) SEQUENCE CHARACTERISTICS:
        (A) LENGTH: 581 amino acids
        (B) TYPE: amino acid
        (C) STRANDEDNESS: single
        (D) TOPOLOGY: linear

    (ii) MOLECULE TYPE: protein

    (iii) HYPOTHETICAL: NO

    (vi) ORIGINAL SOURCE:
        (A) ORGANISM: Homo sapiens

    (xi) SEQUENCE DESCRIPTION: SEQ ID NO:24:

Met Glu Thr Arg Gly Ser Arg Leu Thr Gly Gly Gln Gly Arg Val Tyr
1               5                   10                  15

Asn Phe Leu Glu Arg Pro Thr Gly Trp Lys Cys Phe Val Tyr His Phe
            20                  25                  30

Ala Val Phe Leu Ile Val Leu Val Cys Leu Ile Phe Ser Val Leu Ser
        35                  40                  45

Thr Ile Glu Gln Tyr Ala Ala Leu Ala Thr Gly Thr Leu Phe Trp Met
    50                  55                  60

Glu Ile Val Leu Val Val Phe Phe Gly Thr Glu Tyr Val Val Arg Leu
65                  70                  75                  80

Trp Ser Ala Gly Cys Arg Ser Lys Tyr Val Gly Leu Trp Gly Arg Leu
                85                  90                  95

Arg Phe Ala Arg Lys Pro Ile Ser Ile Ile Asp Leu Ile Val Val Val
            100                 105                 110

Ala Ser Met Val Val Leu Cys Val Gly Ser Lys Gly Gln Val Phe Ala
        115                 120                 125

Thr Ser Ala Ile Arg Gly Ile Arg Phe Leu Gln Ile Leu Arg Met Leu
    130                 135                 140

His Val Asp Arg Gln Gly Gly Thr Trp Arg Leu Leu Gly Ser Val Val
145                 150                 155                 160

Phe Ile His Arg Gln Glu Leu Ile Thr Thr Leu Tyr Ile Gly Phe Leu
                165                 170                 175

Gly Leu Ile Phe Ser Ser Tyr Phe Val Tyr Leu Ala Glu Lys Asp Ala
            180                 185                 190

Val Asn Glu Ser Gly Arg Val Glu Phe Gly Ser Tyr Ala Asp Ala Leu
        195                 200                 205

Trp Trp Gly Val Val Thr Val Thr Thr Ile Gly Tyr Gly Asp Lys Val
    210                 215                 220

Pro Gln Thr Trp Val Gly Lys Thr Ile Ala Ser Cys Phe Ser Val Phe
225                 230                 235                 240

Ala Ile Ser Phe Phe Ala Leu Pro Ala Gly Ile Leu Gly Ser Gly Phe
                245                 250                 255

Ala Leu Lys Val Gln Gln Lys Gln Arg Gln Lys His Phe Asn Arg Gln
            260                 265                 270

Ile Pro Ala Ala Ala Ser Leu Ile Gln Thr Ala Trp Arg Cys Tyr Ala
        275                 280                 285

Ala Glu Asn Pro Asp Ser Ser Thr Trp Lys Ile Tyr Ile Arg Lys Ala
    290                 295                 300

Pro Arg Ser His Thr Leu Leu Ser Pro Ser Pro Lys Pro Lys Lys Ser
305                 310                 315                 320

Val Val Val Lys Lys Lys Lys Phe Lys Leu Asp Lys Asp Asn Gly Val
                325                 330                 335

Thr Pro Gly Glu Lys Met Leu Thr Val Pro His Ile Thr Cys Asp Pro
            340                 345                 350

Pro Glu Glu Arg Arg Leu Asp His Phe Ser Val Asp Gly Tyr Asp Ser
        355                 360                 365

Ser Val Arg Lys Ser Pro Thr Leu Leu Glu Val Ser Met Pro His Phe
    370                 375                 380

Met Arg Thr Asn Ser Phe Ala Glu Asp Leu Asp Leu Glu Gly Glu Thr
385                 390                 395                 400

Leu Leu Thr Pro Ile Thr His Ile Ser Gln Leu Arg Glu His His Arg
                405                 410                 415

Ala Thr Ile Lys Val Ile Arg Arg Met Gln Tyr Phe Val Ala Lys Lys
            420                 425                 430

Lys Phe Gln Gln Ala Arg Lys Pro Tyr Asp Val Arg Asp Val Ile Glu
        435                 440                 445

Gln Tyr Ser Gln Gly His Leu Asn Leu Met Arg Val Ile Lys Glu Leu
    450                 455                 460

Gln Arg Arg Leu Asp Gln Ser Ile Gly Lys Pro Ser Leu Phe Ile Ser
465                 470                 475                 480

Val Ser Glu Lys Ser Lys Asp Arg Gly Ser Asn Thr Ile Gly Ala Arg
                485                 490                 495

Leu Asn Arg Val Glu Asp Lys Val Thr Gln Leu Asp Gln Arg Leu Ala
            500                 505                 510

Leu Ile Thr Asp Met Leu His Gln Leu Leu Ser Leu His Gly Gly Ser
        515                 520                 525

Thr Pro Gly Ser Gly Gly Pro Pro Arg Glu Gly Gly Ala His Ile Thr
    530                 535                 540

Gln Pro Cys Gly Ser Gly Gly Ser Val Asp Pro Glu Leu Phe Leu Pro
545                 550                 555                 560

Ser Asn Thr Leu Pro Thr Tyr Glu Gln Leu Thr Val Pro Arg Arg Gly
                565                 570                 575

Pro Asp Glu Gly Ser
            580

(2) INFORMATION FOR SEQ ID NO:25:

    (i) SEQUENCE CHARACTERISTICS:
        (A) LENGTH: 2821 base pairs
        (B) TYPE: nucleic acid
        (C) STRANDEDNESS: double
        (D) TOPOLOGY: linear

   (ii) MOLECULE TYPE: DNA (genomic)

  (iii) HYPOTHETICAL: NO

   (iv) ANTI-SENSE: NO

   (vi) ORIGINAL SOURCE:
        (A) ORGANISM: Homo sapiens

   (ix) FEATURE:
        (A) NAME/KEY: CDS
        (B) LOCATION: 88..1830

(xi) SEQUENCE DESCRIPTION: SEQ ID NO:25:

GGCTTCCTCG AGCGTCCCAC CGGCTGGAAG TTGTAGACGC GGCCCTGGAC GTGGGTGCGC 60

GCCAACACCG GGCGGCGCGT GCTGTAG ATG GAG ACG CGC GGG TCT AGG CTC 111
Met Glu Thr Arg Gly Ser Arg Leu
1               5

ACC GGC GGC CAG GGC CGC GTC TAC AAC TTC CTC GAG CGT CCC ACC GGC 159
Thr Gly Gly Gln Gly Arg Val Tyr Asn Phe Leu Glu Arg Pro Thr Gly
    10                  15                  20

TGG AAA TGC TTC GTT TAC CAC TTC GCC GTC TTC CTC ATC GTC CTG GTC 207
Trp Lys Cys Phe Val Tyr His Phe Ala Val Phe Leu Ile Val Leu Val
25                  30                  35                  40

TGC CTC ATC TTC AGC GTG CTG TCC ACC ATC GAG CAG TAT GCC GCC CTG 255
Cys Leu Ile Phe Ser Val Leu Ser Thr Ile Glu Gln Tyr Ala Ala Leu
                45                  50                  55

GCC ACG GGG ACT CTC TTC TGG ATG GAG ATC GTG CTG GTG GTG TTC TTC 303
Ala Thr Gly Thr Leu Phe Trp Met Glu Ile Val Leu Val Val Phe Phe
            60                  65                  70

GGG ACG GAG TAC GTG GTC CGC CTC TGG TCC GCC GGC TGC CGC AGC AAG 351
Gly Thr Glu Tyr Val Val Arg Leu Trp Ser Ala Gly Cys Arg Ser Lys
        75                  80                  85

TAC GTG GGC CTC TGG GGG CGG CTG CGC TTT GCC CGG AAG CCC ATT TCC 399
Tyr Val Gly Leu Trp Gly Arg Leu Arg Phe Ala Arg Lys Pro Ile Ser
    90                  95                  100

ATC ATC GAC CTC ATC GTG GTC GTG GCC TCC ATG GTG GTC CTC TGC GTG 447
Ile Ile Asp Leu Ile Val Val Val Ala Ser Met Val Val Leu Cys Val
105                 110                 115                 120

GGC TCC AAG GGG CAG GTG TTT GCC ACG TCG GCC ATC AGG GGC ATC CGC 495
Gly Ser Lys Gly Gln Val Phe Ala Thr Ser Ala Ile Arg Gly Ile Arg
                125                 130                 135

TTC CTG CAG ATC CTG AGG ATG CTA CAC GTC GAC CGC CAG GGA GGC ACC 543
Phe Leu Gln Ile Leu Arg Met Leu His Val Asp Arg Gln Gly Gly Thr
            140                 145                 150

TGG AGG CTC CTG GGC TCC GTG GTC TTC ATC CAC CGC CAG GAG CTG ATA 591
Trp Arg Leu Leu Gly Ser Val Val Phe Ile His Arg Gln Glu Leu Ile
        155                 160                 165

ACC ACC CTG TAC ATC GGC TTC CTG GGC CTC ATC TTC TCC TCG TAC TTT 639
Thr Thr Leu Tyr Ile Gly Phe Leu Gly Leu Ile Phe Ser Ser Tyr Phe
    170                 175                 180

GTG TAC CTG GCT GAG AAG GAC GCG GTG AAC GAG TCA GGC CGC GTG GAG 687
Val Tyr Leu Ala Glu Lys Asp Ala Val Asn Glu Ser Gly Arg Val Glu
185                 190                 195                 200

TTC GGC AGC TAC GCA GAT GCG CTG TGG TGG GGG GTG GTC ACA GTC ACC 735
Phe Gly Ser Tyr Ala Asp Ala Leu Trp Trp Gly Val Val Thr Val Thr
                205                 210                 215

ACC ATC GGC TAT GGG GAC AAG GTG CCC CAG ACG TGG GTC GGG AAG ACC 783
Thr Ile Gly Tyr Gly Asp Lys Val Pro Gln Thr Trp Val Gly Lys Thr
            220                 225                 230

ATC GCC TCC TGC TTC TCT GTC TTT GCC ATC TCC TTC TTT GCG CTC CCA 831
Ile Ala Ser Cys Phe Ser Val Phe Ala Ile Ser Phe Phe Ala Leu Pro
        235                 240                 245

GCG GGG ATT CTT GGC TCG GGG TTT GCC CTG AAG GTG CAG CAG AAG CAG 879
Ala Gly Ile Leu Gly Ser Gly Phe Ala Leu Lys Val Gln Gln Lys Gln
    250                 255                 260

AGG CAG AAG CAC TTC AAC CGG CAG ATC CCG GCG GCA GCC TCA CTC ATT 927
Arg Gln Lys His Phe Asn Arg Gln Ile Pro Ala Ala Ala Ser Leu Ile
265                 270                 275                 280

CAG ACC GCA TGG AGG TGC TAT GCT GCC GAG AAC CCC GAC TCC TCC ACC 975
Gln Thr Ala Trp Arg Cys Tyr Ala Ala Glu Asn Pro Asp Ser Ser Thr
                285                 290                 295

TGG AAG ATC TAC ATC CGG AAG GCC CCC CGG AGC CAC ACT CTG CTG TCA 1023
Trp Lys Ile Tyr Ile Arg Lys Ala Pro Arg Ser His Thr Leu Leu Ser
            300                 305                 310

CCC AGC CCC AAA CCC AAG AAG TCT GTG GTG GTA AAG AAA AAA AAG TTC 1071
Pro Ser Pro Lys Pro Lys Lys Ser Val Val Val Lys Lys Lys Lys Phe
        315                 320                 325

AAG CTG GAC AAA GAC AAT GGG GTG ACT CCT GGA GAG AAG ATG CTC ACA 1119
Lys Leu Asp Lys Asp Asn Gly Val Thr Pro Gly Glu Lys Met Leu Thr
    330                 335                 340

GTC CCC CAT ATC ACG TGC GAC CCC CCA GAA GAG CGG CGG CTG GAC CAC 1167
Val Pro His Ile Thr Cys Asp Pro Pro Glu Glu Arg Arg Leu Asp His
345                 350                 355                 360

TTC TCT GTC GAC GGC TAT GAC AGT TCT GTA AGG AAG AGC CCA ACA CTG 1215
Phe Ser Val Asp Gly Tyr Asp Ser Ser Val Arg Lys Ser Pro Thr Leu
                365                 370                 375

CTG GAA GTG AGC ATG CCC CAT TTC ATG AGA ACC AAC AGC TTC GCC GAG 1263
Leu Glu Val Ser Met Pro His Phe Met Arg Thr Asn Ser Phe Ala Glu
            380                 385                 390

GAC CTG GAC CTG GAA GGG GAG ACT CTG CTG ACA CCC ATC ACC CAC ATC 1311
Asp Leu Asp Leu Glu Gly Glu Thr Leu Leu Thr Pro Ile Thr His Ile
        395                 400                 405

TCA CAG CTG CGG GAA CAC CAT CGG GCC ACC ATT AAG GTC ATT CGA CGC 1359
Ser Gln Leu Arg Glu His His Arg Ala Thr Ile Lys Val Ile Arg Arg
    410                 415                 420

ATG CAG TAC TTT GTG GCC AAG AAG AAA TTC CAG CAA GCG CGG AAG CCT 1407
Met Gln Tyr Phe Val Ala Lys Lys Lys Phe Gln Gln Ala Arg Lys Pro
425                 430                 435                 440

TAC GAT GTG CGG GAC GTC ATT GAG CAG TAC TCG CAG GGC CAC CTC AAC 1455
Tyr Asp Val Arg Asp Val Ile Glu Gln Tyr Ser Gln Gly His Leu Asn
                445                 450                 455

CTC ATG GTG CGC ATC AAG GAG CTG CAG AGG AGG CTG GAC CAG TCC ATT 1503
Leu Met Val Arg Ile Lys Glu Leu Gln Arg Arg Leu Asp Gln Ser Ile
            460                 465                 470

GGG AAG CCC TCA CTG TTC ATC TCC GTC TCA GAA AAG AGC AAG GAT CGC 1551
Gly Lys Pro Ser Leu Phe Ile Ser Val Ser Glu Lys Ser Lys Asp Arg
        475                 480                 485

GGC AGC AAC ACG ATC GGC GCC CGC CTG AAC CGA GTA GAA GAC AAG GTG 1599
Gly Ser Asn Thr Ile Gly Ala Arg Leu Asn Arg Val Glu Asp Lys Val
    490                 495                 500

ACG CAG CTG GAC CAG AGG CTG GCA CTC ATC ACC GAC ATG CTT CAC CAG 1647
Thr Gln Leu Asp Gln Arg Leu Ala Leu Ile Thr Asp Met Leu His Gln
505                 510                 515                 520

CTG CTC TCC TTG CAC GGT GGC AGC ACC CCC GGC AGC GGC GGC CCC CCC 1695
Leu Leu Ser Leu His Gly Gly Ser Thr Pro Gly Ser Gly Gly Pro Pro
                525                 530                 535

AGA GAG GGC GGG GCC CAC ATC ACC CAG CCC TGC GGC AGT GGC GGC TCC 1743
Arg Glu Gly Gly Ala His Ile Thr Gln Pro Cys Gly Ser Gly Gly Ser
            540                 545                 550

GTC GAC CCT GAG CTC TTC CTG CCC AGC AAC ACC CTG CCC ACC TAC GAG 1791
Val Asp Pro Glu Leu Phe Leu Pro Ser Asn Thr Leu Pro Thr Tyr Glu
        555                 560                 565

CAG CTG ACC GTG CCC AGG AGG GGC CCC GAT GAG GGG TCC TGAGGAGGGG 1840
Gln Leu Thr Val Pro Arg Arg Gly Pro Asp Glu Gly Ser
    570                 575                 580

ATGGGGCTGG GGGATGGGCC TGAGTGAGAG GGGAGGCCAA GAGTGGCCCC ACCTGGCCCT 1900

CTCTGAAGGA GGCCACCTCC TAAAAGGCCC AGAGAGAAGA GCCCCACTCT CAGAGGCCCC 1960

AATACCCCAT GGACCATGCT GTCTGGCACA GCCTGCACTT GGGGGCTCAG CAAGGCCACC 2020

TCTTCCTGGC CGGTGTGGGG GCCCCGTCTC AGGTCTGAGT TGTTACCCCA AGCGCCCTGG 2080

CCCCCACATG GTGATGTTGA CATCACTGGC ATGGTGGTTG GGACCCAGTG GCAGGGCACA 2140

GGGCCTGGCC CATGTATGGC CAGGAAGTAG CACAGGCTGA GTGCAGGCCC ACCCTGCTTG 2200

GCCCAGGGGG CTTCCTGAGG GGAGACAGAG CAACCCCTGG ACCCCAGCCT CAAATCCAGG 2260

ACCCTGCCAG GCACAGGCAG GGCAGGACCA GCCCACGCTG ACTACAGGGC CACCGGCAAT 2320

AAAAGCCCAG GAGCCCATTT GGAGGGCCTG GGCCTGGCTC CCTCACTCTC AGGAAATGCT 2380

GACCCATGGG CAGGAGACTG TGGAGACTGC TCCTGAGCCC CCAGCTTCCA GCAGGAGGGA 2440

CAGTCTCACC ATTTCCCCAG GGCACGTGGT TGAGTGGGGG GAACGCCCAC TTCCCTGGGT 2500

TAGACTGCCA GCTCTTCCTA GCTGGAGAGG AGCCCTGCCT CTCCGCCCCT GAGCCCACTG 2560

TGCGTGGGGC TCCCGCCTCC AACCCCTCGC CCAGTCCCAG CAGCCAGCCA AACACACAGA 2620

AGGGGACTGC CACCTCCCCT TGCCAGCTGC TGAGCCGCAG AGAAGTGACG GTTCCTACAC 2680

AGGACAGGGG TTCCTTCTGG GCATTACATC GCATAGAAAT CAATAATTTG TGGTGATTTG 2740

GATCTGTGTT TTAATGAGTT TCACAGTGTG ATTTTGATTA TTAATTGTGC AAGCTTTTCC 2800

TAATAAACGT GGAGAATCAC A 2821

(2) INFORMATION FOR SEQ ID NO:26:

(i) SEQUENCE CHARACTERISTICS:
(A) LENGTH: 581 amino acids
(B) TYPE: amino acid
(D) TOPOLOGY: linear

(ii) MOLECULE TYPE: protein

(xi) SEQUENCE DESCRIPTION: SEQ ID NO:26:

Met Glu Thr Arg Gly Ser Arg Leu Thr Gly Gly Gln Gly Arg Val Tyr
1               5                   10                  15

Asn Phe Leu Glu Arg Pro Thr Gly Trp Lys Cys Phe Val Tyr His Phe
            20                  25                  30

Ala Val Phe Leu Ile Val Leu Val Cys Leu Ile Phe Ser Val Leu Ser
        35                  40                  45

Thr Ile Glu Gln Tyr Ala Ala Leu Ala Thr Gly Thr Leu Phe Trp Met
    50                  55                  60

Glu Ile Val Leu Val Val Phe Phe Gly Thr Glu Tyr Val Val Arg Leu
65                  70                  75                  80

Trp Ser Ala Gly Cys Arg Ser Lys Tyr Val Gly Leu Trp Gly Arg Leu
                85                  90                  95

Arg Phe Ala Arg Lys Pro Ile Ser Ile Ile Asp Leu Ile Val Val Val
            100                 105                 110

Ala Ser Met Val Val Leu Cys Val Gly Ser Lys Gly Gln Val Phe Ala
        115                 120                 125

Thr Ser Ala Ile Arg Gly Ile Arg Phe Leu Gln Ile Leu Arg Met Leu
    130                 135                 140

His Val Asp Arg Gln Gly Gly Thr Trp Arg Leu Leu Gly Ser Val Val
145                 150                 155                 160

Phe Ile His Arg Gln Glu Leu Ile Thr Thr Leu Tyr Ile Gly Phe Leu
                165                 170                 175

Gly Leu Ile Phe Ser Ser Tyr Phe Val Tyr Leu Ala Glu Lys Asp Ala
            180                 185                 190

Val Asn Glu Ser Gly Arg Val Glu Phe Gly Ser Tyr Ala Asp Ala Leu
        195                 200                 205

Trp Trp Gly Val Val Thr Val Thr Thr Ile Gly Tyr Gly Asp Lys Val
    210                 215                 220

Pro Gln Thr Trp Val Gly Lys Thr Ile Ala Ser Cys Phe Ser Val Phe
225                 230                 235                 240

Ala Ile Ser Phe Phe Ala Leu Pro Ala Gly Ile Leu Gly Ser Gly Phe
                245                 250                 255

Ala Leu Lys Val Gln Gln Lys Gln Arg Gln Lys His Phe Asn Arg Gln
            260                 265                 270

Ile Pro Ala Ala Ala Ser Leu Ile Gln Thr Ala Trp Arg Cys Tyr Ala
        275                 280                 285

Ala Glu Asn Pro Asp Ser Ser Thr Trp Lys Ile Tyr Ile Arg Lys Ala
    290                 295                 300

Pro Arg Ser His Thr Leu Leu Ser Pro Ser Pro Lys Pro Lys Lys Ser
305                 310                 315                 320

Val Val Val Lys Lys Lys Lys Phe Lys Leu Asp Lys Asp Asn Gly Val
                325                 330                 335

Thr Pro Gly Glu Lys Met Leu Thr Val Pro His Ile Thr Cys Asp Pro
            340                 345                 350

Pro Glu Glu Arg Arg Leu Asp His Phe Ser Val Asp Gly Tyr Asp Ser
        355                 360                 365

Ser Val Arg Lys Ser Pro Thr Leu Leu Glu Val Ser Met Pro His Phe
    370                 375                 380

Met Arg Thr Asn Ser Phe Ala Glu Asp Leu Asp Leu Glu Gly Glu Thr
385                 390                 395                 400

Leu Leu Thr Pro Ile Thr His Ile Ser Gln Leu Arg Glu His His Arg
                405                 410                 415

Ala Thr Ile Lys Val Ile Arg Arg Met Gln Tyr Phe Val Ala Lys Lys
            420                 425                 430

Lys Phe Gln Gln Ala Arg Lys Pro Tyr Asp Val Arg Asp Val Ile Glu
        435                 440                 445

Gln Tyr Ser Gln Gly His Leu Asn Leu Met Val Arg Ile Lys Glu Leu
    450                 455                 460

Gln Arg Arg Leu Asp Gln Ser Ile Gly Lys Pro Ser Leu Phe Ile Ser
465                 470                 475                 480

Val Ser Glu Lys Ser Lys Asp Arg Gly Ser Asn Thr Ile Gly Ala Arg
                485                 490                 495

Leu Asn Arg Val Glu Asp Lys Val Thr Gln Leu Asp Gln Arg Leu Ala
            500                 505                 510

Leu Ile Thr Asp Met Leu His Gln Leu Leu Ser Leu His Gly Gly Ser
        515                 520                 525

Thr Pro Gly Ser Gly Gly Pro Pro Arg Glu Gly Gly Ala His Ile Thr
    530                 535                 540

Gln Pro Cys Gly Ser Gly Gly Ser Val Asp Pro Glu Leu Phe Leu Pro
545                 550                 555                 560

Ser Asn Thr Leu Pro Thr Tyr Glu Gln Leu Thr Val Pro Arg Arg Gly
                565                 570                 575

Pro Asp Glu Gly Ser
            580

## Description

This application was made with Government support under Grant Nos. R01 HL48074, funded by the National Institutes of Health, Bethesda, Maryland, and Grant No. M01 RR00064 from the U.S. Public Health Service.

### BACKGROUND OF THE INVENTION

The present invention is directed to a gene and gene products associated with long QT syndrome (LQT) and to a process for the diagnosis and prevention of LQT. LQT is diagnosed in accordance with the present invention by analyzing the DNA sequence of the *KVLQT1* gene of an individual to be tested and comparing the respective DNA sequence to the known DNA sequence of a normal *KVLQT1* gene. Alternatively, the *KVLQT1* gene of an individual to be tested can be screened for mutations which cause LQT. Prediction of LQT will enable practitioners to prevent this disorder using existing medical therapy. This invention is further directed to the discovery that the KVLQT1 and minK proteins coassemble to form a cardiac I_{Ks} potassium channel. This knowledge can be used to coexpress these two proteins in a cell and such a transformed cell can be used for screening for drugs which will be useful in treating or preventing LQT.

The publications and other materials used herein to illuminate the background of the invention or provide additional details respecting the practice, are incorporated by reference, and for convenience are respectively grouped in the appended List of References.

Cardiac arrhythmias are a common cause of morbidity and mortality, accounting for approximately 11% of all natural deaths (Kannel, 1987; Willich et al., 1987). In general, presymptomatic diagnosis and treatment of individuals with life-threatening ventricular tachyarrhythmias is poor, and in some cases medical management actually increases the risk of arrhythmia and death (New Engl. J. Med. 327, 227 (1992)). These factors make early detection of individuals at risk for cardiac arrhythmias and arrhythmia prevention high priorities.

Both genetic and acquired factors contribute to the risk of developing cardiac arrhythmias. Long QT syndrome (LQT) is an inherited cardiac arrhythmia that causes abrupt loss of consciousness, syncope, seizures and sudden death from ventricular tachyarrhythmias, specifically *torsade de pointes* and ventricular fibrillation (Ward, 1964; Romano, 1965; Schwartz et al., 1975; Moss et al., 1991). This disorder usually occurs in young, otherwise healthy individuals (Ward, 1964; Romano, 1965; Schwartz, 1975). Most LQT gene carriers manifest prolongation of the QT interval on electrocardiograms, a sign of abnormal cardiac repolarization (Vincent et al., 1992). The clinical features of LQT result from episodic cardiac arrhythmias, specifically repolarization-related ventricular tachyarrhythmias like *torsade de pointes,* named for the characteristic undulating nature of the electrocardiogram in this arrhythmia and ventricular fibrillation (Schwartz et al., 1975; Moss and McDonald, 1970). *Torsade de pointes* may degenerate into ventricular fibrillation, a particularly lethal arrhythmia. Although LQT is not a common diagnosis, ventricular arrhythmias are very common; more than 300,000 United States citizens die suddenly every year (Kannel, et al., 1987; Willich et al., 1987) and, in many cases, the underlying mechanism may be aberrant cardiac repolarization. LQT, therefore, provides a unique opportunity to study life-threatening cardiac arrhythmias at the molecular level.

Both inherited and acquired forms of LQT have been defined. Acquired LQT and secondary arrhythmias can result from cardiac ischemia, bradycardia and metabolic abnormalities such as low serum potassium or calcium concentration (Zipes, 1987). LQT can also result from treatment with certain medications, including antibiotics, antihistamines, general anesthetics, and, most commonly, antiarrhythmic medications (Zipes, 1987). Inherited forms of LQT can result from mutations in at least three different genes. In previous studies, LQT loci were mapped to chromosome 11p15.5 (*LQT1*) (Keating et al., 1991 a; Keating et al., 1991 b), 7q35-36 (*LQT2*) and 3p21-24 (*LQT3*) (Jiang et al., 1994). Of these, the most common cause of inherited LQT is *LQT1.* Our data indicate that mutations in this gene are responsible for more than 50% of inherited LQT (Q. Wang, unpublished results). Recently, a fourth LQT locus (*LQT4*) was mapped to 4q25-27 (Schott et al., 1995). The present work indicates that *minK,* a gene located on chromosome 21, is also involved in LQT.

Autosomal dominant and autosomal recessive forms of this disorder have been reported. Autosomal recessive LQT (also known as Jervell-Lange-Nielson syndrome) has been associated with congenital neural deafness; this form of LQT is rare (Jervell and Lange-Nielson, 1957). Autosomal dominant LQT (Romano-Ward syndrome) is more common, and is not associated with other phenotypic abnormalities. A disorder very similar to inherited LQT can also be acquired, usually as a result of pharmacologic therapy (Schwartz et al., 1975; Zipes, 1987).

The data have implications for the mechanism of arrhythmias in LQT. Two hypotheses for LQT have previously been proposed (Schwartz et al., 1994). One suggests that a predominance of left autonomic innervation causes abnormal cardiac repolarization and arrhythmias. This hypothesis is supported by the finding that arrhythmias can be induced in dogs by removal of the right stellate ganglion. In addition, anecdotal evidence suggests that some LQT patients are effectively treated by β-adrenergic blocking agents and by left stellate ganglionectomy (Schwartz et al., 1994). The second hypothesis for LQT-related arrhythmias suggests that mutations in cardiac-specific ion channel genes, or genes that modulate cardiac ion channels, cause delayed myocellular repolarization. Delayed myocellular repolarization could promote reactivation of L-type calcium channels, resulting in secondary depolarizations (January and Riddle, 1989). These secondary depolarizations are the likely cellular mechanism of *torsade de pointes* arrhythmias (Surawicz, 1989). This hypothesis is supported by the observation that pharmacologic block of potassium channels can induce QT prolongation and repolarization-related arrhythmias in humans and animal models (Antzelevitch and Sicouri, 1994). The discovery that one form of LQT results from mutations in a cardiac potassium channel gene supports the myocellular hypothesis.

In 1991, the complete linkage between autosomal dominant LQT and a polymorphism at HRAS was reported (Keating et al., 1991a; Keating et al., 1991b). This discovery localized *LQT1* to chromosome 11p15.5 and made presymptomatic diagnosis possible in some families. Autosomal dominant LQT was previously thought to be genetically homogeneous, and the first seven families that were studied were linked to 11p15.5 (Keating et al., 1991b). In 1993, it was found that there was locus heterogeneity for LQT (Benhorin et al., 1993; Curran et al., 1993; Towbin et al., 1994). Two additional LQT loci were subsequently identified, *LQT2* on chromosome 7q35-36 (nine families) and *LQT3* on 3p21-24 (three families) (Jiang et al., 1994). Several families remain unlinked to the known loci, indicating additional locus heterogeneity for LQT. This degree of heterogeneity suggests that distinct LQT genes may encode proteins that interact to modulate cardiac repolarization and arrhythmia risk.

Although little is known about the physiology of LQT, the disorder is associated with prolongation of the QT interval on electrocardiograms, a sign of abnormal cardiac repolarization. This association suggests that genes encoding ion channels, or their modulators, are reasonable candidates for LQT. HRAS, which was localized to chromosome 11p15.5, was excluded as a candidate for *LQT1* based on direct DNA sequence analyses (unpublished observations) and by linkage analyses (Roy et al., 1994). A neuroendocrine calcium channel gene (*CACNL1A2 ;* Chin et al., 1991; Seino et al., 1992) and a gene encoding a GTP-binding protein that modulates potassium channels (*GNAI2;* Weinstein et al., 1988; Magovcevic et al., 1992) became candidates for *LQT3* based on their chromosomal location. Subsequent linkage analyses, however, have excluded these genes (Wang and Keating, unpublished data). A skeletal muscle chloride channel (*CLCN1;* Koch et al., 1992) and a cardiac muscarinic-acetylcholine receptor *(CHRM2;* Bonner et al., 1987) became candidates for *LQT2* based on their chromosome 7q35-36 location, but subsequent linkage analyses have excluded these genes (Wang et al., submitted).

In theory, mutations in a cardiac sodium channel gene could cause LQT. Voltage-gated sodium channels mediate rapid depolarization in ventricular myocytes, and also conduct a small current during the plateau phase of the action potential (Attwell et al., 1979). Subtle abnormalities of sodium channel function (e.g., delayed sodium channel inactivation or altered voltage-dependence of channel inactivation) could delay cardiac repolarization, leading to QT prolongation and arrhythmias. In 1992, Gellens and colleagues cloned and characterized a cardiac sodium channel gene, *SCN5A* (Gellens et al., 1992). The structure of this gene was similar to other, previously characterized sodium channels, encoding a large protein of 2016 amino acids. These channel proteins contain four homologous domains (DI-DIV), each of which contains six putative membrane spanning segments (S1-S6). *SCN5A* was recently mapped to chromosome 3p21, making it an excellent candidate gene for *LQT3* (George et al., 1995), and this gene was then proved to be associated with LQT3 (Wang et al., 1995a).

In 1994, Warmke and Ganetzky identified a novel human cDNA, human ether a-go-go related gene (*HERG,* Warmke and Ganetzky, 1994). *HERG* was localized to human chromosome 7 by PCR analysis of a somatic cell hybrid panel (Warmke and Ganetzky, 1994) making it a candidate for LQT2. The function of the protein encoded by *HERG* is not known, but it has predicted amino acid sequence homology to potassium channels. *HERG* was isolated from a hippocampal cDNA library by homology to the *Drosophila* ether a-go-go gene (*eag*), which encodes a calcium-modulated potassium channel (Bruggeman et al., 1993). *HERG* is not the human homolog of *eag,* however, sharing only ∼50% amino acid sequence homology. *HERG* has been shown to be associated with LQT2 (Curran et al., 1995).

A novel potassium channel gene has now been discovered which is named *KVLQT1.* Evidence is presented here indicating that *KVLQT1* is *LQT1.* Sixteen families with mutations in *KVLQT1* were identified and characterized and it was shown that in all sixteen families there was complete linkage between *LQT1* and *KVLQT1. KVLQT1* was mapped to chromosome 11p15.5 making it a candidate gene for *LQT1. KVLQT1* encodes a protein with structural characteristics of potassium channels, and expression of the gene as measured by Northern blot analysis demonstrated that *KVLQT1* is most strongly expressed in the heart. One intragenic deletion and ten different missense mutations which cause LQT were identified in *KVLQT1.* These data define KVLQT1 as a novel cardiac potassium channel gene and show that mutations in this gene cause susceptibility to ventricular tachyarrhythmias and sudden death.

It was known that that one component of the I_{Ks} channel is minK, a 130 amino acid protein with a single putative transmembrane domain (Takumi et al., 1988; Goldstein and Miller, 1991; Hausdorff et al., 1991; Takumi et al., 1991; Busch et al., 1992; Wang and Goldstein, 1995; Wang et al., 1996). The size and structure of this protein made it unlikely that minK alone forms functional channels (Attali et al., 1993; Lesage et al., 1993). Evidence is presented that KVLQT1 and minK coassemble to form the cardiac I_{Ks}, potassium channel. I_{Ks} dysfunction is a cause of cardiac arrhythmia.

### SUMMARY OF THE INVENTION

The present invention demonstrates the molecular basis of long QT syndrome. More specifically, the present invention has determined that molecular variants of the *KVLQT1* gene cause or are involved in the pathogenesis of LQT. Genotypic analyses show that *KVLQT1* is completely linked to *LQT1* in sixteen unrelated families. Analysis of the *KVLQT1* gene will provide an early diagnosis of subjects with LQT. The diagnostic method comprises analyzing the DNA sequence of the *KVLQT1* gene of an individual to be tested and comparing it with the DNA sequence of the native, non-variant gene. In a second embodiment, the *KVLQT1* gene of an individual to be tested is screened for mutations which cause LQT. The ability to predict LQT will enable physicians to prevent the disease with medical therapy such as beta blocking agents.

It is further demonstrated that KVLQT1 and minK coassemble to form a cardiac I_{Ks} potassium channel. I_{Ks} dysfunction is a cause of cardiac arrhythmia. The knowledge that these two proteins coassemble to form the I_{Ks} channel is useful for developing an assay to screen for drugs which are useful in treating or preventing LQT1. By coexpressing both genes in a cell such as an oocyte it is possible to screen for drugs which have an effect on the I_{Ks} channel, both in its wild-type and in its mutated forms. This knowledge is also useful for the analysis of the *minK* gene for an early diagnosis of subjects with LQT. The diagnostic methods are performed as noted above for *KVLQT1.*

### BRIEF DESCRIPTION OF THE FIGURES

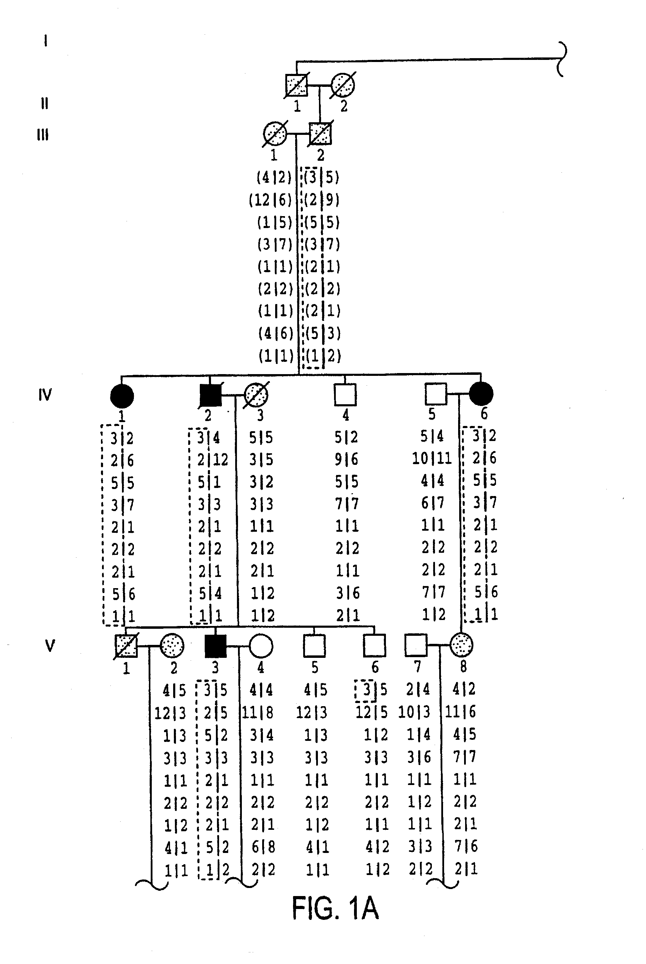

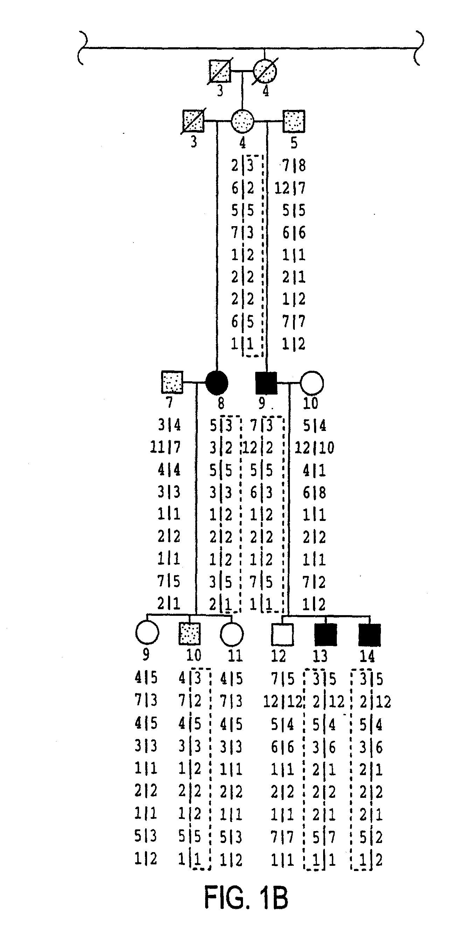

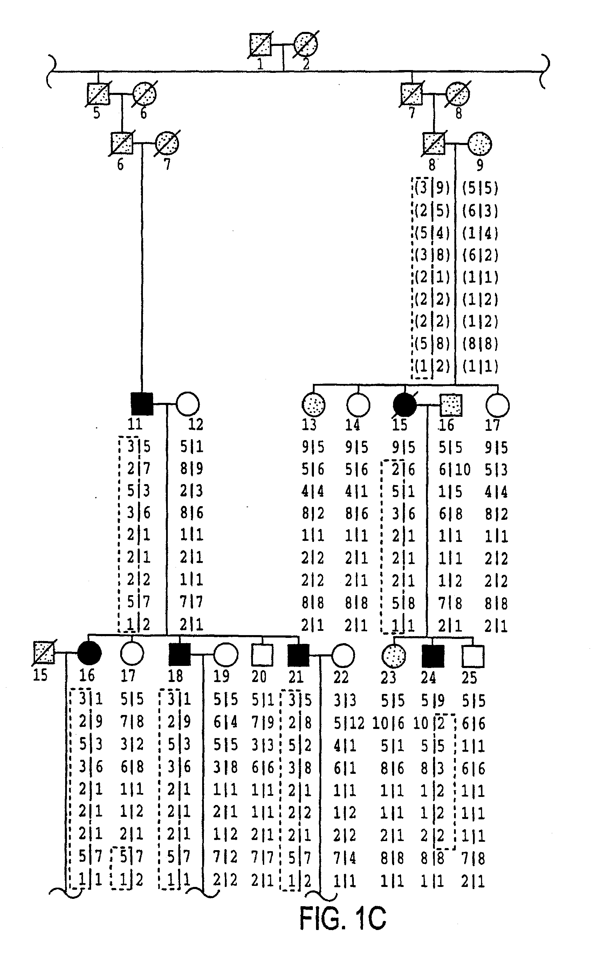

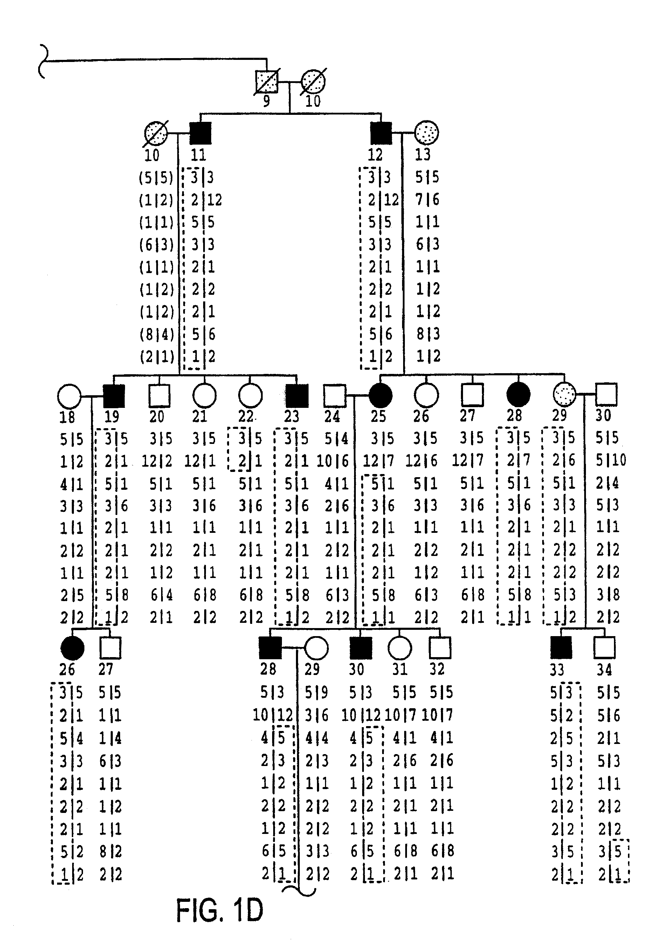

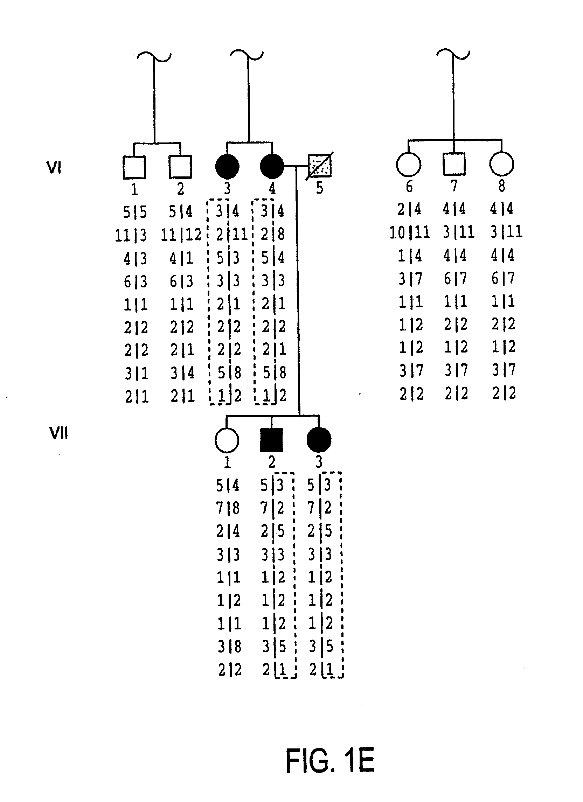

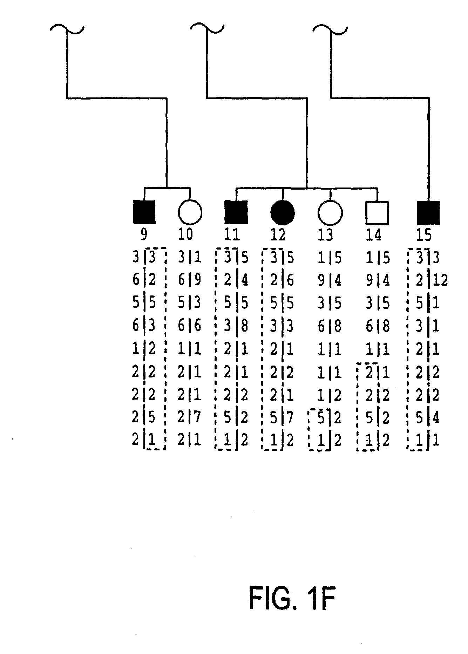

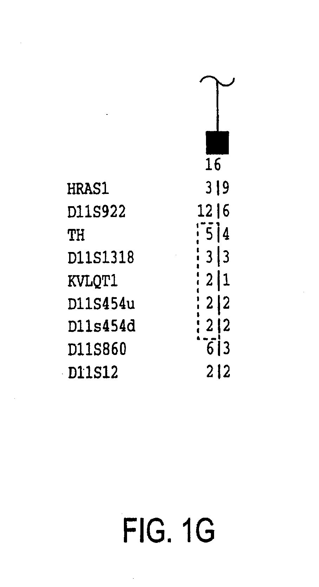

Figure 1. Pedigree structure for a portion of LQT kindred 1532. Affected individuals are shown as filled circles (females) or squares (males), unaffected individuals as empty symbols and individuals with equivocal phenotypes are stippled. Genotypes for chromosome I 1 markers are indicated beneath each symbol and are shown as haplotypes. Marker order (top to bottom) is: Tel-*HRAS-D11S922-TH-D11S1318-D11S454-D11S860-D11S12*-Cen. The accuracy of haplotypes was ensured using genotypes from additional chromosome 11p15.5 markers (Q. Wang, unpublished results). Inferred genotypes are shown in brackets. Disease chromosomes are indicated by boxes and recombination events are indicated with solid horizontal lines. Recombination events affecting disease chromosomes occur in individuals: IV-22, IV-25, V-6, V-17, V-24, V-34, VI-13, VI-14 and VI-16. Recombination events occurring in non-disease chromosomes are not indicated. *KVLQT1* is an SSCP conformer within *KVLQT1* identified by primers 5 and 6; this conformer was only identified in K1532 and represents a disease-associated mutation (allele 2 is the mutant allele). Haplotype analyses indicate that *KVLQT1* is located between flanking markers *D11S922* and *D11S454.*

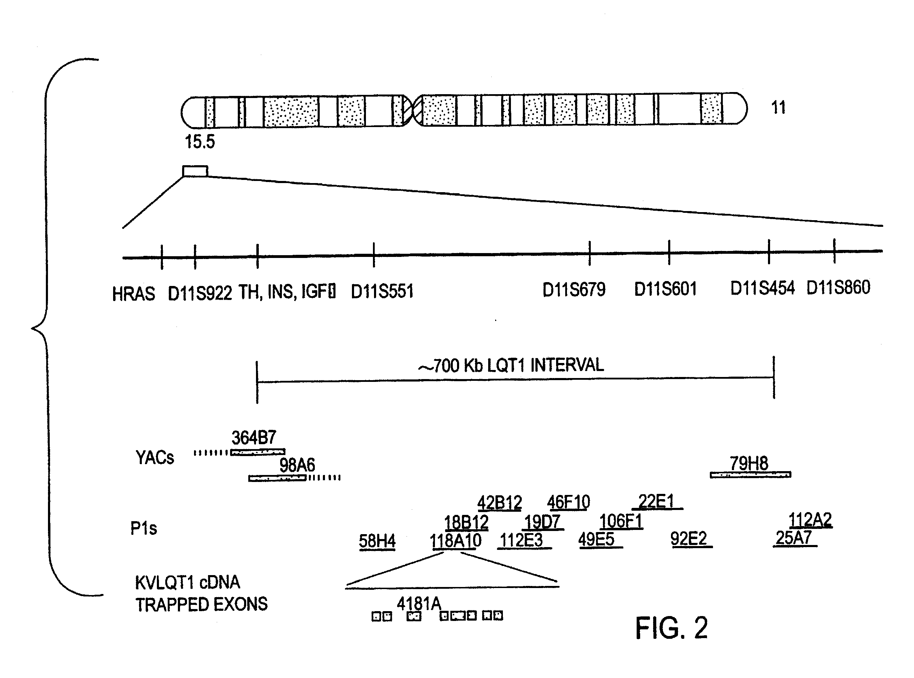

Figure 2. Physical map of the *LQT1* region. Ideogram of chromosome 11 indicates the approximate location of *LQT1* (11p15.5). The location of polymorphic markers and some cosmids are indicated by vertical lines on the map. Refined genetic mapping places *LQT1* between *TH* and *D11S454.* The distance between *TH* and *D11S454* was estimated by pulsed field gel analyses as <700 kb. A physical map of the minimal set of overlapping YAC and P1 clones is shown. The locations of the *KVLQT1* cDNA and trapped exons are indicated. Dashed lines in YACs indicate chimerism.

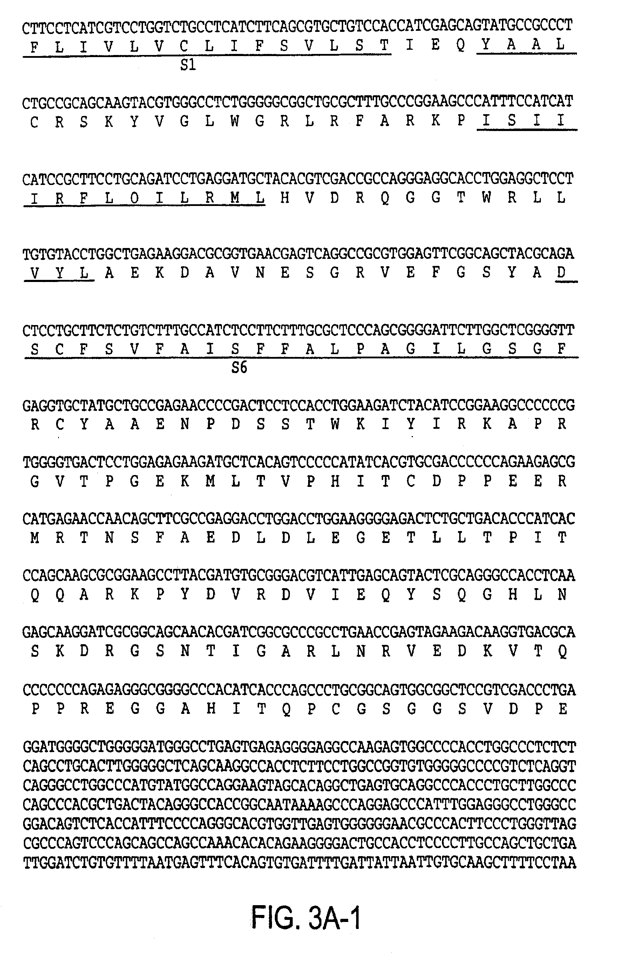

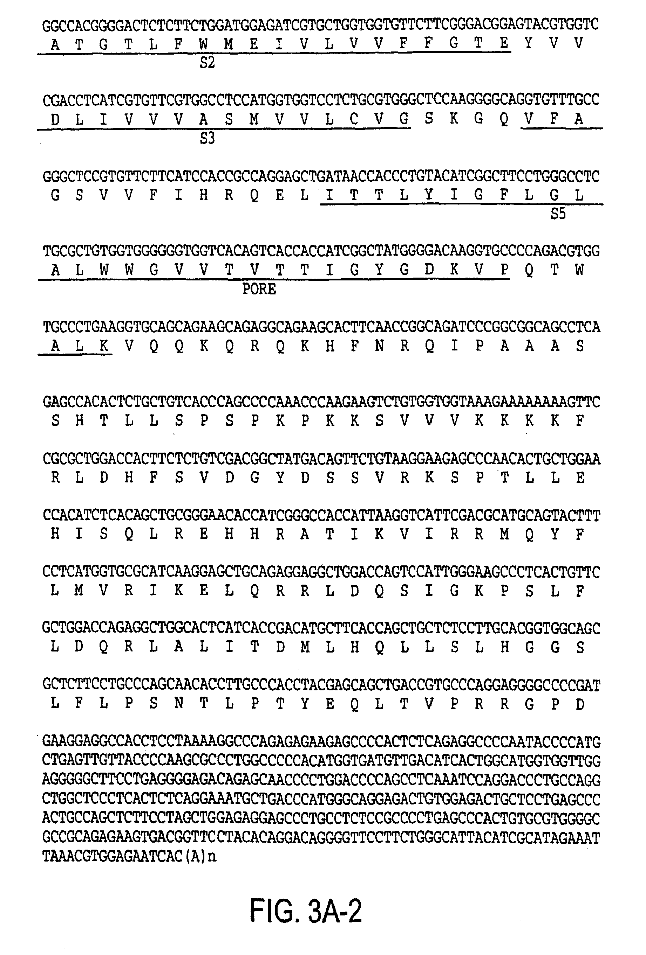

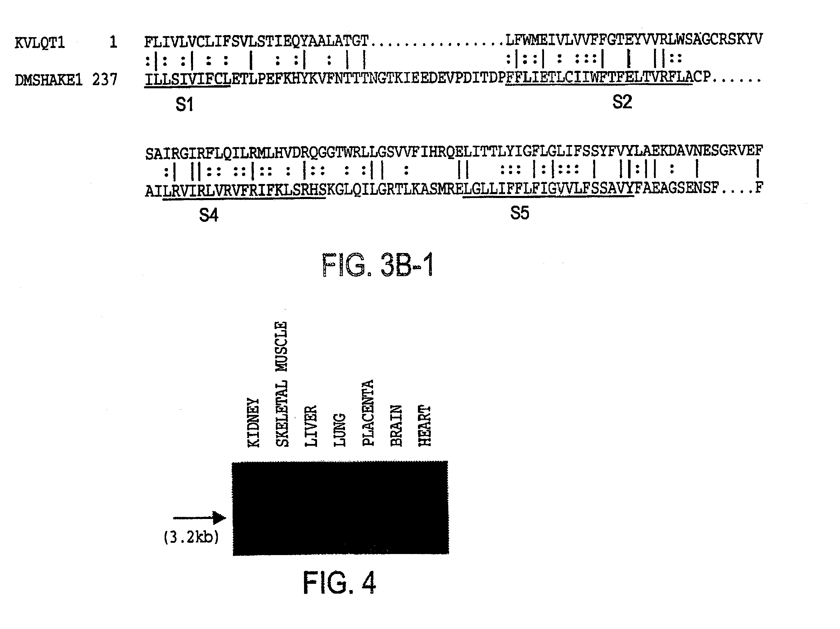

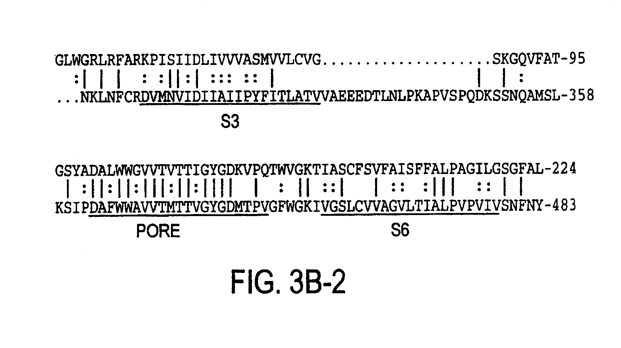

Figures 3A and 3B. Nucleotide and deduced amino acid sequences of *KVLQT1* (not including the region encoding the first 34 amino acids). (A) The composite sequence of *KVLQT1* is shown. The nucleotide sequence is SEQ ID NO:15. The amino acid sequence is SEQ ID NO:16. Six putative transmembrane segments (S1 to S6) and a putative pore region (Pore) are indicated. A potential glycosylation site (N160) is italicized. Two consensus polyadenylation signals are indicated in the 3' untranslated region in bold. Composite cDNA sequences for *KVLQT1* were obtained by end sequencing of overlapping cDNA clones and by primer walking. *KVLQT1* sequences have been assigned GenBank accession number U40990. (B) Alignment of the S1-S6 region of KVLQT1 with *Drosophila* Shaker potassium channel, DMSHAKE1 (SHA) (Pongs et al., 1988). Identity (I) and similarity (:) are indicated. The 3 separate fragments of KVLQT1 are in order: SEQ ID NO:17, SEQ ID NO:18 and SEQ ID NO:19. The 3 separate fragments of DMSHAKE1 are in order: SEQ ID NO:20, SEQ ID NO:21 and SEQ ID NO:22.
Figure 4. Tissue expression pattern of *KVLQT1.* Northern analyses revealed a 3.2 kb *KVLQT1* mRNA in human kidney, lung, placenta, and heart, with highest levels in the heart.

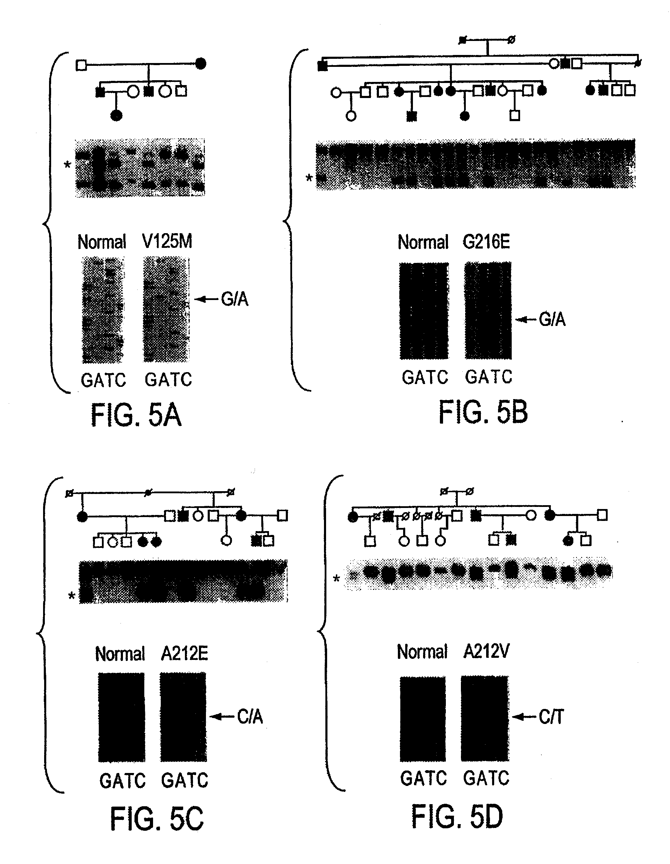

Figures 5A-5D. *KVLQT1* missense mutations cosegregate with LQT in kindreds K1532 (Figure 5A), K2605 (Figure 5B), K1723 (Figure 5C) and K1807 (Figure 5D). The results of SSCP analyses with primer pair 5-6 (K1532), primer pair 9-10 (K1723, K1807), and primer pair 11-12 (K2605) are shown below each pedigree. Aberrant SSCP conformers (indicated by *) cosegregate with LQT in each kindred. For K1532, only eight of the 217 individuals are shown; the results of SSCP analyses in additional members of K1532 are shown in Figure 1 (*KVLQT1* allele 2). Because aberrant SSCP conformers cosegregating with LQT in K161 and K162 were identical to the aberrant conformer defined in K1807, results for these kindreds are not shown. Results of DNA sequence analyses of the normal (left) and aberrant conformers (right) are shown below each pedigree.

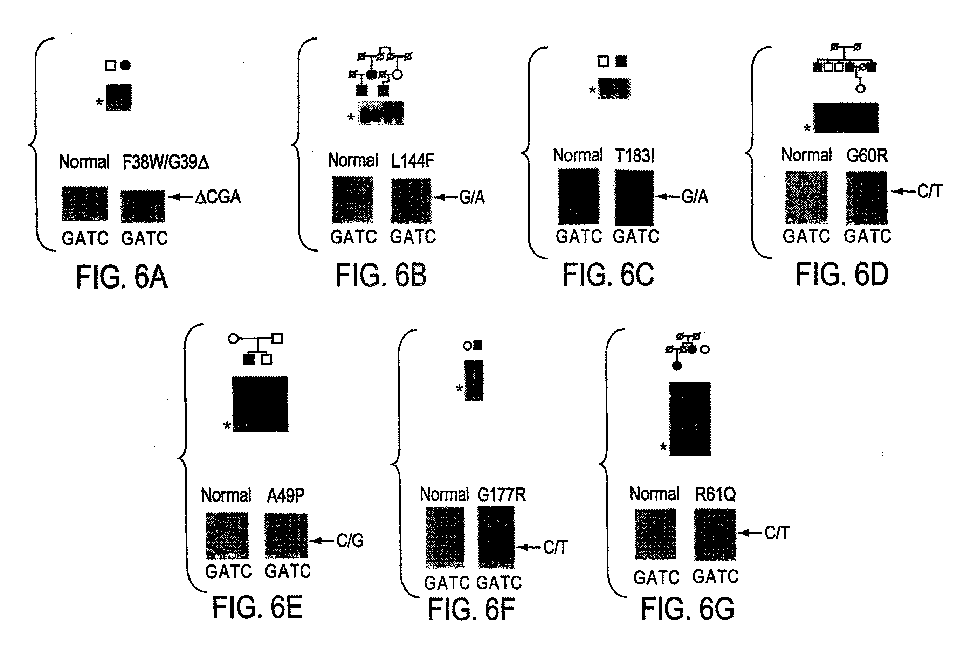

Figures 6A-6G. *KVLQT1* intragenic deletions and missense mutations associated with LQT in kindreds K13216 (Figure 6A), K1777 (Figure 6B), K20925 (Figure 6C), K2557 (Figure 6D), K13119 (Figure 6E), K20926 (Figure 6F) and K15019 (Figure 6G). The results of SSCP analyses with primer pair 1-2 (K13216, K2557, K13119, K15019), primer pair 7-8 (K1777, K20926), and primer pair 9-10 (K20925) are shown below each pedigree. Because aberrant SSCP conformers cosegregating with LQT in K2050, K163 and K164 were identical to the aberrant conformers defined in K1723 and K1807, results for these kindreds are not shown. Results of DNA sequence analyses of the normal (left) and aberrant (right) conformers are shown below each pedigree. Sequences shown are on the antisense strand.

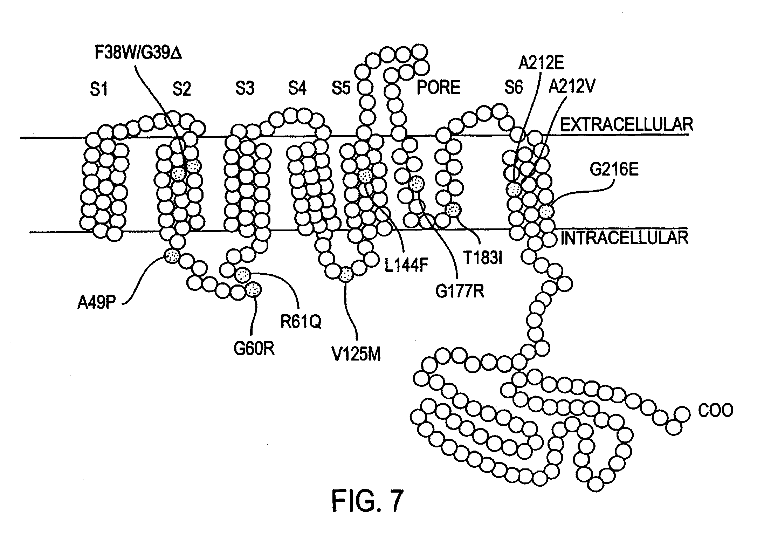

Figure 7. Schematic representation of the predicted topology of KVLQT1 protein and location of *KVLQT1* mutations.

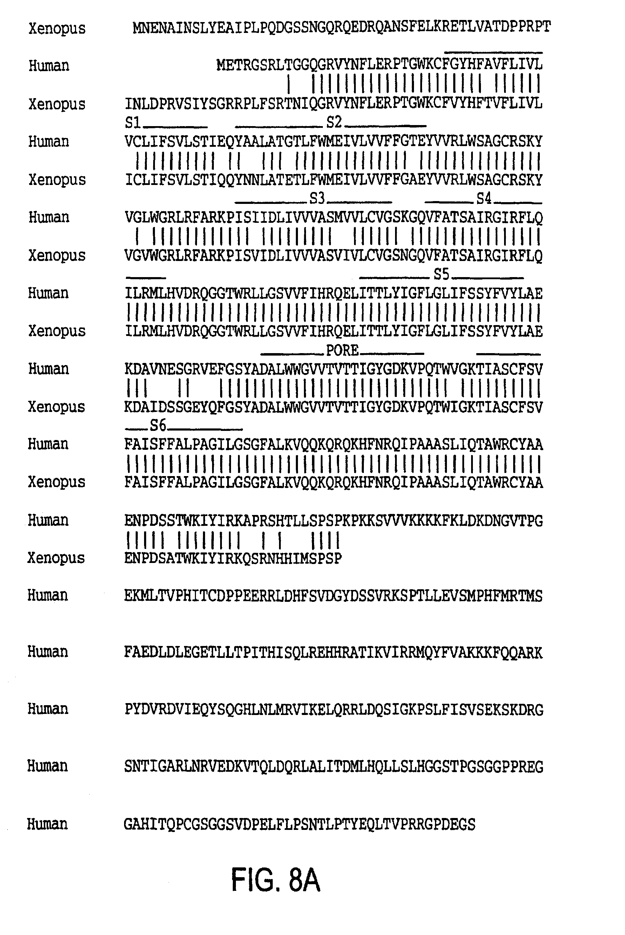

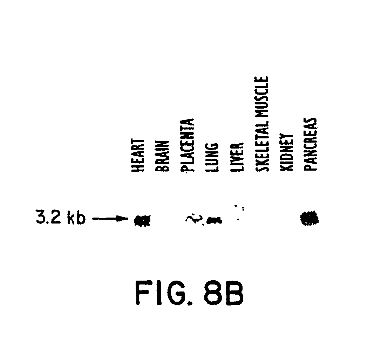

Figures 8A and 8B. Structure of human and *Xenopus* KVLQT1 and tissue-expression pattern of human *KVLQT1.* A) Comparison of human and a partial *Xenopus* KVLQT1 amino acid sequence. Vertical lines indicate identical residues. The Xenopus amino acid sequence is SEQ ID NO:23 and the human amino acid sequence is SEQ ID NO:24. B) Northern analysis indicating expression of *KVLQT1* in human heart, placenta, lung, kidney and pancreas.

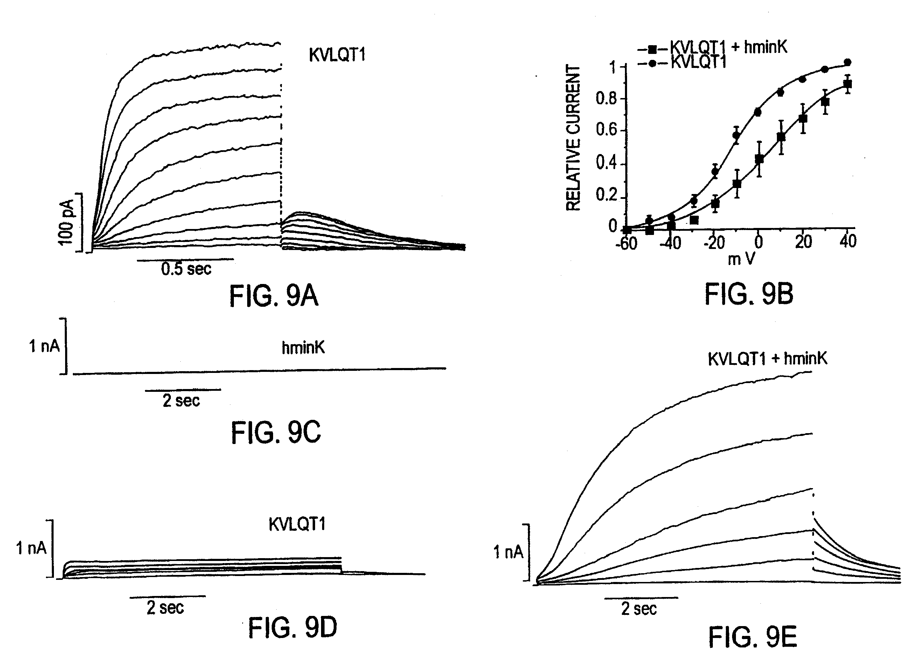

Figures 9A-9E. KVLQT1 and hminK coexpression in CHO cells induces a current nearly identical to cardiac I_{Ks}. A) KVLQT1 currents recorded during 1 sec depolarizing pulses to membrane potentials of -50 to +40 mV, applied from a holding potential of -80 mV. Tail currents were measured at -70 mV. B) Normalized isochronal activation curves for cells transfected with *KVLQT1* (n = 6; 1 sec pulses) or *KVLQT1* and *hminK* (n = 7; 7.5 sec pulses). C-E) Currents recorded during 7.5 sec pulses to -40, -20, -10, 0, +20 and +40 mV in cells transfected with *hminK* (C), *KVLQT1* (D) or *KVLQT1* and *hminK* (E). Tail currents were measured at -70 mV in D, and at -50 mV in C and E. The amplitude of steady state KVLQT1 current at +40 mV was 0.37 ± 0.14 nA (n = 6). In cells cotransfected with *KVLQT1* and *hminK,* time-dependent current during a 7.5-s pulse to +40 mV was 1.62 ± 0.39 nA (n = 7).

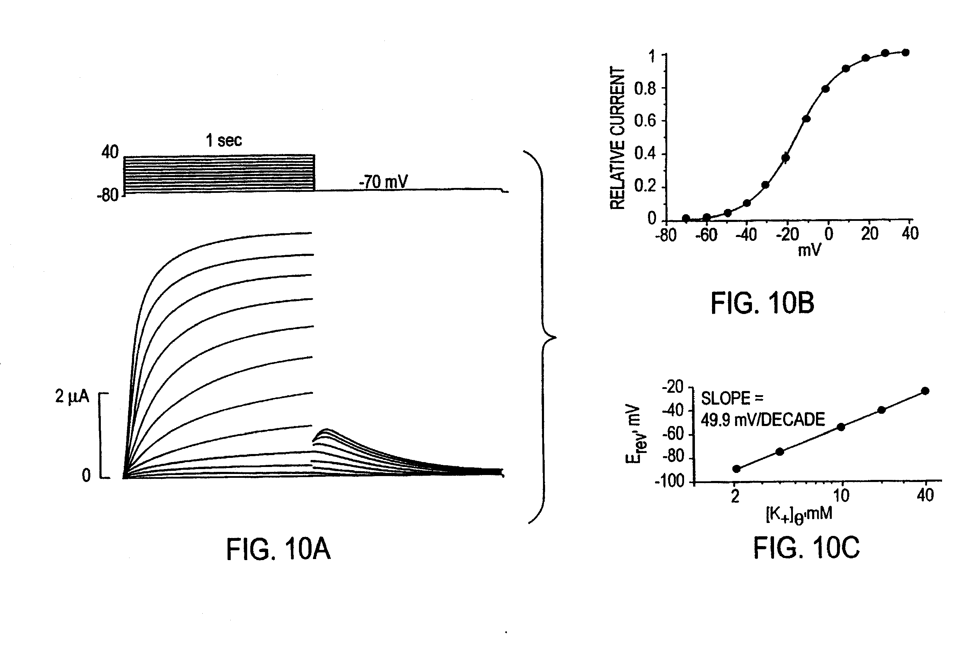

Figures 10A-10C. Expression of KVLQT1 in *Xenopus* oocytes. A) Currents recorded in an oocyte injected with 12.5 ng *KVLQT1* cRNA. Pulses were applied in 10 mV increments from -70 to +40 mV. B) Isochronal (1s) activation curve for KVLQT1 current. The V_{½} was -14.0 ± 0.2 mV and the slope factor was 11.2 ± 0.2 mV (n = 9). C) The relationship of Eᵣₑᵥ versus log[K⁺]ₑ was fit with a linear function and had a slope of 49.9 ± 0.4 mV (n = 6-7 oocytes per point). Tail currents were measured at several voltages after 1.6 sec prepulses to +10 mV.

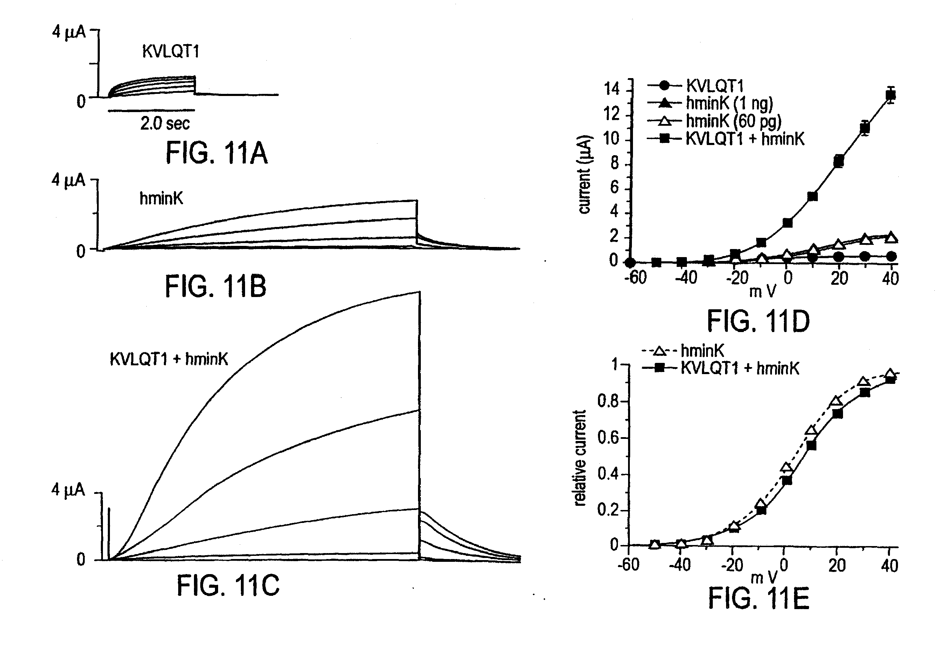

Figures 11A-11E. Coexpression of KVLQT1 and hminK suggests the presence of a KVLQT1 homologue in *Xenopus* oocytes. Currents were recorded at -40, -20, 0, +20 and +40 mV in oocytes injected with either 5.8 ng *KVLQT1* (Figure 11A), ng *hminK* (Figure 11B), or co-injected with both cRNAs (Figure 11C). Figure 11D shows current-voltage relationships measured using 2 sec pulses for KVLQT1, and 7.5 sec pulses for hminK, or KVLQT1 and hminK (n = 20 cells for each condition). For oocytes injected with 60 pg or 1 ng of *hminK* cRNA, I_{sK} at +40 mV was 2.11 ± 0.12 µA and 2.20 ± 0.18 µA. Figure 11E shows normalized isochronal activation curves for oocytes injected with *hminK* (V½= 2.4 ± 0.3 mV; slope = 11.4 ±10.3 mV; n = 16) or co-injected with *KVLQT1* and *hminK* cRNA (V½= 6.2 ± 0.3 mV; slope = 12.3 ± 0.2 mV; n = 20).

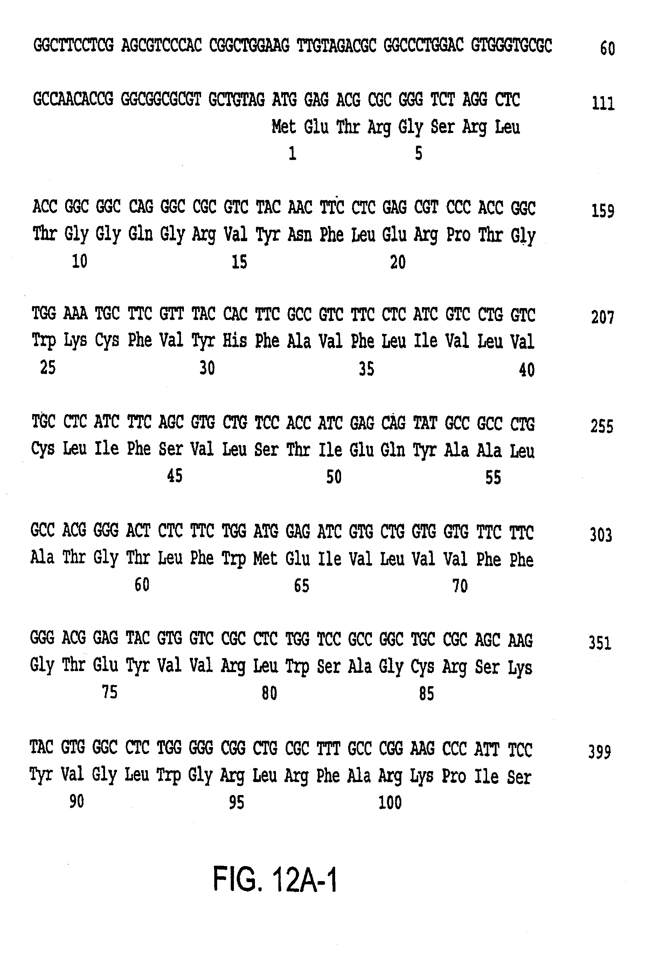

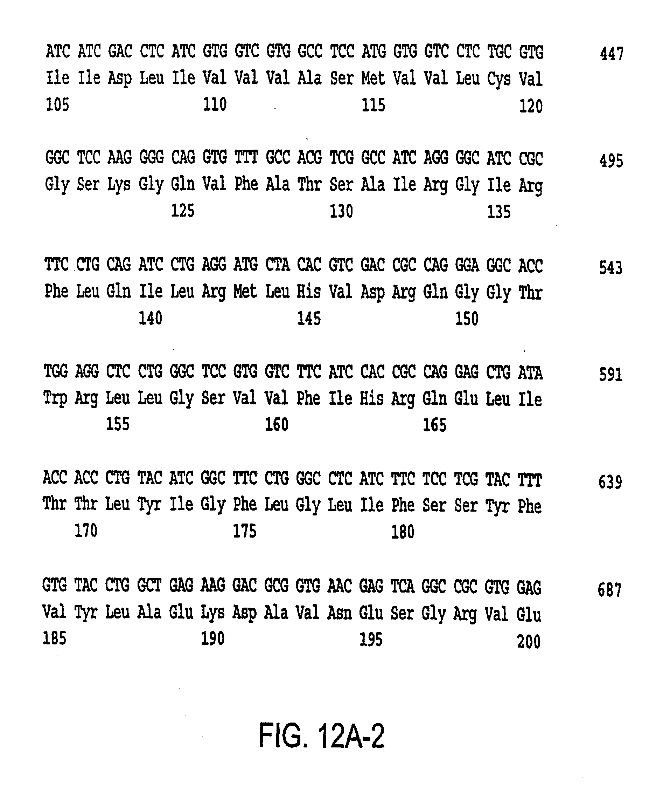

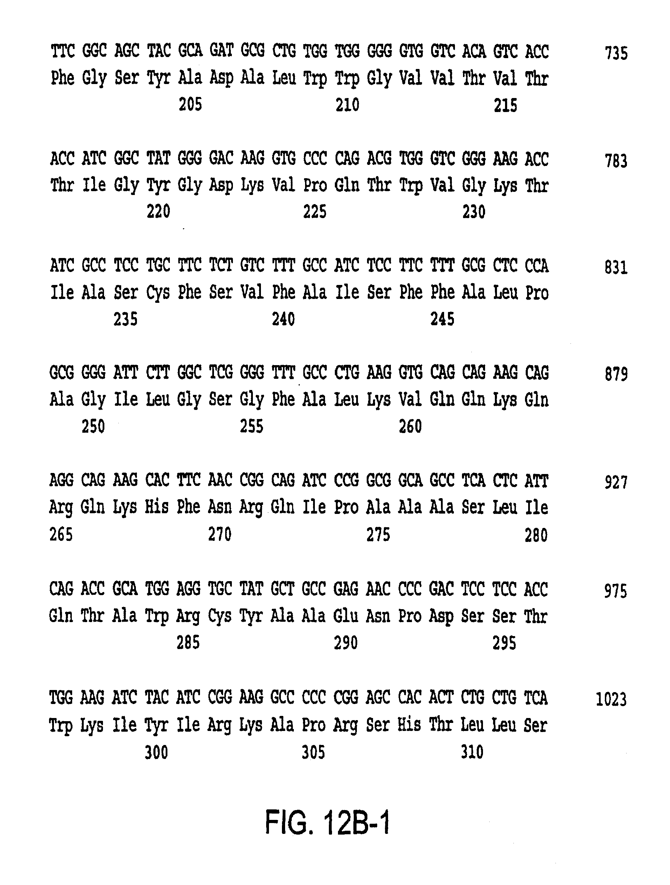

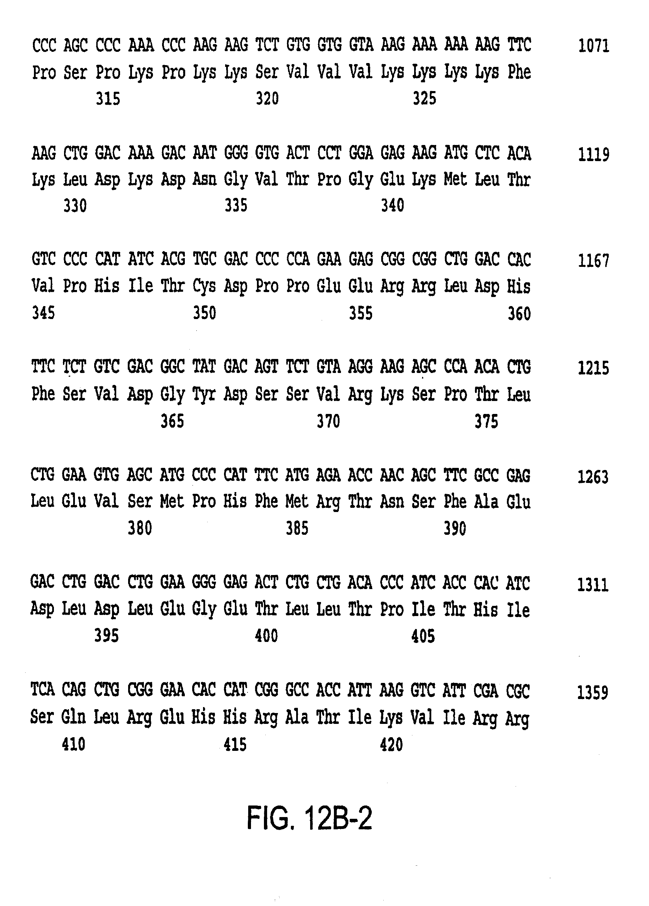

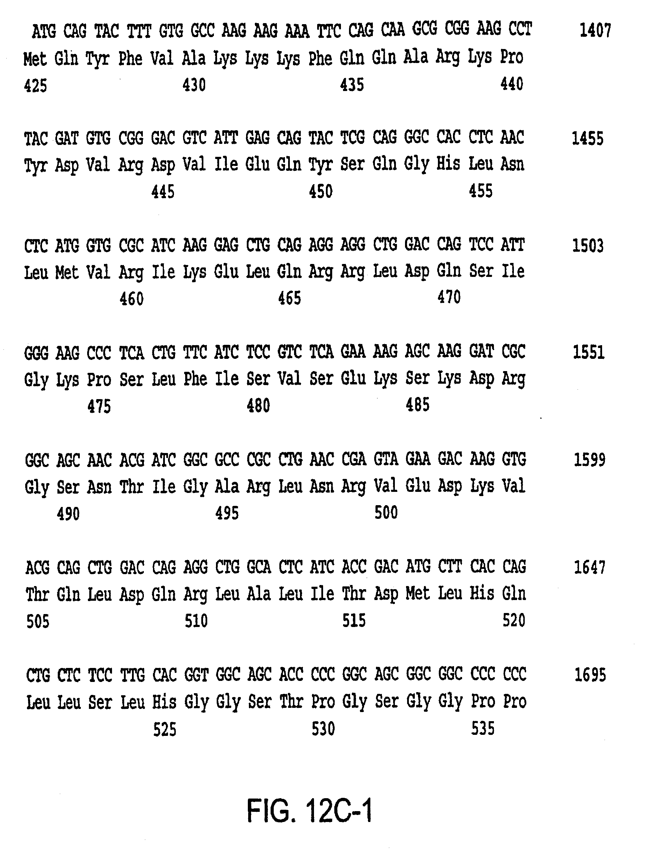

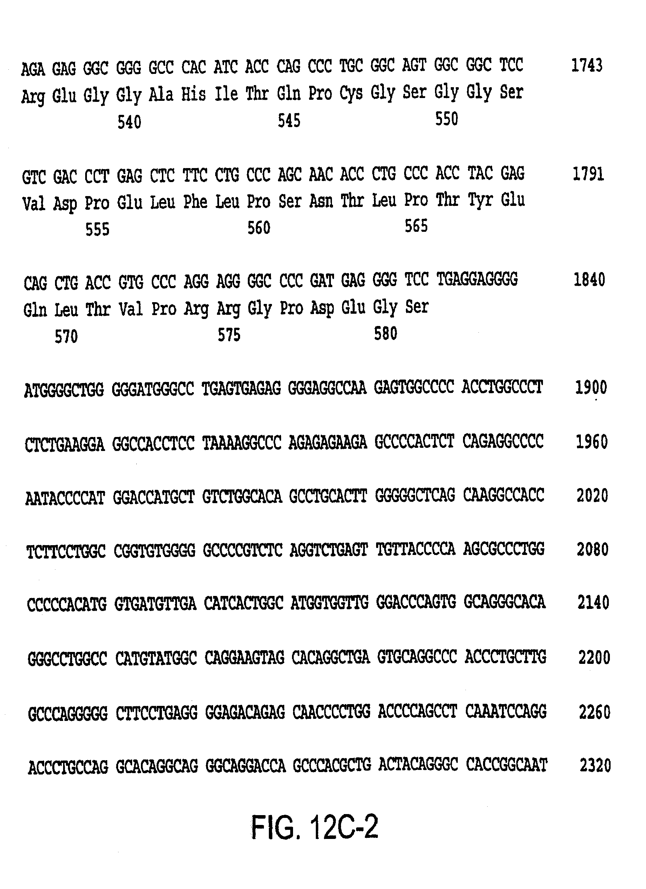

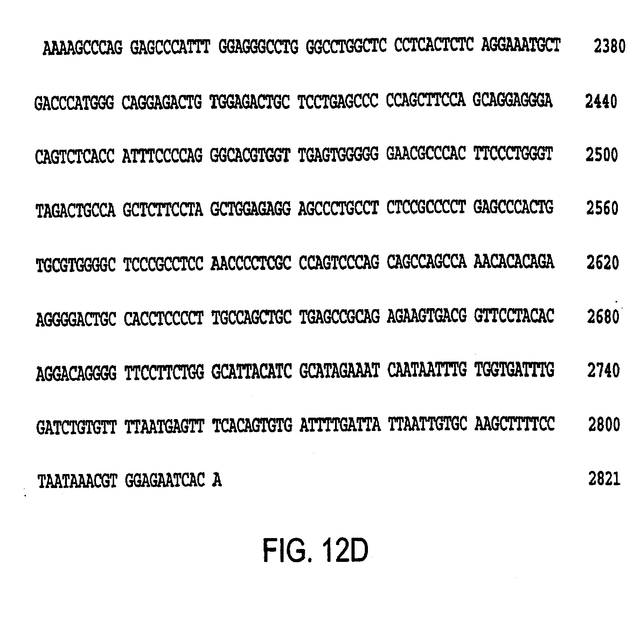

Figures 12A-12D. The nucleotide sequence for *KVLQT1* cDNA and its translation product are shown.

### DETAILED DESCRIPTION OF THE INVENTION

The present invention is directed to the determination that LQT maps to the *KYLQT1* gene and that molecular variants of this gene cause or are involved in the pathogenesis of LQT. It is also directed to the determination that KVLQT1 and minK coassemble to form cardiac I_{Ks} potassium channels. More specifically, the present invention relates to mutations in the *KYLQT1* gene and their use in the diagnosis of LQT. The present invention is further directed to methods of screening humans for the presence of *KVLQT1* gene variants which cause LQT. Since LQT can now be detected earlier (i.e., before symptoms appear) and more definitively, better treatment options will be available in those individuals identified as having LQT. The present invention is also directed to methods for screening for drugs useful in treating or preventing LQT1.

The present invention provides methods of screening the *KVLQT1* gene to identify mutations. Such methods may further comprise the step of amplifying a portion of the *KVLQT1* gene, and may further include a step of providing a set of polynucleotides which are primers for amplification of said portion of the *KVLQT1* gene. The method is useful for identifying mutations for use in either diagnosis of LQT or prognosis of LQT.

Long QT syndrome is an inherited disorder that causes sudden death from cardiac arrhythmias, specifically *torsade de pointes* and ventricular fibrillation. LQT was previously mapped to three loci: *LQT1* on chromosome 11p15.5, *LQT2* on 7q35-36 and *LQT3* on 3p21-24. It is a discovery of the present invention that there is a genetic linkage between *LQT1* and polymorphisms within *KVLQT1,* a cardiac potassium channel gene.

The present invention further demonstrates that *minK* on chromosome 21 is also involved in LQT. The minK protein and KVLQT1 coassemble to form a K⁺ channel. The present invention thus provides methods of screening the *minK* gene to identify mutations. Such methods may further comprise the step of amplifying a portion of the *minK* gene, and may further include a step of providing a set of polynucleotides which are primers for amplification of said portion of the *minK* gene. The method is useful for identifying mutations for use in either diagnosis of LQT or prognosis of LQT.

Finally, the present invention is directed to a method for screening drug candidates to identify drugs useful for treating or preventing LQT. Drug screening is performed by coexpressing mutant *KVLQT1* and/or *min*K genes in cells, such as oocytes, mammalian cells or transgenic animals, and assaying the effect of a drug candidate on the I_{Ks} channel. The effect is compared to the I_{Ks} channel activity of the wild-type *KVLQT1* and *minK* genes.

Proof that the *KVLQT1* gene is involved in causing LQT is obtained by finding sequences in DNA extracted from affected kindred members which create abnormal *KVLQT1* gene products or abnormal levels of the gene products. Such LQT susceptibility alleles will cosegregate with the disease in large kindreds. They will also be present at a much higher frequency in non-kindred individuals with LQT than in individuals in the general population. The key is to find mutations which are serious enough to cause obvious disruption to the normal function of the gene product. These mutations can take a number of forms. The most severe forms would be frame shift mutations or large deletions which would cause the gene to code for an abnormal protein or one which would significantly alter protein expression. Less severe disruptive mutations would include small in-frame deletions and nonconservative base pair substitutions which would have a significant effect on the protein produced, such as changes to or from a cysteine residue, from a basic to an acidic amino acid or vice versa, from a hydrophobic to hydrophilic amino acid or vice versa, or other mutations which would affect secondary or tertiary protein structure. Silent mutations or those resulting in conservative amino acid substitutions would not generally be expected to disrupt protein function.

According to the diagnostic and prognostic method of the present invention, alteration of the wild-type *KVLQT1* gene is detected. In addition, the method can be performed by detecting the wild-type *KVLQT1* gene and confirming the lack of a cause of LQT as a result of this locus. "Alteration of a wild-type gene" encompasses all forms of mutations including deletions, insertions and point mutations in the coding and noncoding regions. Deletions may be of the entire gene or of only a portion of the gene. Point mutations may result in stop codons, frameshift mutations or amino acid substitutions. Somatic mutations are those which occur only in certain tissues and are not inherited in the germline. Germline mutations can be found in any of a body's tissues and are inherited. Point mutational events may occur in regulatory regions, such as in the promoter of the gene, leading to loss or diminution of expression of the mRNA. Point mutations may also abolish proper RNA processing, leading to loss of expression of the *KVLQT1* gene product, or to a decrease in mRNA stability or translation efficiency.

The presence of LQT may be ascertained by testing any tissue of a human for mutations of the *KVLQT1* gene or the *minK* gene. For convenience of reference, the following description will be directed to the *KVLQT1* gene. However, the description is equally applicable for the *minK* gene for testing for mutations. For example, a person who has inherited a germline *KVLQT1* mutation would be prone to develop LQT. This can be determined by testing DNA from any tissue of the person's body. Most simply, blood can be drawn and DNA extracted from the cells of the blood. In addition, prenatal diagnosis can be accomplished by testing fetal cells, placental cells or amniotic cells for mutations of the *KVLQT1* gene. Alteration of a wild-type *KVLQT1* allele, whether, for example, by point mutation or deletion, can be detected by any of the means discussed herein.

There are several methods that can be used to detect DNA sequence variation. Direct DNA sequencing, either manual sequencing or automated fluorescent sequencing can detect sequence variation. Another approach is the single-stranded conformation polymorphism assay (SSCP) (Orita et al., 1989). This method does not detect all sequence changes, especially if the DNA fragment size is greater than 200 bp, but can be optimized to detect most DNA sequence variation. The reduced detection sensitivity is a disadvantage, but the increased throughput possible with SSCP makes it an attractive, viable alternative to direct sequencing for mutation detection on a research basis. The fragments which have shifted mobility on SSCP gels are then sequenced to determine the exact nature of the DNA sequence variation. Other approaches based on the detection of mismatches between the two complementary DNA strands include clamped denaturing gel electrophoresis (CDGE) (Sheffield et al., 1991), heteroduplex analysis (HA) (White et al., 1992) and chemical mismatch cleavage (CMC) (Grompe et al., 1989). None of the methods described above will detect large deletions, duplications or insertions, nor will they detect a regulatory mutation which affects transcription or translation of the protein. Other methods which might detect these classes of mutations such as a protein truncation assay or the asymmetric assay, detect only specific types of mutations and would not detect missense mutations. A review of currently available methods of detecting DNA sequence variation can be found in a recent review by Grompe (1993). Once a mutation is known, an allele specific detection approach such as allele specific oligonucleotide (ASO) hybridization can be utilized to rapidly screen large numbers of other samples for that same mutation.

A rapid preliminary analysis to detect polymorphisms in DNA sequences can be performed by looking at a series of Southern blots of DNA cut with one or more restriction enzymes, preferably with a large number of restriction enzymes. Each blot contains a series of normal individuals and a series of LQT cases. Southern blots displaying hybridizing fragments (differing in length from control DNA when probed with sequences near or including the *KVLQT1* locus) indicate a possible mutation. If restriction enzymes which produce very large restriction fragments are used, then pulsed field gel electrophoresis (PFGE) is employed.

Detection of point mutations may be accomplished by molecular cloning of the *KVLQT1* allele and sequencing the allele using techniques well known in the art.

There are six well known methods for a more complete, yet still indirect, test for confirming the presence of a susceptibility allele: 1) single stranded conformation analysis (SSCP) (Orita et al., 1989); 2) denaturing gradient gel electrophoresis (DGGE) (Wartell et al., 1990; Sheffield et al., 1989); 3) RNase protection assays (Finkelstein et al., 1990; Kinszler et al., 1991); 4) allele-specific oligonucleotides (ASOs) (Conner et al., 1983); 5) the use of proteins which recognize nucleotide mismatches, such as the *E. coli* mutS protein (Modrich, 1991); and 6) allele-specific PCR (Rano and Kidd, 1989). For allele-specific PCR, primers are used which hybridize at their 3' ends to a particular *KVLQT1* mutation. If the particular mutation is not present, an amplification product is not observed. Amplification Refractory Mutation System (ARMS) can also be used, as disclosed in European Patent Application Publication No. 0332435 and in Newton et al., 1989. Insertions and deletions of genes can also be detected by cloning, sequencing and amplification. In addition, restriction fragment length polymorphism (RFLP) probes for the gene or surrounding marker genes can be used to score alteration of an allele or an insertion in a polymorphic fragment. Such a method is particularly useful for screening relatives of an affected individual for the presence of the mutation found in that individual. Other techniques for detecting insertions and deletions as known in the art can be used.

In the first three methods (SSCP, DGGE and RNase protection assay), a new electrophoretic band appears. SSCP detects a band which migrates differentially because the sequence change causes a difference in single-strand, intramolecular base pairing. RNase protection involves cleavage of the mutant polynucleotide into two or more smaller fragments. DGGE detects differences in migration rates of mutant sequences compared to wild-type sequences, using a denaturing gradient gel. In an allele-specific oligonucleotide assay, an oligonucleotide is designed which detects a specific sequence, and the assay is performed by detecting the presence or absence of a hybridization signal. In the mutS assay, the protein binds only to sequences that contain a nucleotide mismatch in a heteroduplex between mutant and wild-type sequences.

Mismatches, according to the present invention, are hybridized nucleic acid duplexes in which the two strands are not 100% complementary. Lack of total homology may be due to deletions, insertions, inversions or substitutions. Mismatch detection can be used to detect point mutations in the gene or in its mRNA product. While these techniques are less sensitive than sequencing, they are simpler to perform on a large number of samples. An example of a mismatch cleavage technique is the RNase protection method. In the practice of the present invention, the method involves the use of a labeled riboprobe which is complementary to the human wild-type *KVLQT1* gene coding sequence. The riboprobe and either mRNA or DNA isolated from the person are annealed (hybridized) together and subsequently digested with the enzyme RNase A which is able to detect some mismatches in a duplex RNA structure. If a mismatch is detected by RNase A, it cleaves at the site of the mismatch. Thus, when the annealed RNA preparation is separated on an electrophoretic gel matrix, if a mismatch has been detected and cleaved by RNase A, an RNA product will be seen which is smaller than the full length duplex RNA for the riboprobe and the mRNA or DNA. The riboprobe need not be the full length of the mRNA or gene but can be a segment of either. If the riboprobe comprises only a segment of the mRNA or gene, it will be desirable to use a number of these probes to screen the whole mRNA sequence for mismatches.

In similar fashion, DNA probes can be used to detect mismatches, through enzymatic or chemical cleavage. See, e.g., Cotton *et al.,* 1988; Shenk *et al.,* 1975; Novack *et al.,* 1986. Alternatively, mismatches can be detected by shifts in the electrophoretic mobility of mismatched duplexes relative to matched duplexes. See, e.g., Cariello, 1988. With either riboprobes or DNA probes, the cellular mRNA or DNA which might contain a mutation can be amplified using PCR (see below) before hybridization. Changes in DNA of the *KVLQT1* gene can also be detected using Southern hybridization, especially if the changes are gross rearrangements, such as deletions and insertions.

DNA sequences of the *KVLQT1* gene which have been amplified by use of PCR may also be screened using allele-specific probes. These probes are nucleic acid oligomers, each of which contains a region of the gene sequence harboring a known mutation. For example, one oligomer may be about 30 nucleotides in length, corresponding to a portion of the gene sequence. By use of a battery of such allele-specific probes, PCR amplification products can be screened to identify the presence of a previously identified mutation in the gene. Hybridization of allele-specific probes with amplified *KVLQT1* sequences can be performed, for example, on a nylon filter. Hybridization to a particular probe under stringent hybridization conditions indicates the presence of the same mutation in the tissue as in the allele-specific probe.

The most definitive test for mutations in a candidate locus is to directly compare genomic *KVLQT1* sequences from patients with those from a control population. Alternatively, one could sequence messenger RNA after amplification, e.g., by PCR, thereby eliminating the necessity of determining the exon structure of the candidate gene.

Mutations from patients falling outside the coding region of *KVLQT1* can be detected by examining the non-coding regions, such as introns and regulatory sequences near or within the genes. An early indication that mutations in noncoding regions are important may come from Northern blot experiments that reveal messenger RNA molecules of abnormal size or abundance in patients as compared to control individuals.

Alteration of *KVLQT1* mRNA expression can be detected by any techniques known in the art. These include Northern blot analysis, PCR amplification and RNase protection. Diminished mRNA expression indicates an alteration of the wild-type gene. Alteration of wild-type genes can also be detected by screening for alteration of wild-type *KVLQT1* protein. For example, monoclonal antibodies immunoreactive with *KVLQT1* can be used to screen a tissue. Lack of cognate antigen would indicate a mutation. Antibodies specific for products of mutant alleles could also be used to detect mutant gene product. Such immunological assays can be done in any convenient formats known in the art. These include Western blots, immunohistochemical assays and ELISA assays. Any means for detecting an altered *KVLQT1* protein can be used to detect alteration of the wild-type *KVLQT1* gene. Functional assays, such as protein binding determinations, can be used. In addition, assays can be used which detect *KVLQT1* biochemical function. Finding a mutant *KVLQT1* gene product indicates alteration of a wild-type *KVLQT1* gene.

A mutant *KVLQT1* gene or gene product can also be detected in other human body samples, such as serum, stool, urine and sputum. The same techniques discussed above for detection of mutant genes or gene products in tissues can be applied to other body samples. By screening such body samples, a simple early diagnosis can be achieved for LQT.

The primer pairs of the present invention are useful for determination of the nucleotide sequence of a particular *KVLQT1* or *minK* allele using PCR. The pairs of single-stranded DNA primers for *KVLQT1* can be annealed to sequences within or surrounding the *KVLQT1* gene on chromosome 11 in order to prime amplifying DNA synthesis of the gene itself. The pairs of single-stranded DNA primers for *minK* can be annealed to sequences within or surrounding the *minK* gene on chromosome 21 in order to prime amplifying DNA synthesis of the gene itself. A complete set of these primers allows synthesis of all of the nucleotides of the gene coding sequences, i.e., the exons. The set of primers preferably allows synthesis of both intron and exon sequences. Allele-specific primers can also be used. Such primers anneal only to particular *KVLQT1* mutant alleles, and thus will only amplify a product in the presence of the mutant allele as a template.

In order to facilitate subsequent cloning of amplified sequences, primers may have restriction enzyme site sequences appended to their 5' ends. Thus, all nucleotides of the primers are derived from *KVLQT1* sequence or sequences adjacent to *KVLQT1,* except for the few nucleotides necessary to form a restriction enzyme site. Such enzymes and sites are well known in the art. The primers themselves can be synthesized using techniques which are well known in the art. Generally, the primers can be made using oligonucleotide synthesizing machines which are commercially available. Given the sequence of *KVLQT1,* design of particular primers is well within the skill of the art.

The nucleic acid probes provided by the present invention are useful for a number of purposes. They can be used in Southern hybridization to genomic DNA and in the RNase protection method for detecting point mutations already discussed above. The probes can be used to detect PCR amplification products. They may also be used to detect mismatches with the *KVLQT1* gene or mRNA using other techniques.

It has been discovered that individuals with the wild-type *KVLQT1* gene do not have LQT. However, mutations which interfere with the function of the *KVLQT1* gene product are involved in the pathogenesis of LQT. Thus, the presence of an altered (or a mutant) *KVLQT1* gene which produces a protein having a loss of function, or altered function, directly causes LQT which increases the risk of cardiac arrhythmias. In order to detect a *KVLQT1* gene mutation, a biological sample is prepared and analyzed for a difference between the sequence of the allele being analyzed and the sequence of the wild-type allele. Mutant *KVLQT1* alleles can be initially identified by any of the techniques described above. The mutant alleles are then sequenced to identify the specific mutation of the particular mutant allele. Alternatively, mutant alleles can be initially identified by identifying mutant (altered) proteins, using conventional techniques. The mutant alleles are then sequenced to identify the specific mutation for each allele. The mutations, especially those which lead to an altered function of the protein, are then used for the diagnostic and prognostic methods of the present invention.

It has also been discovered that the KVLQT1 protein coassembles with the minK protein. Thus, mutations in *min*K which interfere in the function of the *minK* gene product are involved in the pathogenesis of LQT. Thus, the presence of an altered (or a mutant) *minK* gene which produces a protein having a loss of function, or altered function, directly causes LQT which increases the risk of cardiac arrhythmias. In order to detect a *minK* gene mutation, a biological sample is prepared and analyzed for a difference between the sequence of the allele being analyzed and the sequence of the wild-type allele. Mutant *min*K alleles can be initially identified by any of the techniques described above. The mutant alleles are then sequenced to identify the specific mutation of the particular mutant (altered) proteins, using conventional techniques. The mutant alleles are then sequenced to identify the specific mutation for each allele. The mutations, especially those which lead to an altered function of the protein, are then used for the diagnostic and prognostic methods of the present invention.

### Definitions

The present invention employs the following definitions:

"Probes". Polynucleotide polymorphisms associated with *KVLQT1* alleles which predispose to LQT are detected by hybridization with a polynucleotide probe which forms a stable hybrid with that of the target sequence, under stringent to moderately stringent hybridization and wash conditions. If it is expected that the probes will be perfectly complementary to the target sequence, stringent conditions will be used. Hybridization stringency may be lessened if some mismatching is expected, for example, if variants are expected with the result that the probe will not be completely complementary. Conditions are chosen which rule out nonspecific/adventitious bindings, that is, which minimize noise. Since such indications identify neutral DNA polymorphisms as well as mutations, these indications need further analysis to demonstrate detection of a *KVLQT1* susceptibility allele.

Probes for *KVLQT1* alleles may be derived from the sequences of the *KVLQT1* region or its cDNA. The probes may be of any suitable length, which span all or a portion of the *KVLQT1* region, and which allow specific hybridization to the region. If the target sequence contains a sequence identical to that of the probe, the probes may be short, e.g., in the range of about 8-30 base pairs, since the hybrid will be relatively stable under even stringent conditions. If some degree of mismatch is expected with the probe, i.e., if it is suspected that the probe will hybridize to a variant region, a longer probe may be employed which hybridizes to the target sequence with the requisite specificity.

The probes will include an isolated polynucleotide attached to a label or reporter molecule and may be used to isolate other polynucleotide sequences, having sequence similarity by standard methods. For techniques for preparing and labeling probes see, e.g., Sambrook et al., 1989 or Ausubel et al., 1992. Other similar polynucleotides may be selected by using homologous polynucleotides. Alternatively, polynucleotides encoding these or similar polypeptides may be synthesized or selected by use of the redundancy in the genetic code. Various codon substitutions may be introduced, e.g., by silent changes (thereby producing various restriction sites) or to optimize expression for a particular system. Mutations may be introduced to modify the properties of the polypeptide, perhaps to change the polypeptide degradation or turnover rate.

Probes comprising synthetic oligonucleotides or other polynucleotides of the present invention may be derived from naturally occurring or recombinant single- or double-stranded polynucleotides, or be chemically synthesized. Probes may also be labeled by nick translation, Klenow fill-in reaction, or other methods known in the art.

Portions of the polynucleotide sequence having at least about eight nucleotides, usually at least about 15 nucleotides, and fewer than about 6 kb, usually fewer than about 1.0 kb, from a polynucleotide sequence encoding *KVLQT1* are preferred as probes. The probes may also be used to determine whether mRNA encoding *KYLQT1* is present in a cell or tissue.

**"Regulatory** sequences" refers to those sequences normally within 100 kb of the coding region of a locus, but they may also be more distant from the coding region, which affect the expression of the gene (including transcription of the gene, and translation, splicing, stability or the like of the messenger RNA).

**"Substantial** homology or **similarity".** A nucleic acid or fragment thereof is "substantially homologous" ("or substantially similar") to another if, when optimally aligned (with appropriate nucleotide insertions or deletions) with the other nucleic acid (or its complementary strand), there is nucleotide sequence identity in at least about 60% of the nucleotide bases, usually at least about 70%, more usually at least about 80%, preferably at least about 90%, and more preferably at least about 95-98% of the nucleotide bases.

Alternatively, substantial homology or (similarity) exists when a nucleic acid or fragment thereof will hybridize to another nucleic acid (or a complementary strand thereof) under selective hybridization conditions, to a strand, or to its complement. Selectivity of hybridization exists when hybridization which is substantially more selective than total lack of specificity occurs. Typically, selective hybridization will occur when there is at least about 55% homology over a stretch of at least about 14 nucleotides, preferably at least about 65%, more preferably at least about 75%, and most preferably at least about 90%. See, Kanehisa, 1984. The length of homology comparison, as described, may be over longer stretches, and in certain embodiments will often be over a stretch of at least about nine nucleotides, usually at least about 20 nucleotides, more usually at least about 24 nucleotides, typically at least about 28 nucleotides, more typically at least about 32 nucleotides, and preferably at least about 36 or more nucleotides.

Nucleic acid hybridization will be affected by such conditions as salt concentration, temperature, or organic solvents, in addition to the base composition, length of the complementary strands, and the number of nucleotide base mismatches between the hybridizing nucleic acids, as will be readily appreciated by those skilled in the art. Stringent temperature conditions will generally include temperatures in excess of 30°C, typically in excess of 37°C, and preferably in excess of 45°C. Stringent salt conditions will ordinarily be less than 1000 mM, typically less than 500 mM, and preferably less than 200 mM. However, the combination of parameters is much more important than the measure of any single parameter. See, e.g., Wetmur & Davidson, 1968.

Probe sequences may also hybridize specifically to duplex DNA under certain conditions to form triplex or other higher order DNA complexes. The preparation of such probes and suitable hybridization conditions are well known in the art.

### Preparation of recombinant or chemically synthesize nucleic acids; vectors, transformation, host cells

Large amounts of the polynucleotides of the present invention may be produced by replication in a suitable host cell. Natural or synthetic polynucleotide fragments coding for a desired fragment will be incorporated into recombinant polynucleotide constructs, usually DNA constructs, capable of introduction into and replication in a prokaryotic or eukaryotic cell. Usually the polynucleotide constructs will be suitable for replication in a unicellular host, such as yeast or bacteria, but may also be intended for introduction to (with and without integration within the genome) cultured mammalian or plant or other eukaryotic cell lines. The purification of nucleic acids produced by the methods of the present invention are described, e.g., in Sambrook et al., 1989 or Ausubel et al., 1992.

The polynucleotides of the present invention may also be produced by chemical synthesis, e.g., by the phosphoramidite method described by Beaucage & Carruthers, 1981 or the triester method according to Matteucci and Caruthers, 1981, and may be performed on commercial, automated oligonucleotide synthesizers. A double-stranded fragment may be obtained from the single-stranded product of chemical synthesis either by synthesizing the complementary strand and annealing the strand together under appropriate conditions or by adding the complementary strand using DNA polymerase with an appropriate primer sequence.

Polynucleotide constructs prepared for introduction into a prokaryotic or eukaryotic host may comprise a replication system recognized by the host, including the intended polynucleotide fragment encoding the desired polypeptide, and will preferably also include transcription and translational initiation regulatory sequences operably linked to the polypeptide encoding segment. Expression vectors may include, for example, an origin of replication or autonomously replicating sequence (ARS) and expression control sequences, a promoter, an enhancer and necessary processing information sites, such as ribosome-binding sites, RNA splice sites, polyadenylation sites, transcriptional terminator sequences, and mRNA stabilizing sequences. Such vectors may be prepared by means of standard recombinant techniques well known in the art and discussed, for example, in Sambrook et al., 1989 or Ausubel et al., 1992.

An appropriate promoter and other necessary vector sequences will be selected so as to be functional in the host, and may include, when appropriate, those naturally associated with the *KVLQT1* or *minK* gene. Examples of workable combinations of cell lines and expression vectors are described in Sambrook et al., 1989 or Ausubel et al., 1992; see also, e.g., Metzger et al., 1988. Many useful vectors are known in the art and may be obtained from such vendors as Stratagene, New England Biolabs, Promega Biotech, and others. Promoters such as the trp, lac and phage promoters, tRNA promoters and glycolytic enzyme promoters may be used in prokaryotic hosts. Useful yeast promoters include promoter regions for metallothionein, 3-phosphoglycerate kinase or other glycolytic enzymes such as enolase or glyceraldehyde-3-phosphate dehydrogenase, enzymes responsible for maltose and galactose utilization, and others. Vectors and promoters suitable for use in yeast expression are further described in Hitzeman et al., EP 73,675A. Appropriate non-native mammalian promoters might include the early and late promoters from SV40 (Fiers et al., 1978) or promoters derived from murine Molony leukemia virus, mouse tumor virus, avian sarcoma viruses, adenovirus II, bovine papilloma virus or polyoma. In addition, the construct may be joined to an amplifiable gene (e.g., DHFR) so that multiple copies of the gene may be made. For appropriate enhancer and other expression control sequences, see also Enhancers and Eukaryotic Gene Expression, Cold Spring Harbor Press, Cold Spring Harbor, New York (1983).

While such expression vectors may replicate autonomously, they may also replicate by being inserted into the genome of the host cell, by methods well known in the art.

Expression and cloning vectors will likely contain a selectable marker, a gene encoding a protein necessary for survival or growth of a host cell transformed with the vector. The presence of this gene ensures growth of only those host cells which express the inserts. Typical selection genes encode proteins that a) confer resistance to antibiotics or other toxic substances, e.g. ampicillin, neomycin, methotrexate, etc., b) complement auxotrophic deficiencies, or c) supply critical nutrients not available from complex media, e.g., the gene encoding D-alanine racemase for *Bacilli.* The choice of the proper selectable marker will depend on the host cell, and appropriate markers for different hosts are well known in the art.

The vectors containing the nucleic acids of interest can be transcribed *in vitro,* and the resulting RNA introduced into the host cell by well-known methods, e.g., by injection (see, Kubo et al., 1988), or the vectors can be introduced directly into host cells by methods well known in the art, which vary depending on the type of cellular host, including electroporation; transfection employing calcium chloride, rubidium chloride calcium phosphate, DEAE-dextran, or other substances; microprojectile bombardment; lipofection; infection (where the vector is an infectious agent, such as a retroviral genome); and other methods. See generally, Sambrook et al., 1989 and Ausubel et al., 1992. The introduction of the polynucleotides into the host cell by any method known in the art, including, *inter alia,* those described above, will be referred to herein as "transformation." The cells into which have been introduced nucleic acids described above are meant to also include the progeny of such cells.

Large quantities of the nucleic acids and polypeptides of the present invention may be prepared by expressing the *KVLQT1* or minK nucleic acid or portions thereof in vectors or other expression vehicles in compatible prokaryotic or eukaryotic host cells. The most commonly used prokaryotic hosts are strains of *Escherichia coli,* although other prokaryotes, such as *Bacillus subtilis* or *Pseudomonas* may also be used.

Mammalian or other eukaryotic host cells, such as those of yeast, filamentous fungi, plant, insect, or amphibian or avian species, may also be useful for production of the proteins of the present invention. Propagation of mammalian cells in culture is *per se* well known. See, Jakoby and Pastan (eds.), 1979. Examples of commonly used mammalian host cell lines are VERO and HeLa cells, Chinese hamster ovary (CHO) cells, and WI38, BHK, and COS cell lines, although it will be appreciated by the skilled practitioner that other cell lines may be appropriate, e.g., to provide higher expression, desirable glycosylation patterns, or other features.

Clones are selected by using markers depending on the mode of the vector construction. The marker may be on the same or a different DNA molecule, preferably the same DNA molecule. In prokaryotic hosts, the transformant may be selected, e.g., by resistance to ampicillin, tetracycline or other antibiotics. Production of a particular product based on temperature sensitivity may also serve as an appropriate marker.

Prokaryotic or eukaryotic cells transformed with the polynucleotides of the present invention will be useful not only for the production of the nucleic acids and polypeptides of the present invention, but also, for example, in studying the characteristics of KVLQT1 or minK polypeptide.

The probes and primers based on the *KVLQT1* gene sequence disclosed herein are used to identify homologous *KVLQT1* gene sequences and proteins in other species. These gene sequences and proteins are used in the diagnostic/prognostic, therapeutic and drug screening methods described herein for the species from which they have been isolated.

### Methods of Use: Drug Screening

The invention is particularly useful for screening compounds by using KVLQT1 and minK proteins in transformed cells, transfected oocytes or transgenic animals. Since mutations in either the KVLQT1 or minK protein can alter the functioning of the cardiac I_{Ks} potassium channel, candidate drugs are screened for effects on the channel using cells containing either a normal KVLQT1 or minK protein and a mutant minK or KVLQT1 protein, respectively or a mutant KVLQT1 and a mutant minK protein. The drug is added to the cells in culture or administered to a transgenic animal and the effect on the induced current of the I_{Ks} potassium channel is compared to the induced current of a cell or animal containing the wild-type KVLQT1 and minK. Drug candidates which alter the induced current to a more normal level are useful for treating or preventing LQT.

### Methods of Use: Nucleic Acid Diagnosis and Diagnostic Kits

In order to detect the presence of a *KVLQT1* or *minK* allele predisposing an individual to LQT, a biological sample such as blood is prepared and analyzed for the presence or absence of susceptibility alleles of *KVLQT1* or *minK.* In order to detect the presence of LQT or as a prognostic indicator, a biological sample is prepared and analyzed for the presence or absence of mutant alleles of *KVLQT1* or *minK.* Results of these tests and interpretive information are returned to the health care provider for communication to the tested individual. Such diagnoses may be performed by diagnostic laboratories, or, alternatively, diagnostic kits are manufactured and sold to health care providers or to private individuals for self-diagnosis.

Initially, the screening method involves amplification of the relevant *KVLQT1* or *minK* sequences. In another preferred embodiment of the invention, the screening method involves a non-PCR based strategy. Such screening methods include two-step label amplification methodologies that are well known in the art. Both PCR and non-PCR based screening strategies can detect target sequences with a high level of sensitivity.

The most popular method used today is target amplification. Here, the target nucleic acid sequence is amplified with polymerases. One particularly preferred method using polymerase-driven amplification is the polymerase chain reaction (PCR). The polymerase chain reaction and other polymerase-driven amplification assays can achieve over a million-fold increase in copy number through the use of polymerase-driven amplification cycles. Once amplified, the resulting nucleic acid can be sequenced or used as a substrate for DNA probes.

When the probes are used to detect the presence of the target sequences the biological sample to be analyzed, such as blood or serum, may be treated, if desired, to extract the nucleic acids. The sample nucleic acid may be prepared in various ways to facilitate detection of the target sequence, e.g. denaturation, restriction digestion, electrophoresis or dot blotting. The targeted region of the analyte nucleic acid usually must be at least partially single-stranded to form hybrids with the targeting sequence of the probe. If the sequence is naturally single-stranded, denaturation will not be required. However, if the sequence is double-stranded, the sequence will probably need to be denatured. Denaturation can be carried out by various techniques known in the art.

Analyte nucleic acid and probe are incubated under conditions which promote stable hybrid formation of the target sequence in the probe with the putative targeted sequence in the analyte. The region of the probes which is used to bind to the analyte can be made completely complementary to the targeted region of human chromosome 11 for *KVLQT1.* Therefore, high stringency conditions are desirable in order to prevent false positives. However, conditions of high stringency are used only if the probes are complementary to regions of the chromosome which are unique in the genome. The stringency of hybridization is determined by a number of factors during hybridization and during the washing procedure, including temperature, ionic strength, base composition, probe length, and concentration of formamide. These factors are outlined in, for example, Maniatis et al., 1982 and Sambrook et al., 1989. Under certain circumstances, the formation of higher order hybrids, such as triplexes, quadraplexes, etc., may be desired to provide the means of detecting target sequences.

Detection, if any, of the resulting hybrid is usually accomplished by the use of labeled probes. Alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation, random priming or kinasing), biotin, fluorescent groups, chemiluminescent groups (e.g., dioxetanes, particularly triggered dioxetanes), enzymes, antibodies and the like. Variations of this basic scheme are known in the art, and include those variations that facilitate separation of the hybrids to be detected from extraneous materials and/or that amplify the signal from the labeled moiety. A number of these variations are reviewed in, e.g., Matthews & Kricka, 1988; Landegren et al., 1988; U.S. Patent 4,868,105; and in EPO Publication No. 225,807.

As noted above, non-PCR based screening assays are also contemplated in this invention. This procedure hybridizes a nucleic acid probe (or an analog such as a methyl phosphonate backbone replacing the normal phosphodiester), to the low level DNA target. This probe may have an enzyme covalently linked to the probe, such that the covalent linkage does not interfere with the specificity of the hybridization. This enzyme-probe-conjugate-target nucleic acid complex can then be isolated away from the free probe enzyme conjugate and a substrate is added for enzyme detection. Enzymatic activity is observed as a change in color development or luminescent output resulting in a 10³-10⁶ increase in sensitivity. For an example relating to the preparation of oligodeoxynucleotide-alkaline phosphatase conjugates and their use as hybridization probes, see Jablonski et al., 1986.

Two-step label amplification methodologies are known in the art. These assays work on the principle that a small ligand (such as digoxigenin, biotin, or the like) is attached to a nucleic acid probe capable of specifically binding *KVLQT1.* Allele specific probes are also contemplated within the scope of this example and exemplary allele specific probes include probes encompassing the predisposing mutations of this patent application.

In one example, the small ligand attached to the nucleic acid probe is specifically recognized by an antibody-enzyme conjugate. In one embodiment of this example, digoxigenin is attached to the nucleic acid probe. Hybridization is detected by an antibody-alkaline phosphatase conjugate which turns over a chemiluminescent substrate. For methods for labeling nucleic acid probes according to this embodiment see Martin et al., 1990. In a second example, the small ligand is recognized by a second ligand-enzyme conjugate that is capable of specifically complexing to the first ligand. A well known embodiment of this example is the biotin-avidin type of interactions. For methods for labeling nucleic acid probes and their use in biotin-avidin based assays see Rigby et al.,1977 and Nguyen et al., 1992.

It is also contemplated within the scope of this invention that the nucleic acid probe assays of this invention will employ a cocktail of nucleic acid probes capable of detecting *KVLQT1* or *minK.* Thus, in one example to detect the presence of *KVLQT1* in a cell sample, more than one probe complementary to the gene is employed and in particular the number of different probes is alternatively two, three, or five different nucleic acid probe sequences. In another example, to detect the presence of mutations in the *KVLQT1* gene sequence in a patient, more than one probe complementary to these genes is employed where the cocktail includes probes capable of binding to the allele-specific mutations identified in populations of patients with alterations in *KVLQT1.* In this embodiment, any number of probes can be used, and will preferably include probes corresponding to the major gene mutations identified as predisposing an individual to LQT.

### Methods of Use: Peptid Diagnosis and Diagnostic Kits

The presence of LQT can also be detected on the basis of the alteration of wild-type *KVLQT1* or *minK* polypeptide. Such alterations can be determined by sequence analysis in accordance with conventional techniques. More preferably, antibodies (polyclonal or monoclonal) are used to detect differences in, or the absence of *KVLQT1* or *minK* peptides. Techniques for raising and purifying antibodies are well known in the art and any such techniques may be chosen to achieve the preparations claimed in this invention. In a preferred embodiment of the invention, antibodies will immunoprecipitate *KVLQT1* or *minK* proteins from solution as well as react with these proteins on Western or immunoblots of polyacrylamide gels. In another preferred embodiment, antibodies will detect *KVLQT1* or *minK* proteins in paraffin or frozen tissue sections, using immunocytochemical techniques.

Preferred embodiments relating to methods for detecting *KVLQT1* or *minK* or their mutations include enzyme linked immunosorbent assays (ELISA), radioimmunoassays (RIA), immunoradiometric assays (IRMA) and immunoenzymatic assays (IEMA), including sandwich assays using monoclonal and/or polyclonal antibodies. Exemplary sandwich assays are described by David et al., in U.S. Patent Nos. 4,376,110 and 4,486,530, hereby incorporated by reference.

### Methods of Use: Gene Therapy

According to the present invention, a method is also provided of supplying wild-type *KVLQT1* or *minK* function to a cell which carries a mutant *KVLQT1* or *minK* allele, respectively. Supplying such a function should allow normal functioning of the recipient cells. The wild-type gene or a part of the gene may be introduced into the cell in a vector such that the gene remains extrachromosomal. In such a situation, the gene will be expressed by the cell from the extrachromosomal location. More preferred is the situation where the wild-type gene or a part thereof is introduced into the mutant cell in such a way that it recombines with the endogenous mutant gene present in the cell. Such recombination requires a double recombination event which results in the correction of the gene mutation. Vectors for introduction of genes both for recombination and for extrachromosomal maintenance are known in the art, and any suitable vector may be used. Methods for introducing DNA into cells such as electroporation, calcium phosphate co-precipitation and viral transduction are known in the art, and the choice of method is within the competence of the practitioner.

As generally discussed above, the *KVLQT1* or *minK* gene or fragment, where applicable, may be employed in gene therapy methods in order to increase the amount of the expression products of such gene in cells. It may also be useful to increase the level of expression of a given LQT gene even in those heart cells in which the mutant gene is expressed at a "normal" level, but the gene product is not fully functional.

Gene therapy would be carried out according to generally accepted methods, for example, as described by Friedman, 1991. Cells from a patient would be first analyzed by the diagnostic methods described above, to ascertain the production of *KVLQT1* or *minK* polypeptide in the cells. A virus or plasmid vector (see further details below), containing a copy of the *KVLQT1* or *minK* gene linked to expression control elements and capable of replicating inside the cells, is prepared. Suitable vectors are known, such as disclosed in U.S. Patent 5,252,479 and PCT published application WO 93/07282. The vector is then injected into the patient. If the transfected gene is not permanently incorporated into the genome of each of the targeted cells, the treatment may have to be repeated periodically.

Gene transfer systems known in the art may be useful in the practice of the gene therapy methods of the present invention. These include viral and nonviral transfer methods. A number of viruses have been used as gene transfer vectors, including papovaviruses (e.g., SV40, Madzak et al., 1992), adenovirus (Berkner, 1992; Berkner et al., 1988; Gorziglia and Kapikian, 1992; Quantin et al., 1992; Rosenfeld et al., 1992; Wilkinson et al., 1992; Stratford-Perricaudet et al., 1990), vaccinia virus (Moss, 1992), adeno-associated virus (Muzyczka, 1992; Ohi et al., 1990), herpesviruses including HSV and EBV (Margolskee, 1992; Johnson et al., 1992; Fink et al., 1992; Breakfield and Geller, 1987; Freese et al., 1990), and retroviruses of avian (Brandyopadhyay and Temin, 1984; Petropoulos et al., 1992), murine (Miller, 1992; Miller et al., 1985; Sorge et al., 1984; Mann and Baltimore, 1985; Miller et al., 1988), and human origin (Shimada et al., 1991; Helseth et al., 1990; Page et al., 1990; Buchschacher and Panganiban, 1992). Most human gene therapy protocols have been based on disabled murine retroviruses.

Nonviral gene transfer methods known in the art include chemical techniques such as calcium phosphate coprecipitation (Graham and van der Eb, 1973; Pellicer et al., 1980); mechanical techniques, for example microinjection (Anderson et al., 1980; Gordon et al., 1980; Brinster et al., 1981; Constantini and Lacy, 1981); membrane fusion-mediated transfer via liposomes (Felgner et al., 1987; Wang and Huang, 1989; Kaneda et al., 1989; Stewart et al., 1992; Nabel et al., 1990; Lim et al., 1992); and direct DNA uptake and receptor-mediated DNA transfer (Wolff et al., 1990; Wu et al., 1991; Zenke et al., 1990; Wu et al., 1989; Wolff et al., 1991; Wagner et al., 1990; Wagner et al.,1991; Cotten et al., 1990; Curiel et al., 1991a; Curiel et al., 1991b).

In an approach which combines biological and physical gene transfer methods, plasmid DNA of any size is combined with a polylysine-conjugated antibody specific to the adenovirus hexon protein, and the resulting complex is bound to an adenovirus vector. The trimolecular complex is then used to infect cells. The adenovirus vector permits efficient binding, internalization, and degradation of the endosome before the coupled DNA is damaged.

Liposome/DNA complexes have been shown to be capable of mediating direct in vivo gene transfer. While in standard liposome preparations the gene transfer process is nonspecific, localized in vivo uptake and expression have been reported in tumor deposits, for example, following direct *in situ* administration (Nabel, 1992).

Gene transfer techniques which target DNA directly to heart tissue is preferred. Receptormediated gene transfer, for example, is accomplished by the conjugation of DNA (usually in the form of covalently closed supercoiled plasmid) to a protein ligand via polylysine. Ligands are chosen on the basis of the presence of the corresponding ligand receptors on the cell surface of the target cell/tissue type. These ligand-DNA conjugates can be injected directly into the blood if desired and are directed to the target tissue where receptor binding and internalization of the DNA-protein complex occurs. To overcome the problem of intracellular destruction of DNA, coinfection with adenovirus can be included to disrupt endosome function.

The therapy is as follows: patients who carry a *KVLQT1* or *minK* susceptibility allele are treated with a gene delivery vehicle such that some or all of their heart precursor cells receive at least one additional copy of a functional normal *KVLQT1* or *minK* allele. In this step, the treated individuals have reduced risk of LQT to the extent that the effect of the susceptible allele has been countered by the presence of the normal allele.

### Methods of Use: Transformed Hosts

Animals for testing therapeutic agents can be selected after mutagenesis of whole animals or after treatment of germline cells or zygotes. Such treatments include insertion of mutant *KVLQT1* and/or *minK* alleles, usually from a second animal species, as well as insertion of disrupted homologous genes. Alternatively, the endogenous *KVLQT1* or *minK* gene of the animals may be disrupted by insertion or deletion mutation or other genetic alterations using conventional techniques (Capecchi, 1989; Valancius and Smithies, 1991; Hasty *et al.,* 1991; Shinkai *et al.,* 1992; Mombaerts *et al.,* 1992; Philpott *et al.,* 1992; Snouwaert *et al.,* 1992; Donehower *et al.,* 1992). After test substances have been administered to the animals, the presence of LQT must be assessed. If the test substance prevents or suppresses the appearance of LQT, then the test substance is a candidate therapeutic agent for treatment of LQT. These animal models provide an extremely important testing vehicle for potential therapeutic products.

Two strategies had been utilized herein to identify LQT genes, a candidate gene approach and positional cloning. Positional information is now available for three LQT loci with *LQT1* having been mapped to chromosome 11p15.5 (Keating et al., 1991a; Keating et al., 1991b), *LQT2* to 7q35-36 and *LQT3* to 3p21-24 (Jiang et al., 1994). The present invention has also identified *minK,* on chromosome 21, as an LQT gene. The candidate gene approach relies on likely mechanistic hypotheses based on physiology. Although little is known about the physiology of LQT, the disorder is associated with prolongation of the QT interval on electrocardiograms, a sign of abnormal cardiac repolarization. This association suggests that genes encoding ion channels, or their modulators, are reasonable candidates for LQT. This hypothesis is now supported by the discovery that chromosome 7-linked LQT results from mutations in *HERG,* a putative cardiac potassium channel gene. A neuroendocrine calcium channel gene (*CACNL1A2;* Chin et al., 1991; Seino et al., 1992) and a gene encoding a GTP-binding protein that modulates potassium channels (*GNAI2;* Weinstein et al., 1988; Magovcevic et al., 1992) became candidates for *LQT3* based on their chromosomal location. Subsequent linkage analyses, however, have excluded these genes (Wang and Keating, unpublished data). It has now been shown that *LQT3* is associated with *SCN5A* (Wang et al., 1995a). Despite considerable effort, however, a candidate gene approach to chromosome 11-linked LQT has not been successful. Two potassium channel genes (*KCNA4* and *KCNC1*) were mapped to the short arm of chromosome 11 (Wymore et al., 1994), but both were excluded as candidates for *LQT1* by linkage analyses (Russell et al., 1995; the present study). All other previously characterized cardiac potassium, chloride, sodium and calcium channel genes were similarly excluded based on their chromosomal locations. The present study has used positional cloning and mutational analyses to identify *LQT1.*

The present invention has used genotypic analyses to show that *KVLQT1* is tightly linked to *LQT1* in 16 unrelated families (details provided in the Examples). *KVLQT1* is a putative cardiac potassium channel gene and causes the chromosome 11-linked form of LQT. Genetic analyses suggested that KVLQTI1 encodes a voltage-gated potassium channel with functional importance in cardiac repolarization and it is now shown that KVLQT1 coassembles with minK to form a cardiac I_{Ks} potassium channel. If correct, the mechanism of chromosome 11-linked LQT probably involves reduced repolarizing KVLQT1 current. Since potassium channels with six transmembrane domains are thought to be formed from homo- or hetero-tetramers (MacKinnon, 1991; MacKinnon et al., 1993; Covarrubias et al., 1991), it is possible that LQT-associated mutations of KVLQT1 act through a dominant-negative mechanism. The type and location of KVLQT1 mutations described here are consistent with this hypothesis. The resultant suppression of potassium channel function, in turn, would likely lead to abnormal cardiac repolarization and increased risk of ventricular tachyarrhythmias. The mutations identified in *HERG,* and the biophysics of potassium channel alpha subunits, suggest that chromosome 7-linked LQT results from dominant-negative mutations and a resultant reduction in functional channels. In chromosome 3-linked LQT, by contrast, the LQT-associated deletions identified in *SCN5A* are likely to result in functional cardiac sodium channels with altered properties, such as delayed inactivation or altered voltage-dependence of channel inactivation. Delayed sodium channel inactivation would increase inward sodium current, depolarizing the membrane. This effect is similar to the altered membrane potential expected from *HERG* mutations where outward potassium current is decreased. It is unlikely that more deleterious mutations of *SCN5A* would cause LQT. A reduction of the total number of cardiac sodium channels, for example, would be expected to reduce action potential duration, a phenotype opposite that of LQT.

Presymptomatic diagnosis of LQT has depended on identification of QT prolongation on electrocardiograms. Unfortunately, electrocardiograms are rarely performed in young, healthy individuals. In addition, many LQT gene carriers have relatively normal QT intervals, and the first sign of disease can be a fatal cardiac arrhythmia (Vincent et al., 1992). Now that a third LQT gene (KVLQT1) has been identified and *minK* has also been associated with LQT, genetic testing for this disorder can be contemplated. This will require continued mutational analyses and identification of additional LQT genes. With more detailed phenotypic analyses, phenotypic differences between the varied forms of LQT may be discovered. These differences may be useful for diagnosis and treatment.

The identification of the association between the *SCN5A, HERG* and *KVLQT1* gene mutations and LQT permits the early presymptomatic screening of individuals to identify those at risk for developing LQT. To identify such individuals, the *SCN5A, HERG* and/or *KVLQT1* alleles are screened for mutations either directly or after cloning the alleles. Mutations in *minK* can be similarly discovered. The alleles are tested for the presence of nucleic acid sequence differences from the normal allele using any suitable technique, including but not limited to, one of the following methods: fluorescent *in situ* hybridization (FISH), direct DNA sequencing, PFGE analysis, Southern blot analysis, single stranded conformation analysis (SSCP), linkage analysis, RNase protection assay, allele specific oligonucleotide (ASO) dot blot analysis and PCR-SSCP analysis. For example, either (1) the nucleotide sequence of both the cloned alleles and normal *KVLQT1* gene or appropriate fragment (coding sequence or genomic sequence) are determined and then compared, or (2) the RNA transcripts of the *KVLQT1* gene or, gene fragment are hybridized to single stranded whole genomic DNA from an individual to be tested, and the resulting heteroduplex is treated with Ribonuclease A (RNase A) and run on a denaturing gel to detect the location of any mismatches. Two of these methods can be carried out according to the following procedures.

The alleles of the *KVLQT1* or *minK* gene in an individual to be tested are cloned using conventional techniques. For example, a blood sample is obtained from the individual. The genomic DNA isolated from the cells in this sample is partially digested to an average fragment size of approximately 20 kb. Fragments in the range from 18-21 kb are isolated. The resulting fragments are ligated into an appropriate vector. The sequences of the clones are then determined and compared to the normal *KVLQT1* or *minK* gene.

Alternatively, polymerase chain reactions (PCRs) are performed with primer pairs for the 5' region or the exons of the *KVLQT1* gene. PCRs can also be performed with primer pairs based on any sequence of the normal *KVLQT1* gene. For example, primer pairs for one of the introns can be prepared and utilized. Finally, PCR can also be performed on the mRNA. The amplified products are then analyzed by single stranded conformation polymorphisms (SSCP) using conventional techniques to identify any differences and these are then sequenced and compared to the normal gene sequence.

Individuals can be quickly screened for common *KYLQT1* or *minK* gene variants by amplifying the individual's DNA using suitable primer pairs and analyzing the amplified product, e.g., by dot-blot hybridization using allele-specific oligonucleotide probes.

The second method employs RNase A to assist in the detection of differences between the normal *KVLQT1* or *minK* gene and defective genes. This comparison is performed in steps using small (-500 bp) restriction fragments of the *KVLQT1* or *minK* gene as the probe. First, the *KVLQT1* or *minK* gene is digested with a restriction enzyme(s) that cuts the gene sequence into fragments of approximately 500 bp. These fragments are separated on an electrophoresis gel, purified from the gel and cloned individually, in both orientations, into an SP6 vector (e.g., pSP64 or pSP65). The SP6-based plasmids containing inserts of the *KVLQT1* or *minK* gene fragments are transcribed in vitro using the SP6 transcription system, well known in the art, in the presence of [α-³²P]GTP, generating radiolabeled RNA transcripts of both strands of the gene.

Individually, these RNA transcripts are used to form heteroduplexes with the allelic DNA using conventional techniques. Mismatches that occur in the RNA:DNA heteroduplex, owing to sequence differences between the *KVLQT1* or *minK* fragment and the *KVLQT1* or *minK* allele subclone from the individual, result in cleavage in the RNA strand when treated with RNase A. Such mismatches can be the result of point mutations or small deletions in the individual's allele. Cleavage of the RNA strand yields two or more small RNA fragments, which run faster on the denaturing gel than the RNA probe itself.

Any differences which are found, will identify an individual as having a molecular variant of the *KVLQT1* or *minK* gene and the consequent presence of long QT syndrome. These variants can take a number of forms. The most severe forms would be frame shift mutations or large deletions which would cause the gene to code for an abnormal protein or one which would significantly alter protein expression. Less severe disruptive mutations would include small in-frame deletions and nonconservative base pair substitutions which would have a significant effect on the protein produced, such as changes to or from a cysteine residue, from a basic to an acidic amino acid or vice versa, from a hydrophobic to hydrophilic amino acid or vice versa, or other mutations which would affect secondary or tertiary protein structure. Silent mutations or those resulting in conservative amino acid substitutions would not generally be expected to disrupt protein function.

Genetic testing will enable practitioners to identify individuals at risk for LQT at, or even before, birth. Presymptomatic diagnosis of LQT will enable prevention of these disorders. Existing medical therapies, including beta adrenergic blocking agents, may prevent and delay the onset of problems associated with the disease. Finally, this invention changes our understanding of the cause and treatment of common heart disease like cardiac arrhythmias which account for 11 % of all natural deaths. Existing diagnosis has focused on measuring the QT interval from electrocardiograms. This method is not a fully accurate indicator of the presence of long QT syndrome. The present invention is a more accurate indicator of the presence of the disease. Genetic testing and improved mechanistic understanding of LQT provide the opportunity for prevention of life-threatening arrhythmias through rational therapies. It is possible, for example, that potassium channel opening agents will reduce the risk of arrhythmias in patients with KVLQT1, minK or HERG mutations; sodium channel blocking agents, by contrast, may be a more effective treatment for patients with mutations that alter the function of SCN5A. Finally, these studies may provide insight into mechanisms underlying common arrhythmias, as these arrhythmias are often associated with abnormal cardiac repolarization and may result from a combination of inherited and acquired factors.

The present invention is further detailed in the following Examples, which are offered by way of illustration and are not intended to limit the invention in any manner. Standard techniques well known in the art or the techniques specifically described below are utilized.

### EXAMPLE 1

### Methods for Phenotypic Evaluation

For these studies, six large LQT kindreds (K1532, K1723, K2605, K1807, K161 and K162) as well as some small kindreds and sporadic cases were studied. LQT patients were identified from medical clinics throughout North America and Europe. Two factors were considered for phenotyping: 1) historical data (the presence of syncope, the number of syncopal episodes, the presence of seizures, the age of onset of symptoms, and the occurrence of sudden death); and 2) the QT interval on electrocardiograms corrected for heart rate (QT_{c}) (Bazzett, 1920). To avoid misclassifying individuals, the same conservative approach to phenotypic assignment that was successful in previous studies was used (Keating et al., 1991 a; Keating et al., 1991b; Jiang et al., 1994). Informed consent was obtained from each individual, or their guardians, in accordance with local institutional review board guidelines. Phenotypic data were interpreted without knowledge of genotype. Symptomatic individuals with a corrected QT interval (QT_{c}) of 0.45 seconds or greater and asymptomatic individuals with a QT_{c} of 0.47 seconds or greater were classified as affected. Asymptomatic individuals with a QT_{c} of 0.41 seconds or less were classified as unaffected. Asymptomatic individuals with QT_{c} between 0.41 and 0.47 seconds and symptomatic individuals with QT_{c} of 0.44 seconds or less were classified as uncertain.

### EXAMPLE 2

### Genotyping and Linkage Analysis

Genomic DNA was prepared from peripheral blood lymphocytes or cell lines derived from Epstein-Barr virus transformed lymphocytes using standard procedures (Anderson and Gusella, 1984). For genotypic analyses, four small tandem repeat (STR) polymorphisms were used that were previously mapped to chromosome 11p15.5: *D11S922, TH, D11S1318* and *D11S860* (Gyapay et al., 1994). Genotyping of RFLP markers (*HRAS1, D11S459* and *D11S12*) was performed as previously described (Keating et al., 1991 a).

Pairwise linkage analysis was performed using MLINK in LINKAGE v5.1 (Lathrop et al., 1985). Assumed values of 0.90 for penetrance and 0.001 for LQT gene frequency were used. Gene frequency was assumed to be equal between males and females. Male and female recombination frequencies were considered to be equal. STR allele frequencies were 1/n where n=number of observed alleles. Although the maximum LOD score for *D11S454* was identified at a recombination fraction of 0, the presence of one non-obligate recombinant (individual VI-14, Figure 1) places this LQT gene telomeric of *D11S454.*

### EXAMPLE 3

### Physical Mapping

Primers were designed based on sequences from TH-INS-IGFII and D11S454 loci and used to identify and isolate clones from CEPH YAC libraries using the PCR based technique (Green and Olson, 1990; Kwiatowski et al., 1990). YAC terminal sequences were determined by inverse PCR as described (Ochman et al., 1988) and used as STSs.

P1 clones were isolated using single copy probes from previously identified cosmids cosQW22 (this study), cCI11-469 (D11S679), cCI11-385 (D11S551), cCI11-565 (D11S601), cCI11-237 (D11S454) (Tanigami et al., 1992; Tokino et al., 1991; Sternberg, 1990). Newly isolated P1s were mapped to chromosome 11p15 by FISH or Southern analyses. End-specific riboprobes were generated from newly isolated P1s and used to identify additional adjacent clones (Riboprobe Gemini Core System Kit; Promega). DNA for P1 and cosmid clones was prepared using alkaline lysis plasmid isolation and purified by equilibrium centrifugation in CsCl-ethidium bromide gradients as described (Sambrook et al., 1989). P1 insert end sequences were determined by cycle sequencing as described (Wang and Keating, 1994). STSs were generated based on these insert end sequences. Overlap between P1s and cosmids was calculated by summing the restriction fragments in common.

### EXAMPLE 4

### Isolation and Characterization of KVLQT1 Clones

An adult human cardiac cDNA library (Stratagene) was plated, and 1 x 10⁶ plaques were screened using trapped exon 4181A as the probe. Sequences of trapped exon 4181A were used to design oligonucleotide probes for cDNA library screening. The GENETRAPPER^{™} cDNA Positive Selection System was used to screen 1 x 10¹¹ clones from a human heart cDNA library (Life Technologies, Inc.). The sequences of the capture and repair oligonucleotides were 5'-CAGATCCTGAGGATGCT-3' (SEQ ID NO:1) and 5'-GTACCTGGCTGAGAAGG-3' (SEQ ID NO:2).

Composite cDNA sequences for KVLQT1 were obtained by end sequencing of overlapping cDNA clones and by primer walking. Sequencing was performed either automatically, using Pharmacia A.L.F. automated sequencers, or manually, using a Sequenase Version 2.0 DNA Sequencing Kit (United States Biochemical, Inc.). Database analyses and sequence analyses were carried out using the GCG software package, IG software package, and the BLAST network service from the National Center for Biotechnology Information.

The partial genomic structure (from transmembrane domain S2 to S6) of KVLQT1 was determined by cycle sequencing of P1 18B12 as described (Wang and Keating, 1994). Primers were designed based on KVLQT1 cDNA sequence and used for cycle sequencing.

### EXAMPLE 5

### Mutation Analyses

SSCP was carried out as previously described (Wang et al., 1995a; Wang et al., 1995b). Normal and aberrant SSCP products were isolated sequenced directly as described (Wang and Keating, 1994) or subcloned into pBluescript (SK+; Stratagene) using the T-vector method (Marchuk et al., 1990). When the latter method was used, several clones were sequenced by the dideoxy chain termination method using Sequenase^{™} Version 2.0 (United States Biochemicals, Inc.).

### EXAMPLE 6

### Northern Analyses

A multiple tissue Northern filter (Human MTN blot 1, Clontech) was probed with a ³²P-labeled KVLQT1 cDNA probe as previously described (Curran et al., 1995).

### EXAMPLE 7

### Refined Genetic and Physical Localization of LQT1

The precise location of *LQT1* was determined by genotypic analyses in kindred 1532 (K1532), a large Utah family of northern European descent (Figure 1). This kindred had been used in the initial study linking the first LQT gene, *LQT1,* to chromosome 11p15.5 (Keating et al., 1991 a; Keating et al., 1991b). Additional family members were identified and phenotyped for a total sample size of 217 individuals. Phenotypic determination was performed as previously described (Keating et al., 1991a; Keating et al., 1991b; Jiang et al., 1994). Preliminary genotypic analyses using markers at *HRAS, TH, D11S454,* and *D11S12* included all ascertained members of K1532. These experiments identified informative branches of this family. Additional genotypic analyses were performed using three highly polymorphic markers from chromosome 11p15.5: *D11S922, D11S1318,* and *D11S860* (Gyapay et al., 1994). Genotypes and pairwise LOD scores for each marker are shown in Figure 1 and Table 1. Of these markers, *TH* and *D11S1318* were completely linked. Recombination was identified with all other markers tested, including HRAS, but in each case a statistically significant positive LOD score (+3 or greater) was identified. These data indicate that *LQT1* is completely linked to *TH and D11S1318* in this kindred and that the disease gene is located centromeric of *HRAS.*

To refine localization of *LQT1,* haplotype analyses of K1532 were performed (see Figure 1). Nine chromosomes bearing informative recombination events were identified. Telomeric recombination events were observed in unaffected individual IV-22 (between *D11S922* and *TH*), affected individual IV-25 (between *D11S922* and *TH*), unaffected individual V-6 (between *HRAS* and *D11S922*), and affected individual V-24 (between *HRAS* and *D11S922*). Centromeric recombination events were identified in unaffected individual V-17 (between *D11S860* and *D11S454*), affected individual V-24 (between *D11S860* and *D11S454),* unaffected individual V-34 (between *D11S860* and *D11S454*), unaffected individual VI-13 (between *D11S860* and *D11S454*), unaffected individual VI-14 (between *D11S454* and *D11S1318*), and affected individual VI-16 (between *D11S860* and *D11S454*). These data indicate that *LQT1* is located between *D11S922* and *D11S454.* Together with recent studies placing *LQT1* centromeric of *TH* (Russell et al., 1995), these data place *LQT1* in the interval between *TH* and *D11S454.*

The size of the region containing *LQT1* was estimated using pulsed-field gel analyses with genomic probes from chromosome 11P15.5. Probes from *TH, D11S551* and *D11S454* hybridized to a 700 kb Mlu I restriction fragment (Figure 2). These data suggested that the region containing *LQT1* is less than 700 kb. Physical representation of this region was achieved by screening yeast artificial chromosome (YAC) and P1 libraries with probes from the region (Tanigami et al., 1992; Tokino et al., 1991). The order of these clones was confirmed using fluorescent *in situ* hybridization (FISH) analyses as: telomere-*TH-D11S551-D11S679-D11S601-D11S454*-centromere. The clones identified in initial experiments were then used for identification of adjacent, overlapping clones. The minimum set of clones from the *LQT1* interval is shown in Figure 2.

**Table 1 Pairwise LOD scores between LQT1 and 11p15.5 markers**

| Recombination fraction (θ) | | | | | | | | |
|---|---|---|---|---|---|---|---|---|
| | 0.0 | 0.001 | 0.01 | 0.05 | 0.1 | 0.2 | Zₘₐₓ* | θₘₐₓ † |
| | | | | | | | | |
| *HRAS* | 9.67 | 9.94 | 10.50 | 10.38 | 9.62 | 7.57 | 10.59 | 0.021 |
| *D11S922* | 10.05 | 13.05 | 13.85 | 13.59 | 12.59 | 10.01 | 13.92 | 0.019 |
| *TH* | 11.01 | 10.99 | 10.82 | 10.06 | 9.07 | 6.96 | 11.01 | 0.0 |
| *D11S1318* | 10.30 | 10.29 | 10.13 | 9.40 | 8.47 | 6.50 | 10.30 | 0.0 |
| *KVLQT1* | 14.19 | 14.17 | 13.94 | 12.89 | 11.54 | 8.68 | 14.19 | 0.0 |
| *D11S454* | 11.06 | 11.05 | 10.89 | 10.16 | 9.17 | 7.01 | 11.06 | 0.0 |
| *D11S860* | 5.77 | 6.92 | 8.32 | 9.14 | 8.92 | 7.46 | 9.15 | 0.058 |
| *D11S12* | 1.50 | 2.26 | 3.12 | 3.46 | 3.27 | 2.49 | 3.46 | 0.047 |

| | | | | | | | | |
|---|---|---|---|---|---|---|---|---|
| LOD scores were computed with the assumption of 90% penetrance and gene frequency of 0.001 (ref 31). *Zₘₐₓ indicates maximum LOD score. †θₘₐₓ. indicates estimated recombination fraction at Zₘₐₓ. | | | | | | | | |

### EXAMPLE 8

### Identification and Characterization of KVLQT1

Exon amplification with clones from the physical map was performed to identify candidate genes for *LQT1.* Exon trapping was performed using pSPL3B (Burn et al., 1995) on genomic P1 clones as previously described (Buckler et al., 1991; Church et al., 1994). A minimum of 128 trapped exons from each P1 clone were initially characterized by sizing the PCR products. From these, 400 clones were further analyzed by dideoxy sequencing using an A.L.F. automated sequencer (Pharmacia). DNA sequence and database analyses revealed eight possible exons with predicted amino acid sequence similarity to ion channels. The highest similarity was obtained for a 238 base pair trapped exon (4181A), with 53% similarity to potassium channel proteins from multiple species, including similarity to a portion of a putative pore region. PCR analyses were used to map 4181A to the short arm of chromosome 11 and to two P1s from the physical map (118A10, 18B12). These data suggested that 4181A was part of a potassium channel gene on chromosome 11p15.5.

Two different cDNA library screening methods were used to determine if trapped exon 4181A was part of a gene. Traditional plaque filter hybridization with an adult human cardiac cDNA library led to the identification of a single positive clone. A variation of cDNA selection was used to screen a second cardiac cDNA library (the GENETRAPPER^{™} cDNA Positive Selection System, Life Technologies, Inc.), and twelve independent clones were recovered. DNA sequence analyses revealed complete alignment with sequences derived from 4181A and the other trapped exons described above. The composite sequence of these cDNA clones is shown in Figure 3A. The longest open reading frame spans 1654 base pairs. Two consensus polyadenylation signals were identified upstream of the poly(A) tail in the 3' untranslated region. The identity of the initiation codon is not yet certain.

This cDNA predicted a protein with structural characteristics of potassium channels. Hydropathy analyses suggested a topology of six major hydrophobic regions that may represent membrane-spanning α-helices. These regions share sequence similarity with potassium channel transmembrane domains S1-S6. A comparison of the predicted amino acid sequence derived from the identified gene and the Shaker (SHA) potassium channel (Pongs et al., 1988) is shown in Figure 3B. In the region containing S1-S6, the amino acid sequence identity was 30% and similarity was 59%. The sequence located 3' of S1-S6 did not have significant similarity to any known protein. Because this gene has high similarity to voltage-gated potassium channel genes and became a strong candidate for *LQT1,* it was named *KVLQT1.*

Northern blot analyses were used to determine the tissue distribution of *KVLQT1* mRNA. *KVLQT1* cDNA probes detected a 3.2 kb transcript in human heart, kidney, lung, and placenta, but not in skeletal muscle, liver, or brain (Figure 4). The heart showed highest levels of *KVLQT1* mRNA.

### EXAMPLE 9

### Characterization of the Complete KVLQT1 cDNA

The studies described above resulted in the cloning and characterization of an incomplete cDNA for *KVLQT1.* The sequence of this incomplete cDNA predicted a protein with six hydrophobic membrane-spanning α-helices (S1-S6) and a typical K+ channel pore signature sequence (Heginbotham et al., 1994). However, this cDNA appeared to be missing the amino terminal domain and did not functionally express. To define the complete sequence of *KVLQT1,* several cDNA libraries were screened and a new clone was isolated. The screening was performed by radiolabeling a partial *KVLQT1* cDNA with ³²P and screening several cDNA libraries obtained from Clontech. A 1.2 kb clone was isolated from a pancreatic library and subcloned into pBluescript II and sequenced. This clone included a putative translational start site and an ATG sequence in-frame with the original *KVLQT1* clones. This new sequence data was combined with the earlier sequence data to yield the cDNA sequence encoding the complete protein. This cDNA sequence as well as 5' and 3' untranslated regions is shown in Figures 12A-12D. The new cDNA sequence predicts a 581 amino acid protein with a complete S1 domain and a 27 amino acid N-terminal region. This is shown in Figure 8A. To ensure that this new sequence was part of the chromosome 11p15.5-linked *KVLQT1* gene, a 135 base pair *Xho*I restriction fragment from this region was used in hybridization experiments with DNA from a somatic cell hybrid panel. The new 5' end mapped to the short arm of chromosome 11. Northern analysis using the new *KVLQT1* sequence indicated hybridization with a single messenger RNA of 3.2 kb in human pancreas, heart, kidney, lung and placenta, but not in brain, liver or skeletal muscle (Figure 8B). The Northern analyses were performed using a multiple tissue Northern filter (Human MTN blot 1, Clontech) as described by Curran et al., 1995.

### EXAMPLE 10

### Characterization of KVLQT1 Function

To define the function of KVLQT1, Chinese hamster ovary (CHO) cells were transfected with the complete cDNA described above in Example 9. The *KVLQT1* cDNA was subcloned into pCEP4 (InVitrogen). CHO cells were cultured in Ham's F-12 medium and transiently transfected using Lipofectamine (Gibco BRL). Cells were transfected for 18 hours in 35 mm dishes containing 6 µL lipofectamine, 0.5 µg green fluorescent protein (pGreen Lantern-1, Gibco BRL), and 1.5 g of *KVLQT1* in pCEP4. Fluorescent cells were voltage-clamped using an Axopatch 200 patch clamp amplifier (Axon Instruments) 48 to 78 hours after transfection. The bathing solution contained, in mM: 142 NaCl, 2 KCl, 1.2 MgCl₂, 1.8 CaCl₂, 11.1 glucose, 5.5 HEPES buffer (pH 7.4, 22-25°C). The pipette solution contained, in mM: 110 potassium glutamate, 20 KCl, 1.0 MgCl₂, 5 EGTA, 5 K₂ATP, 10 HEPES (pH 7.3). Data acquisition and analyses were done using pCLAMP6 (Axon Instruments). The voltage dependence of current activation was determined by fitting the relationship between tail currents (determined by extrapolation of deactivating phase of current to the end of the test pulse) and test potential with a Boltzmann function. Tail currents were normalized relative to the largest value for each oocyte.

A voltage-dependent, outward K⁺ current was observed after membrane depolarization to potentials above -60 mV (Figure 9A). This current reached a steady state within 1 second at +40 mV. Activation of the current was preceded by a brief delay, and repolarization to -70 mV elicited a tail current with an initial increase in amplitude (a hook) before deactivation. Similar tail current hooks were previously observed for HERG K⁺ channels, and were attributed to recovery of channels from inactivation at a rate faster than deactivation (Sanguinetti et al., 1995; Smith et al., 1996; Spector et al., 1996). The activation curve for KVLQT1 current was half-maximal (V_{½}) at -11.6 ± 0.6 mV, and had a slope factor of 12.6 ± 0.5 mV (n = 6; Figure 9B).

The biophysical properties of KVLQT1 were unlike other known cardiac K⁺ currents. It was hypothesized that KVLQT1 might coassemble with another subunit to form a known cardiac channel. The slowly activating delayed rectifier K⁺ current, I_{Ks}, modulates repolarization of cardiac action potentials. Despite intensive study, the molecular structure of the I_{Ks} channel is not understood. Physiological data suggest that one component of the I_{Ks} channel is minK (Goldstein and Miller, 1991; Hausdorff et al., 1991; Takumi et al., 1991; Busch et al., 1992; Wang and Goldstein, 1995; Wang et al., 1996), a 130 amino acid protein with a single putative transmembrane domain (Takumi et al., 1988). The size and structure of this protein, however, have led to doubt that minK alone forms functional channels (Attali et al., 1993; Lesage et al., 1993).

To test this hypothesis, CHO cells were cotransfected with *KVLQT1* and human *minK* (*hminK*) cDNAs. An *hminK* cDNA was subcloned in pCEP4 (InVitrogen) and transfection was performed as described above for *KVLQT1* alone. For the cotransfection of *KVLQT1* and *hminK,* 0.75 µg of each cDNA was used. As reported previously (Lesage et al., 1993), transfection of CHO cells with *hminK* alone did not induce detectable current (n = 10, Figure 9C). Cotransfection of *hminK* with *KVLQT1* induced a slowly activating delayed-rectifier current that was much larger than the current in cells transfected with *KVLQT1* alone (Figures 9D and 9E). The slow activation of current in cotransfected CHO cells was preceded by a delay that lasted several hundred msec, indicating that no significant homomeric KVLQT1 channel current was present. Current did not saturate during long depolarizing pulses, and required a three-exponential function to best describe the initial delay and two phases of current activation. During a 30 sec depolarizing pulse to +40 mV, current was activated with time constants of 0.68 ± 0.18, 1.48 ± 0.16, and 8.0 ± 0.6 sec (n = 4). The isochronal (7.5 sec) activation curve for current had a V_{½}, of 7.5 ± 0.9 mV, and a slope factor of 16.5 ± 0.8 mV (n = 7; Figure 9B). By comparison, the V_{½} and slope of the activation curve for human cardiac I_{Ks} are 9.4 mV and 11.8 mV (Li et al., 1996). Like KVLQT1 and hminK coexpressed in CHO cells, activation of cardiac I_{Ks} is extremely slow and was best described by a three-exponential function (Balser et al., 1990; Sanguinetti and Jurkiewicz, 1990). Quinidine (50 µM) blocked tail currents in cotransfected CHO cells by 30 ± 8% (n = 5), similar to its effect (40-50% block) on I_{Ks} in isolated myocytes (Balser et al, 1991). Thus, coexpression of KVLQT1 and hminK in CHO cells induced a K⁺ current with biophysical properties nearly identical to cardiac I_{Ks}.

To characterize the properties of hminK and KVLQT1 further, these channels were expressed separately and together in *Xenopus* oocytes. *Xenopus laevis* oocytes were isolated and injected with cRNA as described by Sanguinetti et al., 1995. *KVLQT1* cDNA was subcloned into pSP64 (Promega). *HminK* cDNA was a gift from R. Swanson. Roughly equimolar concentrations of *KVLQT1* cRNA (5.8 ng per oocyte) and *hminK* (1 ng per oocyte) cRNA were used for the co-injection experiments. The bathing solution contained, in mM: 98 NaCl, 2 KCl, 2 MgCl₂, 0.1 CaCl₂, and 5 HEPES (pH 7.6, 22-25°C). For reversal-potential experiments, osmolarity was maintained by equimolar substitution of external NaCl for KCl. Currents were recorded using standard two-microelectrode voltage clamp techniques 3 days after injection of oocytes with cRNA (Sanguinetti et al., 1995). Currents were filtered at 0.5 kHz and digitized at 2 kHz. Data are presented as mean ± s.e.m.

Oocytes injected with *KVLQT1* complementary RNA expressed a rapidly activating outward K⁺ current with a voltage dependence of activation nearly identical to CHO cells transfected with *KVLQT1* cDNA (Figures 10A and 10B). The K⁺ selectivity of KVLQT1 channels was determined by measuring the reversal potential (Eᵣₑᵥ) of tail currents in different concentrations of extracellular K ([K⁺]ₑ). The slope of the relationship between Eᵣₑᵥ and log[K⁺]ₑ was 49.9 ± 0.4 mV (n = 7; Figure 10C), significantly less than predicted by the Nernst equation (58 mV) for a perfectly selective K⁺ channel. Co-injection of oocytes with *KVLQT1* and *hminK* cRNA induced a current similar to I_{Ks} (Figure 11C). The slope of the relationship between Eᵣₑᵥ and log[K⁺]ₑ for co-injected oocytes was 49.9 ± 4 mV (n = 6), similar to KVLQT1 alone and to guinea pig cardiac I_{Ks} (49 mV) (Matsuura et al., 1987). The isochronal (7.5 sec) activation curve for co-injected oocytes had a V_{½} of 6.2 mV and a slope of 12.3 mV (Figure 11E), similar to cardiac I_{Ks}.

### EXAMPLE 11

### Identification of a KVLQT1 Gene in Xenopus

By contrast with CHO cells, *hminK* was able to undergo functional expression in *Xenopus* oocytes (Figure 11B). The induced current (I_{sK}) was smaller than the current induced in co-injected oocytes, but the kinetics and voltage dependence of activation were similar (Figures 11 A-E). Two observations have led to the hypothesis that I_{sK} in *Xenopus* oocytes results from channels formed by coassembly of minK with an unidentified, constitutively expressed subunit. First, the magnitude of I_{sK} saturates after injection of very small amounts of *minK* cRNA (Figure 11D), suggesting that an endogenous component of limited quantity is required for functional expression ((Wang and Goldstein, 1995; Cui et al., 1994). Second, heterologous expression of minK in mammalian cells does not induce detectable current (Lesage et al., 1993) (Figure 9C), suggesting that minK is not sufficient to form functional channels. It was hypothesized that this unidentified subunit might be a homologue of KVLQT1. To test this hypothesis, a *Xenopus* oocyte cDNA library (Clontech) was screened with a *KVLQT1* cDNA clone spanning the S3-S5 domains. A 1.6 kb partial clone (*XKVLQT1,* Figure 8A) was isolated. *XKVLQT1* is 88% identical at the amino acid level with the corresponding region of *KVLQT1* (Figure 8A). These data suggest that I_{sK} results from the coassembly of the XKVLQT1 and minK proteins.

It was concluded that KVLQT1 and hminK coassemble to form the cardiac I_{Ks} channel. Two delayed-rectifier K⁺ currents, I_{Kr} and I_{Ks}, modulate action-potential duration in cardiac myocytes (Li et al., 1996; Sanguinetti and Jurkiewicz, 1990). Previous studies have implicated dysfunction of I_{Kr} channels in long QT syndrome (Sanguinetti et al., 1995; Curran et al., 1995; Sanguinetti et al., 1996). The observation that *KVLQT1* mutations also cause this disorder (Wang et al., 1996), and the discovery that KVLQT1 forms part of the I_{Ks} channel, indicate that dysfunction of both cardiac delayed-rectifier K⁺ channels contribute to risk of sudden death from cardiac arrhythmia.

### EXAMPLE 12

### Cosegregation of KVLQT1 Missense Mutations with LQT in Six Large Families

To test the hypothesis that *KVLQT1* is *LQT1,* single-strand conformational polymorphism (SSCP) analyses were used to screen for functional mutations in affected members of K1532, the largest LQT family that showed linkage to chromosome 11. SSCP was carried out as previously described (Wang et al., 1995a; Wang et al., 1995b). Normal and aberrant SSCP products were isolated and sequenced directly as described (Wang and Keating, 1994) or subcloned into pBluescript (SK+) (Stratagene) using the T-vector method (Marchuk et al., 1990). When the latter method was used, several clones were sequenced by the dideoxy chain termination method using Sequenase^{™} Version 2.0 (United States Biochemicals, Inc.). Analyses were focused on the region between S2 and S6 since these regions might be important for KVLQT1 function. We designed oligonucleotide primers based on cDNA sequences and used these primers for cycle sequencing reactions with the *KVLQT1*-containing P1, 18B12 (Wang and Keating, 1994). These experiments defined intronic sequences flanking exons encoding S2-S6. Additional primers were then generated from these intronic sequences and used for SSCP analyses (Table 2).

SSCP analyses identified an anomalous conformer in the 70 affected members of K1532 (Figure 5). This aberrant conformer was not observed in the 147 unaffected members of this kindred or in genomic DNA from more than 200 unrelated control individuals (Q. Wang, unpublished results). The two-point LOD score for linkage between this anomaly and LQT was 14.19 at a recombination fraction of 0 (Table 1). No recombination was observed between *KVLQT1* and *LQT1,* indicating that these loci are completely linked. DNA sequence analyses of the normal and aberrant SSCP conformers revealed a single base substitution, a G to A transition, at the first nucleotide of codon Val-125 (Figure 5 and Table 3). This mutation results in a valine to methionine substitution in the predicted intracellular domain between S4 and S5.

To further test the hypothesis that mutations in *KVLQT1* cause LQT, DNA samples from affected members of five additional large LQT kindreds were studied. Linkage analyses with polymorphic markers from this region had shown that the disease phenotype was linked to chromosome 11 in these families (Q. Wang, unpublished results). Aberrant SSCP conformers were identified in affected members of K1723, K2605, K1807 (Figure 5), K161 and K162 (Q. Wang, unpublished results). The SSCP anomalies identified in K161 and K162 were identical to that observed in K1807 (Q. Wang, unpublished results). The aberrant SSCP conformer was not seen in unaffected members of these kindreds or in DNA samples from more than 200 unrelated control individuals (Figure 5; Q. Wang, unpublished results). The normal and aberrant conformers identified in each family were sequenced. The nucleotide change, coding effect, and location of each mutation are summarized in Table 3.

**Table 2. PCR primers used to define KVLQT1 mutations.**

| Primer | Sequence | Region amplified | SEQ.ID NO. |
|---|---|---|---|
| 1 | GAGATCGTGCTGGTGGTGTTCT | S2-S3 | 3 |
| 2 | CTTCCTGGTCTGGAAACCTGG | | 4 |
| | | | |
| 3 | CTCTTCCCTGGGGCCCTGGC | S3-S4 | 5 |
| 4 | TGCGGGGGAGCTTGTGGCACAG | | 6 |
| | | | |
| 5 | GGGCATCCGCTTCCTGCAGA | S4 | 7 |
| 6 | CTGGGCCCCTACCCTAACCC | | 8 |
| | | | |
| 7 | TCCTGGAGCCCGAACTGTGTGT | S5-Pore | 9 |
| 8 | TGTCCTGCCCACTCCTCAGCCT | | 10 |
| | | | |
| 9 | CCCCAGGACCCCAGCTGTCCAA | Pore-S6 | 11 |
| 10 | AGGCTGACCACTGTCCCTCT | | 12 |
| | | | |
| 11 | GCTGGCAGTGGCCTGTGTGGA | S6 | 13 |
| 12 | AACAGTGACCAAAATGACAGTGAC | | 14 |

**Table 3 Summary of KVLQT1 mutations**

| Codon | Nucleotide change | Coding effect | Mutation | Region | Kindred | Number of affected |
|---|---|---|---|---|---|---|
| 38-39 | △TCG | Deletion | F38W/G39△ | S2 | K13216 | 1 |
| 49 | GCC to CCC | Missense | A49P | S2-S3 | K13119 | 1 |
| 60 | GGG to AGG | Missense | G60R | S2-S3 | K2557 | 3 |
| 61 | CGG to CAG | Missense | R61Q | S2-S3 | K15019 | 2 |
| 125 | GTG to ATG | Missense | V125M | S4-S5 | K1532 | 70 |
| 144 | CTC to TTC | Missense | L144F | S5 | K1777 | 2 |
| 177 | GGG to AGG | Missense | G177R | Pore | K20926 | 1 |
| 183 | ACC to ATC | Missense | T183I | Pore | K20925 | 1 |
| 212 | GCG to GAG | Missense | A212E | S6 | K1723 | 6 |
| 212 | GCG to GAG | Missense | A212E | S6 | K2050 | 2 |
| 212 | GCG to GTG | Missense | A212V | S6 | K1807 | 6 |
| 212 | GCG to GTG | Missense | A212V | S6 | K161 | 18 |
| 212 | GCG to GTG | Missense | A212V | S6 | K162 | 18 |
| 212 | GCG to GTG | Missense | A212V | S6 | K163 | 3 |
| 212 | GCG to GTG | Missense | A212V | S6 | K164 | 2 |
| 216 | GGG to GAG | Missense | G216E | S6 | K2605 | 11 |

### EXAMPLE 13

### A KVLQT1 Intragenic Deletion and Nine Missense Mutations Associated with LQT in Small Families and Sporadic Cases

To identify additional LQT-associated mutations in *KVLQT1,* further SSCP analyses were performed for small kindreds and sporadic cases. SSCP revealed an aberrant conformer in kindred 13216 (Figure 6). Analyses of more than 200 unrelated control individuals failed to show this anomaly (Q. Wang, unpublished results). This aberrant conformer was cloned and sequenced, revealing a three base pair deletion encompassing codons 38 and 39. This mutation results in a phenylalanine to tryptophan substitution and deletion of a glycine in the putative S2 domain (Table 3).

Aberrant SSCP conformers were identified in affected members of nine additional kindreds: K1777, K20925, K13119, K20926, K15019 (Figure 6), K2050, K163 and K164 (Q. Wang, unpublished results). An aberrant SSCP conformer identified in K2050 was identical to that in K1723, and aberrant conformers identified in K163 and K164 were identical to that observed in K1807. None of the aberrant conformers was identified in DNA samples from more than 200 control individuals (Q. Wang, unpublished results). In each case, the normal and aberrant conformers were sequenced. These data are shown in Figure 6 and summarized in Table 3. In total, *KVLQT1* mutations associated with LQT in 16 families or sporadic cases (Figure 7) were identified, providing strong molecular genetic evidence that mutations in *KVLQT1* cause the chromosome 11-linked form of LQT.

### EXAMPLE 14

### Generation of Polyclonal Antibody against KVLQT1

Segments of *KVLQT1* coding sequence are expressed as fusion protein in *E. coli.* The overexpressed protein is purified by gel elution and used to immunize rabbits and mice using a procedure similar to the one described by Harlow and Lane, 1988. This procedure has been shown to generate Abs against various other proteins (for example, see Kraemer et al., 1993).

Briefly, a stretch of *KVLQT1* coding sequence is cloned as a fusion protein in plasmid PET5A (Novagen, Inc., Madison, WI). After induction with IPTG, the overexpression of a fusion protein with the expected molecular weight is verified by SDS/PAGE. Fusion protein is purified from the gel by electroelution. Identification of the protein as the *KVLQT1* fusion product is verified by protein sequencing at the N-terminus. Next, the purified protein is used as immunogen in rabbits. Rabbits are immunized with 100 µg of the protein in complete Freund's adjuvant and boosted twice in 3 week intervals, first with 100 µg of immunogen in incomplete Freund's adjuvant followed by 100 µg of immunogen in PBS. Antibody containing serum is collected two weeks thereafter.

This procedure is repeated to generate antibodies against the mutant forms of the *KVLQT1* gene. These antibodies, in conjunction with antibodies to wild type *KVLQT1,* are used to detect the presence and the relative level of the mutant forms in various tissues and biological fluids.

### EXAMPLE 15

### Generation of Monoclonal Antibodies Specific for KVLQT1

Monoclonal antibodies are generated according to the following protocol. Mice are immunized with immunogen comprising intact *KVLQT1* or *KVLQT1* peptides (wild type or mutant) conjugated to keyhole limpet hemocyanin using glutaraldehyde or EDC as is well known.

The immunogen is mixed with an adjuvant. Each mouse receives four injections of 10 to 100 µg of immunogen and after the fourth injection blood samples are taken from the mice to determine if the serum contains antibody to the immunogen. Serum titer is determined by ELISA or RIA. Mice with sera indicating the presence of antibody to the immunogen are selected for hybridoma production.

Spleens are removed from immune mice and a single cell suspension is prepared (see Harlow and Lane, 1988). Cell fusions are performed essentially as described by Kohler and Milstein, 1975. Briefly, P3.65.3 myeloma cells (American Type Culture Collection, Rockville, MD) are fused with immune spleen cells using polyethylene glycol as described by Harlow and Lane, 1988. Cells are plated at a density of 2x10⁵ cells/well in 96 well tissue culture plates. Individual wells are examined for growth and the supernatants of wells with growth are tested for the presence of *KVLQT1* specific antibodies by ELISA or RIA using wild type or mutant *KVLQT1* target protein. Cells in positive wells are expanded and subcloned to establish and confirm monoclonality.

Clones with the desired specificities are expanded and grown as ascites in mice or in a hollow fiber system to produce sufficient quantities of antibody for characterization and assay development.

### EXAMPLE 16

### Sandwich Assay for KVLQT1

Monoclonal antibody is attached to a solid surface such as a plate, tube, bead or particle. Preferably, the antibody is attached to the well surface of a 96-well ELISA plate. 100 µl sample (e.g., serum, urine, tissue cytosol) containing the *KVLQT1* peptide/protein (wild-type or mutants) is added to the solid phase antibody. The sample is incubated for 2 hrs at room temperature. Next the sample fluid is decanted, and the solid phase is washed with buffer to remove unbound material. 100 µl of a second monoclonal antibody (to a different determinant on the *KVLQT1* peptide/protein) is added to the solid phase. This antibody is labeled with a detector molecule (e.g., 125-I, enzyme, fluorophore, or a chromophore) and the solid phase with the second antibody is incubated for two hrs at room temperature. The second antibody is decanted and the solid phase is washed with buffer to remove unbound material.

The amount of bound label, which is proportional to the amount of *KVLQT1* peptide/protein present in the sample, is quantitated. Separate assays are performed using monoclonal antibodies which are specific for the wild-type *KVLQT1* as well as monoclonal antibodies specific for each of the mutations identified in *KVLQT1.*

### EXAMPLE 17

### Assay to Screen Drugs Affecting the KVLQT1 and minK K⁺ Channel

With the knowledge that KVLQT1 and minK coassemble to form a cardiac I_{Ks} potassium channel, it is now possible to devise an assay to screen for drugs which will have an effect on this channel. The two genes, *KVLQT1* and *minK,* are cotransfected into oocytes or mammalian cells and coexpressed as described above. The cotransfection is performed using any combination of wild-type or specifically mutated *KVLQT1* and *minK.* When one of the genes used for cotransfection contains a mutation which causes LQT a change in the induced current is seen as compared to cotransfection with wild-type genes only. A drug candidate is added to the bathing solution of the transfected cells to test the effects of the drug candidates upon the induced current. A drug candidate which alters the induced current such that it is closer to the current seen with cells cotransfected with wild-type *KVLQT1* and *minK* is useful for treating LQT.

While the invention has been disclosed in this patent application by reference to the details of preferred embodiments of the invention, it is to be understood that the disclosure is intended in an illustrative rather than in a limiting sense, as it is contemplated that modifications will readily occur to those skilled in the art, within the spirit of the invention and the scope of the appended claims.

### LIST OF REFERENCES

Anderson, et al. (1980). Proc. Natl. Acad. Sci. USA 77,5399-5403.
Anderson, M.A. and Gusella, J.K. (1984). Use of cyclosporin A in establishing Epstein-Barr virus-transformed human lymphoblastoid cell lines. In Vitro 20, 856-858.
Antzelevitch, C. and Sicouri, S. (1994). Clinical relevance of cardiac arrhythmias generated by afterdepolarizations: Role of M cells in the generation of U waves, triggered activity and torsade de pointes. J. Am. Col. Card. 23, 259-277.
Attali, B., et al. (1993). Nature 365, 850-852.
Attwell, D. et al. (1979). Steady-state TTX-sensitive ("window") current in cardiac Purkinje fibres. Pflugers Arch. 379, 137-142.
Ausubel, F.M., et al. (1992). Current Protocols in Molecular Biology, (J. Wiley and Sons, N.Y.)
Balser, J.R. et al. (1991). Circ. Res. 69, 519-529.
Balser, J.R. et al. (1990). J. Gen. Physiol. 96, 835-863.
Bazett, H. C. (1920). An analysis of the time-relationships of electrocardiograms. Heart 7,353-370.
Beaucage & Carruthers (1981). Tetra. Letts. 22,1859-1862.
Benhorin, J. et al. (1993). Evidence of genetic heterogeneity in the long QT syndrome, Science 260, 1960-1962*.*
Berkner, et al. (1988). BioTechniques 6, 616-629.
Berkner (1992). Curr. Top. Microbiol. Immunol. 158, 39-61.
Bonner, T. I. et al. (1987). Identification of a family of muscarinic acetylcholine receptor genes. Science 237, 527-532.
Brandyopadhyay and Temin (1984). Mol. Cell. Biol. 4, 749-754.
Breakfield and Geller (1987). Mol. Neurobiol. 1, 337-371.
Brinster, et al. (1981). Cell 27, 223-231.
Bruggeman, A. et al. (1993). Ether-a-go-go encodes a voltage-gated channel permeable to K+ and Ca2+ and modulated by cAMP. Nature 365, 445-448.
Buchschacher and Panganiban (1992). J. Virol. 66,2731-2739.
Buckler, A.J., et al. (1991). Exon amplification: a strategy to isolate mammalian genes based on RNA splicing. Proc. Natl. Acad. Sci. USA 88, 4005-4009.
Bum, T.C. et al. (1995). Increased exon trapping efficiencies through modifications to the pSPL3 splicing vector. Gene 161, 183-187.
Busch, A.E. et al. (1992). Science 255, 1705-1707.
Capecchi, M.R. (1989). Science 244, 1288.
Cariello (1988). Human Genetics 42, 726.
Chin, H., et al. (1991). A brain L-type calcium channel α1 subunit gene (CCHL1A2) maps to mouse chromosome 14 and human chromosome 3. Genomics 11, 914-919.
Church, D.M., et al. (1994). Isolation of genes from complex sources of mammalian genomic DNA using exon amplification. Nature Genet. 6, 98-104.
Conner, B.J., et al. (1983). Proc. Natl. Acad. Sci. USA 80, 278-282.
Constantini and Lacy (1981). Nature 294, 92-94.
Cotten, M. et al. (1990). Transferrin-polycation-mediated introduction of DNA into human leukemic cells: stimulation by agents that affect the survival of transfected DNA or modulate transferrin receptor levels. Proc. Natl. Acad. Sci. USA 87, 4033-4037.
Cotton, R.G. et al. (1988). Reactivity of cytosine and thymine in single-base-pair mismatches with hydroxylamine and osmium tetroxide and its application to the study of mutations. Proc. Natl. Acad. Sci. USA 85, 4397-4401.
Covarrubias, M. et al. (1991). Shaker, shal, shab, and shaw express independent K+ current systems. Neuron 7, 763-773.
Cui, J. et al. (1994). J. Gen. Physiol. 104, 87-105.
Curiel, et al. (1991a). Hum. Gene Ther. 3, 147-154.
Curiel, et al. (1991b). Proc. Natl. Acad. Sci. USA 88, 8850-8854.
Curran, M. E. et al. (1993). Locus heterogeneity of autosomal dominant long QT syndrome. J. Clin. Invest. 92, 799-803.
Curran, M.E. et al. (1995). A Molecular Basis for Cardiac Arrhythmia: HERG Mutations Cause Long QT Syndrome. Cell 80, 795-803.
Donehower, L.A., et al. (1992). Nature 356, 215.
Enhancers and Eukaryotic Gene Expression, Cold Spring Harbor Press, Cold Spring Harbor, New York (1983).
Feigner, et al. (1987). Proc. Natl. Acad. Sci. USA 84, 7413-7417.
Fiers, et al. (1978). Nature 273, 113.
Fink, et al. (1992). Hum. Gene Ther. 3, 11-19.
Finkelstein, J., et al. (1990). Genomics 7, 167-172.
Freese, et al. (1990). Biochem. Pharmacol. 40, 2189-2199.
Friedman, T. (1991). In Therapy for Genetic Diseases, T. Friedman, ed., Oxford University Press, pp. 105-121.
Gellens, M. et al. (1992). Primary structure and functional expression of the human cardiac tetrodotoxin-insensitive voltage-dependent sodium channel. Proc. Natl. Acad. Sci. USA 89, 554-558.
George, A.L. et al. (1995). Assignment of the human heart tetrodotoxin-resistant voltage-gated Na+ channel α-subunit gene (SCN5A) to band 3p21. Cytogenet. Cell. Genet. 68, 67-70.
Goldstein, S.A.N. and Miller, C. (1991). Neuron 7,403-408.
Gordon, et al. (1980). Proc. Natl. Acad. Sci. USA 77,7380-7384.
Gorziglia and Kapikian (1992). J. Virol. 66, 4407-4412.
Graham and van der Eb (1973). Virology 52, 456-467.
Green, E.D. and Olson, M.V. (1990). Systematic screening of yeast artificial-chromosome libraries by use of the polymerase chain reaction. Proc. Natl. Acad. Sci. USA 87, 1213-1217.
Grompe, M., (1993). Nature Genetics 5, 111-117.
Grompe, M., et al., (1989). Proc. Natl. Acad. Sci. USA 86, 5855-5892.
Gyapay, G. et al. (1994). The 1993-94 Genethon human genetic linkage map. Nat. Genet. 7, 246-339.
Harlow & Lane (1988). Antibodies: A Laboratory Manual (Cold Spring Harbor Laboratory, Cold Spring Harbor, N.Y.).
Hasty, P., K., et al. (1991). Nature 350, 243.
Hausdorff, S.F. et al. (1991). Biochem. 30, 3341-3346.
Heginbotham, L. et al. (1994). Biophys. J. 66, 1061-1067.
Helseth, et al. (1990). J. Virol. 64, 2416-2420.
Jablonski, E., et al. (1986). Nucl. Acids Res. 14, 6115-6128.
Jakoby, W.B. and Pastan, I.H. (eds.) (1979). Cell Culture. Methods in Enzymology, volume 58 (Academic Press, Inc., Harcourt Brace Jovanovich (New York)).
January, C. T. and Riddle, J. M. (1989). Early afterdepolarizations: Mechanism of induction and block. Circ. Res. 64, 977-990.
Jervell, A. and Lange-Nielson, F. (1957). Congenital deaf mutism, functional heart disease with prolongation of the QT interval, and sudden death. Am. Heart J. 54, 59-78.
Jiang, C. et al. (1994). Two long QT syndrome loci map to chromosomes 3 and 7 with evidence for further heterogeneity. Nat. Genet. 8, 141-147.
Johnson, et al. (1992). J. Virol. 66, 2952-2965.
Kaneda, et al. (1989). J. Biol. Chem. 264, 12126-12129.
Kanehisa (1984). Nucl. Acids Res. 12, 203-213.
Kannel, W. B. et al. (1987). Sudden death risk in overt coronary heart diseases: The Framingham study. Am. Heart J. 113, 799-804.
Keating, M. T. et al. (1991a). Linkage of a cardiac arrhythmia, the long QT syndrome, and the Harvey ras-1 gene. Science 252, 704-706.
Keating, M. T. et al. (1991b). Consistent linkage of the long QT syndrome to the Harvey ras-1 locus on chromosome 11. Am. J. Hum. Genet. 49, 1335-1339.
Kinszler, K.W., et al. (1991). Science 251,1366-1370.
Koch, M. C. et al. (1992). The skeletal muscle chloride channel in dominant and recessive human myotonia. Science 257, 797-800.
Kohler, G. and Milstein, C. (1975). Nature 256, 495-497.
Kraemer, F.B. et al. (1993). J. Lipid Res. 34, 663-672.
Kubo, T., et al. (1988). FEBS Letts. 241, 119.
Kwiatowski, T.J. et al. (1990). Rapid identification of yeast artificial chromosome clones by matrix pooling and crude lysate PCR. Nucl. Acids Res. 17, 7191-7192.
Landegren, et al. (1988). Science 242, 229.
Lathrop, G. M. et al. (1985). Multilocus linkage analysis in humans: detection of linkage and estimation of recombination. Am. J. Hum. Genet. 37,482-498.
Lesage, F., et al. (1993). Receptors and Channels 1, 143-152.
Li, G.-R. et al. (1996). Circ. Res. 78, 689-696.
Lim, et al. (1992). Circulation 83, 2007-2011.
MacKinnon, R. (1991). Determination of the subunit stoichiometry of a voltage-activated potassium channel. Nature 350, 232-235.
MacKinnon, R. et al. (1993). Functional stoichiometry of shaker potassium channel inactivation. Science 262, 757-759.
Madzak, et al. (1992). J. Gen. Virol. 73,1533-1536.
Magovcevic, I. et al. (1992). Regional localization of the human G protein ai2 (GNAI2) gene: Assignment to 3021 and a related sequence (GNAI2L) to 12p12-p13. Genomics 12, 125-129.
Maniatis, T. et al. (1982). Molecular Cloning: A Laboratory Manual (Cold Spring Harbor Laboratory, Cold Spring Harbor, N.Y.).
Mann and Baltimore (1985). J. Virol. 54, 401-407.
Marchuk, D. et al. (1990). Construction of T-vectors, a rapid and general system for direct cloning of unmodified PCR products. Nucl. Acids Res. 19, 1154.
Margolskee (1992). Curr. Top. Microbiol. Immunol. 158, 67-90.
Martin, R., et al. (1990). BioTechniques 9, 762-768.
Matsuura, H. et al. (1987). Pflugers Arch. 410, 596-603.
Matteucci, M.D. and Caruthers, M.H. (1981). J. Am. Chem. Soc. 103, 3185.
Matthews & Kricka (1988). Anal. Biochem. 169, 1.
Metzger, et al. (1988). Nature 334, 31-36.
Miller (1992). Curr. Top. Microbiol. Immunol. 158, 1-24.
Miller, et al. (1985). Mol. Cell. Biol. 5, 431-437.
Miller, et al. (1988). J. Virol. 62, 4337-4345.
Modrich, P. (1991). Ann. Rev. Genet. 25, 229-253.
Mombaerts, P., et al. (1992). Cell 68, 869.
Moss, A.J. and McDonald, J. (1970). Unilateral cervicothoracic sympathetic ganglionectomy for the treatment of long QT interval syndrome. N. Engl. J. Med. 285, 903-904.
Moss, A.J. et al. (1991). The long QT syndrome: prospective longitudinal study of 328 families. Circulation 84, 1136-1144.
Moss (1992). Curr. Top. Microbiol. Immunol. 158, 25-38.
Muzyczka (1992). Curr. Top. Microbiol. Immunol. 158, 97-123.
Nabel, et al. (1990). Science 249, 1285-1288.
Nabel (1992). Hum. Gene Ther. 3, 399-410.
"The Cardiac Arrhythmia Suppression Trial II Investigators. Effect of the antiarrhythmic agent moricizine on survival after myocardial infarction." (1992). N. Engl. J. Med. 327, 227.
Newton, C.R. et al. (1989). Nucl. Acids Res. 17, 2503-2516.
Nguyen, Q., et al. (1992). BioTechniques 13, 116-123.
Novack, et al. (1986). Proc. Natl. Acad. Sci. USA 83, 586.
Ochman, H. et al. (1988). Genetic application of an inverse polymerase chain reaction. Genetics 120, 621-623.
Ohi, et al. (1990). Gene 89, 279-282.
Orita, M. et al. (1989). Detection of polymorphisms of human DNA by gel electrophoresis as single strand conformation polymorphisms. Proc. Natl. Acad. Sci. USA 86, 2766-2770.
Page, et al. (1990). J. Virol. 64, 5370-5276.
Pellicer, et al. (1980). Science 209, 1414-1422.
Petropoulos, et al. (1992). J. Virol. 66, 3391-3397.
Philpott, K.L., et al. (1992). Science 256, 1448.
Pongs, O., et al. (1988). Shaker encodes a family of putative potassium channel proteins in the nervous system of Drosophila. EMBO J. 7, 1087-1095.
Quantin, et al. (1992). Proc. Natl. Acad. Sci. USA 89, 2581-2584.
Rano & Kidd (1989). Nucl. Acids Res. 17, 8392.
Rigby, P.W.J., et al. (1977). J. Mol. Biol. 113, 237-251.
Romano, C. (1965). Congenital cardiac arrhythmia. Lancet 1, 658-659.
Rosenfeld, et al. (1992). Cell 68, 143-155.
Roy, N. et al. (1994). Exclusion of HRAS from long QT locus. Nat. Genet. 8, 113-114.
Russell, M.W., et al. (1995). Localization of Romano-Ward long QT syndrome gene, LQT1, to the interval between tyrosine hydroxylase (TH) and D11S1349. Am. J. Hum. Genet. 57, 503-507.
Sambrook, J., et al. (1989). Molecular Cloning: A Laboratory Manual, 2nd Ed. (Cold Spring Harbor Laboratory, Cold Spring Harbor, N.Y.).
Sanguinetti, M.C. et al. (1996). Proc. Natl. Acad. Sci. USA 93, 2208-2212.
Sanguinetti, M.C. et al. (1995). Cell 81, 299-307.
Sanguinetti, M.C. and Jurkiewicz, N.K. (1990). J. Gen. Physiol. 96, 195-215.
Schott, J., et al. (1995). Mapping of a gene for long QT syndrome to chromosome 4q25-27. Am. J. Hum. Genet. 57, 1114-1122.
Schwartz, P. J. et al. (1975). The long QT syndrome. Am. Heart J. 109, 378-390.
Schwartz, P. J. et al. (1994). The long QT syndrome. In Cardiac Electrophysiology: from cell to bedside. D. P. Zipes and J. Jalife eds. (W.B. Sanders Company) pp.788-811.
Seino, S. et al. (1992). Assignment of the gene encoding the α1 subunit of the neuroendocrine/brain-type calcium channel (CACNL1A2) to human chromosome 3, band p14.3. Genomics 13, 1375-1377.
Sheffield, V.C., et al. (1989). Proc. Natl. Acad. Sci. USA 86, 232-236.
Sheffield, V.C., et al., (1991). Am. J. Hum. Genet. 49, 699-706.
Shenk, T.E. et al. (1975). Biochemical method for mapping mutational alterations in DNA with S 1 nuclease; the location of deletions and temperature-sensitive mutations in simian virus 40. Proc. Natl. Acad. Sci. USA 72, 989-993.
Shimada, et al. (1991). J. Clin. Invest. 88,1043-1047.
Shinkai, Y., et al. (1992). Cell 68, 855.
Smith, P.L. et al. (1996). Nature 379,833-836.
Snouwaert, J.N., et al. (1992). Science 257, 1083.
Sorge, et al. (1984). Mol. Cell. Biol. 4, 1730-1737.
Spector, P.S. et al. (1996). J. Gen. Physiol. 107, 611-619.
Sternberg, N. (1990). Bacteriophage P1 cloning system for the isolation, amplification, and recovery of DNA fragments as large as 100 kilobase pairs. Proc. Natl. Acad. Sci. USA 87, 103-107.
Stewart, et al. (1992). Hum. Gene Ther. 3, 267-275.
Stratford-Penicaudet, et al. (1990). Hum. Gene Ther. 1, 241-256.
Surawicz, B. (1989). Electrophysiologic substrate of torsade de pointes: Dispersion of repolarization or early afterdepolarizations? J. Am. Coll. Cardiol. 14, 172-184.
Takumi, T. et al. (1988). Science 242, 1042-1045.
Takumi, T., et al. (1991). J. Biol. Chem. 266, 22192-22198.
Tanigami, A., et al. (1992). Mapping of 262 DNA markers into 24 intervals on human chromosome 11. Am. J. Hum. Genet. 50, 56-64.
Tokino, T., et al. (1991). Isolation and mapping of 62 new RFLP markers on human chromosome 11. Am. J. Hum. Genet. 48, 258-268.
Towbin, J. A. et al. (1994). Evidence of genetic heterogeneity in Romano-Ward long QT syndrome (LQTs): Analysis of 23 families. Circulation 90, 2635-2644.
Valancius, V. & Smithies, O. (1991). Mol. Cell Biol. 11, 1402.
Vincent, G. M. et al. (1992). The spectrum of symptoms and QT intervals in carriers of the gene for the long QT syndrome. N. Engl. J. Med. 327, 846-852.
Wagner, et al. (1991). Proc. Natl. Acad. Sci. USA 88, 4255-4259.
Wagner, et al. (1990). Proc. Natl. Acad. Sci. USA 87, 3410-3414.
Wang and Huang (1989). Biochemistry 28, 9508-9514.
Wang, K.-W. and Goldstein, S.A.N. (1995). Neuron 14,1303-1309.
Wang, K., Tai, K. and Goldstein, S.A.N. (1996). Neuron 16, 571-577.
Wang, Q. and Keating, M. T. (1994). Isolation of P1 insert ends by direct sequencing. BioTechniques 17,282-284.
Wang, Q. et al. (1995a). SCN5A Mutations Associated with an Inherited Cardiac Arrhythmia, Long QT Syndrome. Cell 80, 805-811.
Wang, Q., et al. (1995b). Cardiac sodium channel mutations in patients with long QT syndrome, an inherited cardiac arrhythmia. Hum. Mol. Genet. 4, 1603-1607.
Wang, Q., et al. (1996). Nature Genetics 12, 17-23.
Ward, O. C. (1964). A new familial cardiac syndrome in children. J. Ir. Med. Assoc. 54, 103-106.
Warmke, J. E. and Ganetzky, B. (1994). A family of potassium channel genes related to eag in Drosophila and mammals. Proc. Natl. Acad. Sci. 91, 3438-3442.
Wartell, R.M., et al. (1990). Nucl. Acids Res. 18, 2699-2705.
Weinstein, L.S. et al. (1988). Cloning and characterization of the human gene for the α-subunit of Gi2, a GTP-binding signal transduction protein. FEBS Letters 232, 333-340.
Wetmur & Davidson (1968). J. Mol. Biol. 31, 349-370.
White, M.B., et al. (1992). Genomics 12, 301-306.
Wilkinson, et al. (1992). Nucleic Acids Res. 20, 2233-2239*.*
Willich, S. N. et al. (1987). Circadian variation in the incidence of sudden cardiac death in the Framingham heart study population. Am. J. Cardiol. 60, 801-806.
Wolff, et al. (1990). Science 247, 1465-1468.
Wolff, et al. (1991). BioTechniques 11, 474-485.
Wu, et al. (1989). J. Biol. Chem. 264,16985-16987.
Wu, et al. (1991). J. Biol. Chem. 266, 14338-14342.
Wymore, R.S., et al. (1994). Genomic organization, nucleotide sequence, biophysical properties, and localization of the voltage-gated K+ channel gene KCN4A/Kv1.4 to mouse chromosome 2/human 11p14 and mapping of KCNC1/Kv3.1 to mouse 7/human 11p14.3-p15.2 and KCNA1/Kv1.1 to human 12p13. Genomics 20, 191202.
Zenke, et al. (1990). Proc. Natl. Acad. Sci. USA 87, 3655-3659.
Zipes, D.P. (1987). Proarrhythmic effects of antiarrhythmic drugs. Am. J. Cardiol. 59, 26E-31E.
European Patent Application Publication No. 0332435.
EPO Publication No. 225,807.
Hitzeman et al., EP 73,675A.

### Patents and Patent Applications:

PCT published application WO 93/07282.
U.S. Patent 4,376,110.
U.S. Patent 4,486,530.
U.S. Patent 4,868,105.
U.S. Patent 5,252,479.

The invention relates to the following items:
1. An isolated nucleic acid coding for a human KVLQT1 polypeptide having the amino acid sequence set forth in SEQ ID NO:26.
2. The isolated nucleic acid of item 1 which is a DNA having the nucleotide sequence set forth in SEQ ID NO:25, its complement, an allelic variant thereof or corresponding RNA.
3. An isolated nucleic acid coding for a mutated form of the human KVLQT1 polypeptide set forth in SEQ ID N0:26 which causes long QT syndrome, its complement or corresponding RNA.
4. The isolated nucleic acid of item 3, wherein the mutation is selected from the group consisting of a deletion mutation, a nonsense mutation, an insertion mutation and a missense mutation.
5. The isolated nucleic acid according to item 3 wherein said isolated DNA contains one or more of the mutations shown in Table 3.
6. An isolated nucleic acid having at least 15 contiguous nucleic acid as claimed in any one of items 1 to 5 wherein the nucleic acid sequence suitable for use as a hybridization probe to detect in a sample (i) a DNA having a nucleotide sequence selected from the nucleotide sequence set forth in SEQ ID NO:1, allelic variants thereof and mutated forms thereof or (ii) an RNA corresponding to said DNA.
7. An isolated nucleic acid as claimed in item 6 having at least 15 contiguous nucleic acid as claimed in any one of items 3 to 5 encompassing a mutation.
8. A replicative cloning vector which comprises an isolated nucleic acid as claimed in any one of items 1 to 7 and a replicon operative in a host cell.
9. An expression vector which comprises an isolated nucleic acid of any one of items 1 to 5 wherein the coding sequence for the KVLQT1 polypeptide or modified form thereof is operably linked to suitable control sequences capable of directing expression of said coding sequence in host cells for said vector.
10. Host cells transformed with a vector as claimed in item 8 or 9.
11. A method of producing a polypeptide which is the KVLQT1 polypeptide having the amino acid sequence set forth in SEQ ID NO:26 or a modified form of said polypeptide as defined in item 1 which comprises (i) culturing the host cells of claim 10 containing an expression vector encoding said polypeptide under conditions suitable for the production of said KVLQT1 polypeptide and (ii) recovering said polypeptide.
12. An isolated human KVLQT1 polypeptide having the amino acid sequence se forth in SEQ ID NO:26.
13. An isolated human mutant KVLQT1 polypeptide comprising a mutated form of the KVLQT1 polypeptide having the amino acid sequence set forth in SEQ ID NO:26.
14. The isolated KVLQT1 polypeptide according to item 13 wherein said mutation is one shown in Table 3.
15. An antibody capable of specifically binding one or more polypeptides as claimed in any one of items 12 to 14_{.}
16. A method of assessing a risk in a human subject for long QT syndrome which comprises screening said subject for a mutation in a KVLQT1 gene by comparing the sequence of the KVLQT1 gene or its expression products isolated from a tissue sample of said subject with a wild-type KVLQT1 gene or its expression products, wherein a mutation in the sequence of the subject is indicative of a risk for long QT synderome.
17. The method of item 16 wherein said expression product is selected from the group consisting of mRNA of the KVLQT1 gene and a KVLQT1 polypeptide encoded by the KVLQT1 gene.
18. The method of item 16 or 17 wherein one or more of the following procedures is carried out:
   (a) observing shifts in electrophoretic mobility of single-stranded DNA from said sample on non-denaturing polyacrylamide gels;
   (b) hybridizing a KVLQT1 gene probe to genomic DNA isolated from said sample under conditions suitable for hybridization of said probe to said gene;
   (c) determining hybridization of an allele-specific probe to genomic DNA from said sample;
   (d) amplifying all or part of the KVLQT1 gene from said sample to produce an amplified sequence and sequencing the amplified sequence;
   (e) determining by nucleic acid amplification the presence of a specific KVLQT1 mutant allele in said sample;
   (f) molecularly cloning all or part of the KVLQT1 gene from said sample to produce a cloned sequence and sequencing the cloned sequence;
   (g) determining whether there is a mismatch between molecules (1) KVLQT1 gene genomic DNA or KVLQT1 mRNA isolated from said sample, and (2) a nucleic acid probe complementary to the human wild-type KVLQT1 gene DNA, when molecules (1) and (2) are hybridized to each other to form a duplex;
   (h) amplification of KVLQT1 gene sequences in said sample and hybridization of the amplified sequences to nucleic acid probes which comprise wild-type KVLQT1 gene sequences;
   (i) amplification of KVLQT1 gene sequences in said tissue and hybridization of the amplified sequences to nucleic acid probes which comprise mutant KVLQT1 gene sequences;
   (j) screening for a deletion mutation;
   (k) screening for a point mutation;
   (l) screening for an insertion mutation;
   (m) determining in *situ* hybridization of the KVLQT1 gene in said sample with one or more nucleic acid probes which comprise the KVLQT1 gene sequence or a mutant KVLQT1 gene sequence;
   (n) immunoblotting;
   (o) immunocytochemistry;
   (p) assaying for binding interactions between KVLQT1 gene protein isolated from said tissue and a binding partner capable of specifically binding the polypeptide expression product of a KVLQT1 mutant allele and/or a binding partner for the KVLQT1 polypeptide having the amino acid sequence set forth in SEQ ID NO:26; and
   (q) assaying for the inhibition of biochemical activity of said binding partner.
19. The method of any one of items 16 to 18 wherein the DNA or RNA encoding amino acids 38-39 of a KVQLT1 polypeptide or amino acids 38-39 of the KVLQT1 polypeptide is compared.
20. The method of any one of items 16 to 18 wherein the DNA or RNA is compared for the mutations shown in Table 3.
21. A cell transfected with the DNA of item 1 or 2.
22. A cell transfected with the DNA of item 3, 4 or 5.
23. A cell transfected with RNA complementary to the DNA of item 1 or 2.
24. A cell transfected with RNA complementary to the DNA of item 3, 4 or 5.
25. A nonhuman, transgenic animal wherein said animal comprises wild-type human KVLQT1.
26. The animal of item 25 further containing wild-type human minK.
27. A nonhuman, transgenic animal wherein said animal comprises mutant human KVLQT1.

## Claims

1. A method for the prediction of long QT syndrome (LQT), comprising screening the KVLQT1 gene of an individual for mutations which cause LQT, wherein the wild-type KVLQT1 gene has the sequence of SEQ ID NO:15.

2. The method of claim 1, wherein said screening involves comparing the sequence of the KVLQT1 gene or its expression products isolated from a tissue sample of said individual with a wild-type KVLQT1 gene or its expression products.

3. The method of claim 2, wherein said expression product is selected from the group consisting of mRNA of the KVLQT1 gene and a KVLQT1 polypeptide encoded by the KVLQT1 gene.

4. The method of any one of claims 1 to 3, wherein the DNA or RNA encoding amino acids 38-39 of the KVQLT1 polypeptide of SEQ ID NO:16 or amino acids 38-39 of the KVLQT1 polypeptide of SEQ ID NO:16 are compared.

5. The method of any one of claims 1 to 4, wherein the mutations which cause LQT are those shown in Table 3.
